(19) Europäisches Patentamt
European Patent Office
Office européen des brevets

(11) EP 1 569 656 B1

(12) EUROPEAN PATENT SPECIFICATION

(45) Date of publication and mention of the grant of the patent:
**03.03.2010 Bulletin 2010/09**

(21) Application number: **03768074.1**

(22) Date of filing: **02.12.2003**

(51) Int Cl.:
*A61K 31/66* (2006.01) *A61K 45/06* (2006.01)
*A61P 35/00* (2006.01) *A61P 37/00* (2006.01)
*A61P 35/04* (2006.01)

(86) International application number:
**PCT/IB2003/006375**

(87) International publication number:
**WO 2004/050096 (17.06.2004 Gazette 2004/25)**

(54) **PHOSPHOANTIGENS FOR REGULATING AN IMMUNE RESPONSE**

PHOSPHOANTIGEN ENTHALTENE ZUSAMMENSETZUNGEN ZUR REGULIERUNG VON IMMUNEREAKTIONEN

COMPOSITIONS ET TECHNIQUES DE REGULATION DE REPONSE IMMUNE CHEZ UN PATIENT

(84) Designated Contracting States:
**AT BE BG CH CY CZ DE DK EE ES FI FR GB GR HU IE IT LI LU MC NL PT RO SE SI SK TR**

(30) Priority: **02.12.2002 EP 02292963**

(43) Date of publication of application:
**07.09.2005 Bulletin 2005/36**

(73) Proprietor: **Innate Pharma**
**13009 Marseille (FR)**

(72) Inventors:
- **ROMAGNE, François**
**F-13600 La Ciotat (FR)**
- **SICARD, Hélène**
**F-13009 Marseille (FR)**
- **TIOLLIER, Jérôme**
**F-13008 Marseille (FR)**
- **BELMANT, Christian**
**83140 Six-Fours-Les-Plages (FR)**

(74) Representative: **Gallois, Valérie et al**
**Cabinet BECKER & ASSOCIES**
**25, rue Louis Le Grand**
**75002 Paris (FR)**

(56) References cited:
**WO-A-00/12519** **WO-A-00/59916**
**WO-A-03/009855** **US-A1- 2002 142 996**

- **KUNZMANN VOLKER ET AL: "Stimulation of gammadelta T cells by aminobisphosphonates and induction of antiplasma cell activity in multiple myeloma" BLOOD, vol. 96, no. 2, 15 July 2000 (2000-07-15), pages 384-392, XP002276947 & ISSN: 0006-4971 cited in the application**
- **WILHELM MARTIN ET AL: "gammadelta T cells for immune therapy of patients with lymphoid malignancies." BLOOD, vol. 102, no. 1, 1 July 2003 (2003-07-01), pages 200-206, XP002276948 & ISSN: 0006-4971**
- **DATABASE PROMT 15 December 2003 (2003-12-15), XP002276950 retrieved from STN Database accession no. 2003:627750 & "Phosphostim Innate Pharma partnering opportunity, Worldwide (solid tumor) Innate Pharma phase change I, France (solid tumor)" R D FOCUS DRUG NEWS, 2003,**
- **DATABASE PHIN 8 March 2002 (2002-03-08), XP002276951 retrieved from STN Database accession no. :2002:6085 & ANONYMOUS: "Innate gets Euros2 million loan from French agency" SCRIP, no. 2727, 8 March 2002 (2002-03-08), page 7,**
- **DATABASE CBNB "Innate Pharma exceeds objectives in obtaining funding for first clinical trials." XP002276952 retrieved from STN Database accession no. 19(9):12851 & PHARMACHEM (NOV-DEC 2002) 1 (11-12), 76 AVAILABILITY: B5 SRL. VIA CESARE DA SESTO, 10-20123 MILAN, ITALY. TEL. +39 02 83241119. FAX. + 39 02 8376457. WEB SITE WWW.B5SRL.COM., 2002,**

EP 1 569 656 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **MONTALVETTI ANDREA ET AL: "Farnesyl pyrophosphate synthase is an essential enzyme in Trypanosoma brucei: In vitro RNA interference and in vivo inhibition studies" J. BIOL. CHEM.; JOURNAL OF BIOLOGICAL CHEMISTRY MAY 9 2003, vol. 278, no. 19, 9 May 2003 (2003-05-09), pages 17075-17083, XP002276949**

## Description

### FIELD OF THE INVENTION

The present invention relates to compositions an methods for regulating an immune response in a subject, particularly a T cell response in a subject. The present invention more specifically discloses efficient methods of regulating the innate immunity in a subject, such as by regulating the activity of γδ T cells in a subject. The invention further provides that said methods that are to be used in the treatment of solid tumors and particularly tumors involving metastases.

### BACKGROUND

Most human peripheral blood γδ T cells express a γδTCR heterodimer encoded by Vγ9/Vδ2 genes, some NK-lineage receptors for MHC class I and almost no CD4 nor CD8. These cells have been shown to exhibit strong, non MHC-restricted, cytolytic activity against virus-infected cells (Poccia et al (1999), parasite-infected cells (Constant et al (1995)), or tumor cells (Fournie et Bonneville (1996)). These cells are also physiologically amplified in the context of several unrelated infectious diseases such as tuberculosis, malaria, tularemia, colibacillosis and also by B-cell tumors (for review see Hayday, 2000).

Beside their anti-infectious activity, it was shown in short term cytotoxicity assays that Vγ9/Vδ2 T cells are able to lyse a wide variety of tumor cell lines from very diverse origins : lymphoma and leukemia from B-cell, T-cell or myeloid lineages (Fisch et al., 1997; Selin et al., 1992; Sicard et al., 2001; Sturm et al., 1990; Zheng et al., 2001a), breast carcinoma (Bank et al., 1993), glioblastoma (Fujimiya et al., 1997; Yamaguchi et al., 1997), renal cell carcinoma (Choudhary et al., 1995; Kobayashi et al., 2001; Mitropoulos et al., 1994), nasopharyngeal carcinoma (Zheng et al., 2001b), lung adenocarcinoma (Ferrarini et al., 1996).

In microbes, Vγ9/Vδ2$^+$ lymphocytes spontaneously recognize a structurally related set of nonpeptide antigens, referred to as natural phosphoantigens and alkylamines. In B cell tumors, the nature of antigens for the γδ T cells remains unidentified. Vγ9/Vδ2 $^+$ lymphocytes are also responsive to a variety of virally infected-, activated- or tumoral cell types without prior exposure. Again, in these situations, the responsible antigens remain unknown (for review see Fisch, 2000). It has been shown that, in vitro, Vγ9/Vδ2 2$^+$ lymphocytes respond to synthetic drugs such as therapeutic aminobisphosphonates (reviewed in Espinosa, 2001), leading to their in vitro activation. Recognition of natural non-peptide antigens is mediated by the γδ TCR, through amino acid residues located on both Vy9- and Vδ2- CDR3 regions. Although neither processing nor presentation by CD1 or MHC molecules is involved, Vγ9/Vδ2 $^+$ lymphocyte activation by non-peptide antigens appears to require cell-to-cell contact (Lang, 1995 ; Morita, 1995 ; Miyagawa, 2001 , Rojas, 2002).

The stimulating bacterial antigens have been shown to be small non peptidic compounds classically referred to as phosphoantigens (Behr et al., 1996; Belmant et al., 2000; Constant et al., 1994; Poquet et al., 1998; Tanaka et al., 1995), owing to the presence of phosphate groups in most instances.

*Endogenous metabolites of the mevalonate pathway: IPP*

Vγ9/Vδ2 T cells can also be activated through endogenous metabolites (acting in the micromolar range) such as isopentenyl pyrophosphate or IPP (Espinosa et al., 2001b; Tanaka et al., 1995), which is produced through the conventional mevalonate pathway shared by both microorganisms and mammalian cells. Production of IPP in the latter cells can be up-regulated in situations of cell stress and transformation. In particular a recent study has reported a correlation between the endogenous production levels of IPP in tumor cells and their susceptibility to Vγ9/Vδ2 T cell-mediated lysis (Gober et al., 2003).

*Compounds regulating endogenous metabolites: statins and aminobisphosphonates*

Also consistent with a direct contribution of endogenous metabolites of the mevalonate pathway to Vγ9/Vδ2 T cell recognition, cell treatment with pharmacological agents preventing IPP biosynthesis (such as statins) or leading to IPP accumulation (such as aminobisphosphonates, see below) lead respectively to decreased or enhanced Vγ9/Vδ2 T cell stimulating properties of the treated cells (Gober et al., 2003; Kato et al., 2001).

Aminobisphosphonates are thought to inhibit FPP synthase, an enzyme in the mevalonate pathway, the inhibition of which causes the accumulation and release of upstream isoprenoid lipids such as IPP. Aminobisphosphonate compounds had been used in human therapy for the treatment of bone metastases in cancer patients, and provided a first set of evidence for in vivo expansion of human Vγ9/Vδ2 $^+$ lymphocytes induced by phosphoantigen agonists, reporting increases of circulating γδ T cells within one to three weeks in human adults with multiple myeloma after therapeutic intravenous injection of 60-90 mg of pamidronate (Kunzmann et al, 1999). However, such compounds require presentation by antigen presenting cells and cannot produce substantial stimulation of Vγ9/Vδ2 T cell activity as assessed by

cytokine secretion in a pure Vγ9/Vδ2 T cell culture. Moreover, pamidronate shows very low potency of activation of γδ T cells, reported to achieve at best only 2-fold increase in γδ T cell count (Wilhelm et al., 2003).

*High specific activity phosphoantigens*

Recently, several highly potent γδ T cell activating pyrophosphate-containing compounds have been described which directly activate γδ T cells. In particular, phosphalyhydrin and phosphoepoxyde compounds were described by the group of J.J. Fournie. (R, S)-3-(bromomethyl)-3-butanol-1-yl-diphosphate, also referred to as BrHPP (BromoHydrin PyroPhosphate) is currently used in ongoing human clinical studies to stimulate the proliferation of γδ T cells ex vivo. Other pyrophosphate containing compounds with high specific activity (EC50 in the nanomolar or better range) are produced through an isoprenoid biosynthetic pathway called the 'Rohmer" or "nonmevalonate" pathway, which is specific to pro- and eukaryotic microorganisms (Feurle et al., 2002; Jomaa et al (2003); Jomaa et al., 1999a; Jomaa et al., 1999b; Rohmer et al., 1993).

In contrast to aminobisphosphonates and statins discussed above, high specific activity phosphoantigen compounds such as the compounds of formula I to formula XVII are capable of regulating Vγ9/Vδ2 T cell activity in a population of Vγ9/Vδ2 T cell clones in culture at millimolar concentrations, where regulation is assessed by monitoring cytokine secretion. While the precise mode of recognition of phosphoantigens remains unclear, a direct contribution of the Vγ9/Vδ2 TCR to phosphoantigen-mediated activation has been demonstrated by gene transfer experiments (Bukowski et al., 1995). Accordingly recent structural data drawn from crystallographic analysis of Vγ9/Vδ2 TCR are compatible with cognate interactions between phosphoantigens and γδ TCR, through electrostatic interactions between the negatively charged phosphate residues on the antigen side with several positively charged amino-acids on the TCR side (Allison et al., 2001).

*Methods of treatment involving administration of γδ T cell activating compounds*

Despite the foregoing, studies of phosphoantigens including the synthesis and in vitro testing of analogs from a variety of groups of compounds indicate structures providing high γδ T cell activation, in particular compounds according to formula I described herein. However, no methods or treatment regimens have been proposed for the use of phosphoantigens with high specific activity in vivo. Accordingly, no methods or treatment regimens have been proposed for strategies involving an in vivo stimulation sufficient to generate a large increase in γδ T cell activity.

In one aspect, research into treatment regimens based on γδ T cell activating compounds has been hampered by the lack of suitable in vivo models. Evidence for a general immune surveillance function of the innate immune system has been provided by various *in vivo* models: mice deficient in innate effector cells such as NK cells, NKT cells or γδ T cells show a significantly increased incidence of tumors (Girardi et al., 2001; Kim et al., 2000; Smyth et al., 2000). However, such results can only be transposed to the human situation with caution, as these cell populations are somewhat different in humans as compared to mice. In particular, the human Vγ9/Vδ2 cell population for example does not have a formal equivalent in rodents.

In view of the foregoing, although several compounds have been shown to have in vitro activity, their in vivo activity and more generally the in vivo kinetics of γδ T cells in response to stimulation had not been explored. Accordingly, efficient methods are needed to selectively activate γδ T cells in vivo, in a subject, under conditions suitable for therapy.

Furthermore, no therapeutic strategy involving stimulating a manifold increase of circulating γδ T cells in vivo had been developed for the treatment of tumors, and in particular for solid tumors, especially those with metastases. The safety and efficacy of treatments for tumors can be altered by a variety of factors, and treatments can be affected by tumor growth kinetics, drug resistance of tumor cells, total-body tumor cell burden, toxic effects of therapy on cells and tissues other than the tumor, and distribution of therapeutic agents within the tissues of the patient. The greater the size of the primary tumor, the greater the probability that a large number of cells (drug resistant and drug sensitive) have metastasized before diagnosis and that the patient will relapse. Solid tumors and carcinomas account for more than 90% of all cancers in man, and although the use of monoclonal antibodies and immunotoxins has been investigated in the therapy of lymphomas and leukemias, many such agents have been disappointingly ineffective in clinical trials against carcinomas and other solid tumors. One possible reason for the ineffectiveness of effector-cell-based treatments is that cells are not readily transported into solid tumors. Alternatively, even once within a tumor mass, these cells may fail to distribute evenly due to the presence of tight junctions between tumor cells, fibrous stroma, interstitial pressure gradients and binding site barriers.

## SUMMARY OF THE INVENTION

The present invention now discloses particular compositions to be used to efficiently regulate the activity of γδ T cells, particularly the activation and proliferation of γδ T cells, in vivo in a subject. These uses are particularly suited

for immuno-therapy in a subject, namely in a subject having a solid tumor and are defined in claim 1.

The uses provided herein by the inventors are based on a series of results. In one aspect, a therapeutic strategy using autologous γδ T cells activated ex-vivo by a high specific activity pyrophosphate compound shows indications of anti-tumor activity in human patients in an ongoing clinical study using ex-vivo stimulated γδ T cells for the treatment of metastatic renal cell carcinoma. In another aspect, invention is based on a series of findings resulting from the first known experiments in animals involving regulating the activity of γδ T cells, including both in vivo increase of the biological activity of γδ T cells as well as manifold expansion of the γδ T cell population. Furthermore, in a novel Nod-Scid murine model adapted for the assessment of γδ T cell activation and γδ T cell mediated anti-tumor activity, it has been found that high potency γδ T cell activating compounds administered to the animal can regulate γδ T cell activity in vivo, that γδ T cells can infiltrate solid tumors, and moreover that such treatment is effective in decreasing the mass of solid tumors, and more particularly metastatic tumors. The anti-tumoral effect of γδ T cell activator-stimulate γδ T cells was also observed toward fresh cells in culture obtained from human patients having metastatic solid tumors, but was not observed towards non-tumoral cells from the same patients. Based on such discoveries, the inventors have devised therapies for solid tumors using compounds capable of regulating the activity of γδ T cells.

In further experiments, in vivo kinetics of high specific activity γδ T cell activators was determined.

More specifically, the elucidation of in vivo γδ T cell kinetics resulted in the following findings, among others:

(a) that the activity of γδ T cells may be regulated repeatedly, including activating cells as demonstrated by cytokine release and expansion of the cell population, using a γδ T cell activator, depending on the administration regimen, and that certain intervals of drug administration provide for optimal re-stimulation of γδ T cell activity;
(b) that addition of a cytokine in an administration regimen, particularly IL-2, and more particularly certain doses of IL-2 provide improved in vivo expansion of γδ T cells
(c) methods for translating in vitro activity to in vivo dosage regimens for γδ T cell activator capable of increasing γδ T cell activity;
(d) specific dosage and administration regimens allowing the increase of γδ T cell activity using γδ T cell activators.

In one aspect, the invention discloses a treatment of solid a tumor, said method comprising the step of administering, in at least one treatment, a therapeutically effective amount of a γδ T cell activator, together with a pharmaceutically acceptable carrier, to a warm-blooded animal in need of such treatment. Also provided is a treatment of a solid tumor, said treatment comprising the step of administering, in at least one treatment, a therapeutically effective amount of a γδ T cell activator, together with a pharmaceutically acceptable carrier, to a warm-blooded animal having a solid tumor with metastases in need of such treatment. Said treatments of tumor can be carried out in any suitable fashion. Preferably said treatments comprise the step of contacting a γδ T cell in a warm-blooded animal having a solid tumor, with a therapeutically effective amount of a γδ T cell activator, or optionally said treatments comprise the step of providing in the bloodstream of a warm-blooded animal having a solid tumor a therapeutically effective amount of a γδ T cell activator. In a particular embodiment, said tumor is a solid tumor with metastases. Preferably, said tumor is selected from the group consisting of lung, colorectal, prostate, breast or epidermoid head or neck tumors. In a preferred aspect of the invention, said tumor is a renal cancer, preferably a metastatic renal cancer. Alternatively, said tumor is selected from the group consisting of a melanoma, ovarian cancer, pancreas cancer, neuroblastoma, head or neck cancer, bladder cancer, renal cancer, brain cancer and gastric cancer. Preferably, said treatment comprises at least two treatments. Preferably, said γδ T cell activator is administered with an interval of about two weeks to about eight weeks between treatments, more preferably with an interval of about three to about four weeks between treatments. Optionally, at least three, four or six treatments are administered to said animal. Preferably, the biological activity of γδ T cells are increased in said warm-blooded animal. Preferably, the number of circulating γδ T cells are increased in said warm-blooded animal. In a particular embodiment, the amount of said γδ T cell activator is sufficient to expand the γδ T cell population in a subject to reach at least 30%, 40%, 50% or 60%, or between 30-90% of total circulating lymphocytes. In an other particular embodiment, the amount of said γδ T cell activator is sufficient to induce an at least 10-fold increase in the γδ T cell population in a subject. Preferably, said γδ T cell population is assessed between day 4 and day 8 following administration of said γδ T cell activator, more preferably at day 5, 6 or 7 following administration of said γδ T cell activator. Preferably, said γδ T cell population is assessed by flow cytometry. Preferably, said γδ T cells are Vγ9/Vδ2 T cells. Optionally, said γδ T cell activator is administered by intravenous infusion, preferably said infusion takes place during about 5 to about 120 min, more preferably during about 5 to about 30 min. In further preferred aspects, the methods may comprise further administering a cytokine, preferably IL-2.

In another aspect, disclosed is a treatment of a solid tumor disease in a warm-blooded animal, said treatment comprising administering, in more than one treatment, a therapeutically effective amount of a γδ T cell activator, together with a pharmaceutically acceptable carrier, to a warm-blooded animal in need of such treatment, wherein the γδ T cell activator is administered in more than one treatment with an interval of about two weeks to about eight weeks between treatments.

Also disclosed is a method for stimulating a γδ T cell in a warm-blooded animal, said method comprising: (a) contacting a γδ T cell in a warm-blooded animal with a therapeutically effective amount of a γδ T cell activator; and (b) repeating step (a) at least once within about two weeks to about eight weeks after said contacting in step (a). Also disclosed is a method for stimulating a γδ T cell in a warm-blooded animal, said method comprising: (a) providing in the bloodstream of a warm-blooded animal a therapeutically effective amount of a γδ T cell activator; and (b) repeating step (a) at least once within about two weeks to about eight weeks after said contacting in step (a). Optionally, step (b) is said methods may comprise repeating step (a) at least twice, at least three times, at least four times or at least six times.

The inventors also disclose a method for treating a solid tumor, said method comprising the step of contacting a γδ T cell in a warm-blooded animal having a solid tumor with a γδ T cell activator in an amount sufficient to expand the γδ T cell population in a subject to reach at least 30%, 40%, 50% or 60%, or between 30-90%, of total circulating lymphocytes. Also provided is method for treating a solid tumor, said method comprising the step of providing in the bloodstream of a warm-blooded animal having a solid tumor a γδ T cell activator in an amount sufficient to expand the γδ T cell population in a subject to reach at least 30%, 40%, 50% or 60%, or between 30-90%, of total circulating lymphocytes. Preferably the γδ T cell population is assessed between day 4 and day 8, most preferably at about day 5, day 6 or day 7, following administration of the a γδ T cell activator.

One aspect of the invention is to increase the γδ T cell population. The invention encompasses a method for treating a solid tumor, said method comprising the step of contacting a γδ T cell in a warm-blooded animal having a solid tumor with a γδ T cell activator in an amount sufficient to induce an at least 10-fold increase in the γδ T cell population in a subject. Also encompassed is a method for treating a solid tumor, said method comprising the step of providing in the bloodstream of a warm-blooded animal having a solid tumor a γδ T cell activator in an amount sufficient to induce an at least 10-fold increase in the γδ T cell population in a subject compared to the level prior to treatment. Preferably the γδ T cell population is assessed between day 4 and day 8, most preferably at about day 5, day 6 or day 7, following administration of the a γδ T cell activator. Preferably the γδ T cell population is assessed by flow cytometry.

Also encompassed by the invention is the use of an γδT activator for the manufacture of a pharmaceutical preparation for the treatment of a solid tumor, comprising admixing said γδT activator with a pharmaceutically acceptable carrier, said pharmaceutical preparation being administering to said subject. In a particular embodiment, said tumor is a solid tumor with metastases. Optionally, said tumor is a metastatic tumor. Preferably, said tumor is selected from the group consisting of lung, colorectal, prostate, breast or epidermoid head or neck tumors. In a preferred aspect of the invention, said tumor is a renal cancer, preferably a metastatic renal cancer. Alternatively, said tumor is selected from the group consisting of a melanoma, ovarian cancer, pancreas cancer, neuroblastoma, head or neck cancer, bladder cancer, renal cancer, brain cancer and gastric cancer. Preferably, said pharmaceutical preparation is administered at least twice, more preferably with an interval of about two weeks to about eight weeks between treatments, still more preferably with an interval of about three to about four weeks between treatments. Optionally, said pharmaceutical preparation is administered is administered at least three, four or six times.

Preferably, said pharmaceutical preparation increases the biological activity of γδ T cells in said subject. Preferably, said pharmaceutical preparation increases the number of circulating γδ T cells in said subject. In a particular embodiment, the amount of said γδ T cell activator is sufficient to expand the γδ T cell population in a subject to reach between 30-90% of total circulating lymphocytes. In an other particular embodiment, the amount of said γδ T cell activator is sufficient to induce an at least 10-fold increase in the γδ T cell population in a subject. Preferably, said γδ T cell population is assessed between day 4 and day 8 following administration of said γδ T cell activator, more preferably at day 7 following administration of said γδ T cell activator. Preferably, said γδ T cell population is assessed by flow cytometry. Preferably, said γδ T cells are Vγ9/Vδ2 T cells. Optionally, said pharmaceutical preparation is administered by intravenous infusion, preferably said infusion takes place during about 5 to about 120 min, more preferably during about 5 to about 30 min. In further preferred aspects, the methods may comprise further administering a cytokine, preferably IL-2.

In one aspect of the methods of the invention, a γδ T cell activator is administered in the absence of administration of a cytokine. In other aspects the methods if the invention comprise the use of a γδ T cell activator and an interleukin-2 polypeptide, for the manufacture of a pharmaceutical composition for regulating the activity of γδ T cells in a mammalian subject, the γδ T cell activator and interleukin-2 polypeptide being administered separately to the subject.

In preferred aspects of the any of the methods described herein, at least two, three, four or six treatments are administered to said animal. In preferred aspects of any of the methods described herein, the γδ T cell activator is administered in more than one treatment with an interval of about two to about eight weeks between treatments, or yet more preferably about three to about four weeks between treatments.

In any of the method described herein, the warm- blooded animal in the present methods may be a rodent or a non-human primate. In preferred aspects, the warm blooded animal in the present methods is a human.

In preferred aspects of any of the aspects of the present invention, the methods result in an increase in the biological activity of γδ T cells said warm-blooded animal or said subject, or an increase in the number of circulating γδ T cells in said warm-blooded animal or said subject. Preferably, the γδ T cells referred to in the methods of the invention are Vγ9/Vδ2 T cells.

As described in the examples, the methods of the invention may be used for the treatment of a solid tumor as well as a solid tumor with metastases. Preferably the γδ T activator is administered to a human in need of such treatment in a dose that is appropriate for the treatment of said disease. However, preferred and particularly effective doses are further provided herein. Optionally, in any of the methods described herein, a γδT activator is administered in a dose that is greater than the EC50 human dose; and one or more further doses each greater than the EC50 human dose are administered in at least one additional treatment after an interval between the treatments of two to eight weeks.

In any of the methods provided herein, the invention provides further administering a cytokine. In preferred aspects of any of the methods described herein, the cytokine is IL-2. Preferably, the interleukin-2 polypeptide is administered at low doses. In further aspects of any of the methods of the invention, the cytokine, and most preferably an interleukin-2 polypeptide, is administered over a period of time comprised between 1 and 10 days. Preferably, the interleukin-2 polypeptide is administered at a daily dose comprised between 0.2 and 2 MU per day, even more preferably between 0.2 and 1.5 MU, further preferably between 0.2 and 1 MU. The daily dose of cytokine, preferably an interleukin-2 polypeptide, is administered as a single injection or in two injections. Preferably the γδ T cell activator is administered as a single dose at the beginning of the treatment.

In preferred aspects, provided is a method for stimulating a γδ T cell in a subject, or a method of treating a cancer, in a subject, comprising: separately administering to a subject in need thereof an effective amount of a γδT activator and a cytokine, preferably an interleukin-2 polypeptide, over a period of time comprised between 1 and 10 days. Preferably the γδ T cell activator is administered as a single dose at the beginning of the treatment. Preferably the γδ T cell activator is administered as a single dose at the beginning of the treatment and the cytokine, preferably IL-2, is administered on at least two, three, four or five days within the ten day period following administration of the γδ T cell activator. The invention also encompasses a product comprising a γδ T cell activator and an interleukin-2 polypeptide, for separate use, for regulating the activity of γδ T cells in a mammalian subject.

The invention further concerns the use of a γδ T cell activator and an interleukin-2 polypeptide, for the manufacture of a pharmaceutical composition for regulating the activity of γδ T cells in a mammalian subject, the γδ T cell activator and interleukin-2 polypeptide being administered separately to the subject. Preferably, said γδT activator is a synthetic γδT activator. Preferably, said interleukin-2 polypeptide is administered at low doses. Preferably, said interleukin-2 polypeptide is administered over a period of time comprised between 1 and 10 days. More preferably, said interleukin-2 polypeptide is administered at a daily dose comprised between 0.2 and 2 MU per day, even more preferably between 0.2 and 1.5 MU, further preferably between 0.2 and 1 MU. Optionally, said daily dose of interleukin-2 polypeptide is administered as a single injection or in two injections. Preferably, said γδ T cell activator is administered as a single dose at the beginning of the treatment. Optionally, said γδ T cell activator is a ligand of the T receptor of γδ T lymphocytes. Preferably, said γδ T cell activator is administered at least twice, more preferably with an interval of about two weeks to about eight weeks between treatments, still more preferably with an interval of about three to about four weeks between treatments. Optionally, said γδ T cell activator is administered is administered at least three, four or six times. Preferably, said pharmaceutic composition increases the biological activity of γδ T cells in said subject. Preferably, said pharmaceutic preparation increases the number of circulating γδ T cells in said subject. Preferably, said γδ T cell activator is administered in an amount sufficient to expand the γδ T cell population in a subject to reach between 30-90% of total circulating lymphocytes. Optionally, said γδ T cell activator is administered in an amount sufficient to induce an at least 10-fold increase in the γδ T cell population in a subject. Preferably, said γδ T cells are Vγ9/Vδ2 T cells. Optionally, said γδ T cell activator is administered by intravenous infusion, preferably said infusion takes place during about 5 to about 120 min, more preferably during about 5 to about 30 min. In a particular embodiment, said pharmaceutical composition is used for treating a solid tumor. In a particular embodiment, said cancer is a solid tumor with metastases. Optionally, said cancer is a metastatic tumor. Preferably, said cancer is selected from the group consisting of lung, colorecta1, prostate, breast or epidermoid head or neck tumors. In a preferred aspect of the invention, said cancer is a renal cancer, preferably a metastatic renal cancer. Alternatively, said cancer is selected from the group consisting of a melanoma, ovarian cancer, pancreas cancer, neuroblastoma, head or neck cancer, bladder cancer, renal cancer, brain cancer and gastric cancer.

The invention concerns a method of treating a solid tumour in a subject, comprising separately administering to a subject in need thereof an effective amount of a γδT activator and an interleukin-2 polypeptide. Preferably, said γδT activator is a synthetic γδT activator. Preferably, said interleukin-2 polypeptide is administered at low doses. Preferably, said interleukin-2 polypeptide is administered over a period of time comprised between 1 and 10 days. More preferably, said interleukin-2 polypeptide is administered at a daily dose comprised between 0.2 and 2 MU per day, even more preferably between 0.2 and 1.5 MU, further preferably between 0.2 and 1 MU. Optionally, said daily dose of interleukin-2 polypeptide is administered as a single injection or in two injections. Preferably, said γδ T cell activator is administered as a single dose at the beginning of the treatment. Optionally, said γδ T cell activator is a ligand of the T receptor of γδ T lymphocytes. In a particular embodiment, said γδ T cell activator is a PED or PHD compound and is administered as a single injection at a dose comprised between 10 and 50 mg/kg, at the beginning of the treatment, and wherein said interleukin-2 polypeptide is administered at a daily dose comprised between 0.2 and 2 MU per day over a period of time comprised between 1 and 10 days. Preferably, said γδ T cell activator is administered at least twice, more preferably

with an interval of about two weeks to about eight weeks between treatments, still more preferably with an interval of about three to about four weeks between treatments. Optionally, said γδ T cell activator is administered is administered at least three, four or six times. Preferably, said pharmaceutic composition increases the biological activity of γδ T cells in said subject. Preferably, said pharmaceutic preparation increases the number of circulating γδ T cells in said subject. Preferably, said γδ T cell activator is administered in an amount sufficient to expand the γδ T cell population in a subject to reach between 30-90% of total circulating lymphocytes. Optionally, said γδ T cell activator is administered in an amount sufficient to induce an at least 10-fold increase in the γδ T cell population in a subject. Preferably, said γδ T cells are Vγ9/Vδ2 T cells. Optionally, said γδ T cell activator is administered by intravenous infusion, preferably said infusion takes place during about 5 to about 120 min, more preferably during about 5 to about 30 min. Preferably, said cancer is selected from the group consisting of lung, colorectal, prostate, breast or epidermoid head or neck tumors. In a preferred aspect of the invention, said cancer is a renal cancer, preferably a metastatic renal cancer. Alternatively, said cancer is selected from the group consisting of a melanoma, ovarian cancer, pancreas cancer, neuroblastoma, head or neck cancer, bladder cancer, renal cancer, brain cancer and gastric cancer.

A number of suitable γδ T cell activators are provided herein that can be used in accordance with any of the methods described herein. Preferably a γδ T cell activator is a compound capable of regulating the activity of, or preferably of inducing the proliferation of a γδ T cell in a pure population of γδ T cell clones, preferably Vγ9/Vδ2 T cells. More preferably, the γδ T cell activator is a compound capable of regulating the activity of a γδ T cell in a population of γδ T cell clones when the γδ T cell activator is present in culture at a concentration of less than 100 mM, less than 10 mM or most preferably less than 1 mM. Further preferred γδ T cell activators are described in detail herein.

Particularly preferred γδ T cell activators comprise a composition comprising a compound of formula (II):

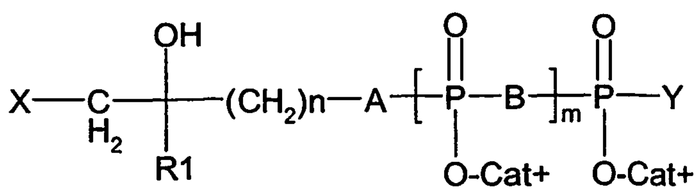

(II)

in which X is an halogen (preferably selected from I, Br and Cl), B is O or NH, m is an integer from 1 to 3, R1 is a methyl or ethyl group, Cat+ represents one (or several, identical or different) organic or mineral cation(s) (including the proton), and n is an integer from 2 to 20, A is O, NH, CHF, $CF_2$ or $CH_2$, and Y is $O^-$Cat+, a nucleoside, or a radical -A-R, wherein R is selected from the group of 1), 2) or 3). Preferably, Y is $O^-$Cat+, or a nucleoside. More preferably, Y is $O^-$Cat+. Preferably, R1 is a methyl. Preferably, A is O or $CH_2$. More preferably, A is O. Preferably, n is 2. Preferably, X is a bromide. Preferably, B is O. Preferably, m is 1 or 2. More preferably, m is 1. Preferably such a compound is administered in a dose to humans that is between about 10 mg/kg and about 100 mg/kg, preferably between about 5 mg/kg and about 60 mg/kg, together with a pharmaceutically acceptable carrier. In other preferred aspects such compound is administered in two or more treatments; preferably the compound is administered in a dose to humans that is calculated according to the formula (A) single dose (mg/kg)=(10 to y) * N (A) where N is the number of weeks between treatments and y is 100; more preferably in a dose to humans that is calculated according to the formula B single dose (mg/kg)=(5 to 60) * N (B); or preferably in a dose of about 20 mg/kg. Yet more preferably, said γδT activator is administered by intravenous infusion in a dose to humans that is calculated according to the formula (C) single dose (mg/kg)=(10 to 100) * N (C) where N is the number of weeks between treatments such that N is between about 3 and about 4.

In preferred aspects of any of the methods described herein, the γδT activator is a synthetic γδT activator.

In other preferred aspects of any of the methods described herein, the γδ T cell activator is selected from phosphohalohydrin (PHD) compounds, phosphoepoxyde (PED) compounds, and alkylamines. More preferably, the γδ T cell activator is selected from the following compounds:

3-(bromomethyl)-3-butanol-1-yl-diphosphate (BrHPP)
3-(iodomethyl)-3-butanol-1-yl-diphosphate (IHPP)
3-(chloromethyl)-3-butanol-1-yl-diphosphate (ClHPP)
3-(bromomethyl)-3-butanol-1-yl-triphosphate (BrHPPP)
3-(iodomethyl)-3-butanol-1-yl-triphosphate (IHPPP)
α,γ-di-[3-(bromomethyl)-3-butanol-1-yl]-triphosphate (diBrHTP)
α,γ-di-[3-(iodomethyl)-3-butanol-1-yl]-triphosphate (diIHTP)
3,4,-epoxy-3-methyl-1-butyl-diphosphate (Epox-PP)
3,4,-epoxy-3-methyl-1-butyl-triphosphate (Epox-PPP)

α,γ-di-3,4,-epoxy-3-methyl-1-butyl-triphosphate (di-Epox-TP)

In other aspects, the γδ T cell activator is a PED or PHD compound and is administered as a single injection at a dose comprised between 10 and 50 mg/kg, at the beginning of the treatment, and wherein the interleukin-2 polypeptide is administered at a daily dose comprised between 0.2 and 2 MU per day over a period of time comprised between 1 and 10 days.

In further aspects, the invention provides a method for stimulating a γδ T cell in a warm-blooded animal, said method comprising administering, in more than one treatment, a composition comprising a compound of formula XII, together with a pharmaceutically acceptable carrier, to a warm-blooded animal, wherein the γδ T cell activator is administered in more than one treatment with an interval of about two weeks to about eight weeks between treatments.

In further aspects, the invention provides a method for stimulating a γδ T cell in a warm-blooded animal, said method comprising administering, in more than one treatment, a composition comprising a compound of formula II, together with a pharmaceutically acceptable carrier, to a warm-blooded animal, wherein the γδ T cell activator is administered in more than one treatment with an interval of about two weeks to about eight weeks between treatments.

In another aspect of the invention, disclosed is a method for stimulating a γδ T cell in a human subject, said method comprising administering a composition comprising a HDMAPP compound in a dose that is between about 10 μg/kg and about 2.5 mg/kg, together with a pharmaceutically acceptable carrier, to the subject. In further aspects, the invention provides a method for stimulating a γδ T cell in a human subject, said method comprising administering a composition comprising a CHDMAPP compound in a dose that is between about 10 μg/kg and about 2.5 mg/kg, together with a pharmaceutically acceptable carrier, to the subject.

In further aspects, the invention provides a method for stimulating a γδ T cell in a human subject, said method comprising administering a composition comprising a CBrHPP compound in a dose that is between about 5 mg/kg and about 60 mg/kg, together with a pharmaceutically acceptable carrier, to the subject.

The present invention also provides method for administering a γδ T cell activator to a subject. Thus, in any methods of the invention, said methods may comprise administering the γδ T cell activator by intravenous infusion. Preferably, each infusion takes place during about 5 to about 120 min, or more preferably during about 5 to about 30 min. Most preferably, only a single such infusion (also referred to as single shot) of the γδ T cell activator takes place on any given day (e.g. within a period of less than 24 hours). Preferably only a single infusion takes place in an administration of a γδ T cell activator, that is, per treatment cycle.

In one particularly preferred aspect, the invention relates to a method of regulating γδ T cells in a human subject, the method comprising: administering, preferably by intravenously infusing, a composition comprising a compound of formula XII into said subject a dose of between 10 μg/kg to 20 mg/kg, more preferably between 10 μg/kg to 5 mg/kg or yet more preferably between 10 μg/kg to 1 mg/kg, of said compound per kilogram of the subject's weight, preferably in a single administration (single shot), and preferably when by infusion within a period of less than 24 hours. In one particularly preferred aspect, the invention relates to a method of regulating Vγ9/Vδ2$^+$ T cells in a human subject, the method comprising: administering, preferably by intravenously infusing, a composition comprising a HDMAPP or CHDMAPP compound (of formula XV and XVI respectively) into said subject a dose of between 10 μg/kg to 20 mg/kg, more preferably between 10 μg/kg to 5 mg/kg or yet more preferably between 10 μg/kg to 1 mg/kg, of said compound per kilogram of the subject's weight, preferably in a single administration (single shot), and preferably when by infusion within a period of less than 24 hours.

## DESCRIPTION OF THE DRAWINGS

**Figure 1**

Successive intravenous injections of increasing doses of BrHPP do not sustain γδ cell amplification in the periphery of M. fascicularis. Two animals received successively 1, 4, 16 and 32mg/kg BrHPP daily and their PBMCs were monitored by flow cytometry. Total blood was stained with anti-delta2-FITC and anti-CD3-PE antibodies until 20 days after BrHPP administration.

**Figure 2**

**A- BrHPP and IL2 co-administration induces reproducible γδ cell increase in *M. fascicularis*.**

Two animals received 20mg/kg BrHPP ("1 dose") and two others received 4mg/kg BrHPP daily during 5 days ("split doses"). All of them were co-injected with 0.9 million UI IL2 per day for 5 days. The four BrHPP/IL2 co-treated animals showed an increase in peripheral γδ cells, with a peak at day 7. As a control, a fifth animal was treated with IL2 alone and exhibited no change in its peripheral γδ rate. A BrHPP/IL2 co-administration to this

animal at day 14 demonstrated it was able to answer with the same increase in γδ rate 7 days after.

**B- Flow cytometry analyses of total blood of *M. fascicularis* at day 7 after administration.**
These graphs represent the anti-delta2-FITC/anti-CD3-PE staining on total blood of the animals treated with IL2 alone (Z604 D7 IL2 only) or with BrHPP and IL2 (Z604, X973, Z059, Z135, Z714, D7 IL2+BrHPP) 7 days after the administration.

**C- Absolute number of circulating γδ cells increases 5- to 40-fold in BrHPP/IL2 co-injected *M. fascicularis*.** Absolute numbers of peripheral γδ cells were determined by flow cytometry in the four BrHPP/IL2 co-injected animals. Peripheral γδ cell number was found 5 to 40 times higher at day 7 after administration than before, depending on animals. A mean 24-fold increase was obtained for a total amount of 20mg/kg BrHPP injected, single doses being more efficient than split ones.

**Figure 3**

**A Peripheral γδ cell rate increase upon BrHPP/IL2 administration is dose-dependant.**
**Upper panel** : A first injection was done with increasing amounts (0, 0.2, 4, 20, 80 mg/kg) of BrHPP in 10 animals (numbered 2031 to 2040), with 2 animals per dose (1 male + 1 female). **Lower panel** : Same animals underwent a second injection 3 weeks after the first injection with even higher amounts of BrHPP (20, 80, 120, 160 mg/kg). Animals that received the lowest doses at first injection (groups 0.2 and 4 mg/kg) were injected with the two new highest doses (120 and 160 mg/kg) to minimize potential effects of first injection on second injection.

**B- Circulating γδ cell number increase upon BrHPP/IL2 co-treatment is also dose-dependant.**
The fold increase in peripheral γδ cell number was calculated as the ratio of γδ absolute count 7 days after treatment on γδ absolute count before treatment. Despite some discrepancy between animals in the same dose-group, this increase is clearly dose-dependant.

**C- Flow cytometry images of total blood of animal #2034, before and 5 days after it received 160mg/kg BrHPP,** stained with anti-gamma9-FITC and anti-CD3-PE antibodies.

**Figure 4**

**A- Primate γδ cells respond *in vivo* equally to the first and the second BrHPP/IL2 co-**treatment. The mean amplification in γδ cell number observed 7 days after the first 20mg/kg BrHPP treatment (fractionated or not) is comparable to the mean increase observed after the 20mg/kg BrHPP recall.

**B- γδ amplification *in vivo* decreases after successive BrHPP/IL2 administration.** In this experiment, two animals received successively 80mg/kg and 22 days after 20mg/kg, or 20mg/kg followed by 80mg/kg. In each case, the response in γδ cell number fold increase is lower at the first recall of a given dose than at first injection (left panel). Moreover, the five females were treated with a second and a third recall at 80mg/kg, at 3 weeks intervals. The resulting amplification rates is still detectable but become lower at each recall (right panel).

**Figure 5**

**A- Serum TNFα is produced *in vivo* after BrHPP/IL2 co-treatment.** TNFα was detected by Elisa in the serum of the monkeys first treated with IL2 and increasing doses of BrHPP (0 to 80mg/kg, with two animals per dose, Cf figure 3A), 1 and 4 hours after BrHPP injection.

**B- *In vivo* peripheral BrHPP-amplified primate γδ cells produce cytokines in response to BrHPP** challenge. As a fourth BrHPP/IL2 treatment, females 2032 and 2034 received 80mg/kg BrHPP, which induced 3- and 7.9-fold increases in γδ cell number respectively. At the time of γδ cell peripheral peak (day 5 after the 4$^{th}$ injection), these animals were re-injected with 80mg/kg BrHPP (without IL2) and serum INFγ and TNFα were detected 60 and 120 minutes after injection by Elisa.

**Figure 6**

**Different IL2 co-treatment do not influence the maintenance of peripheral γδ rate.**
Concomitantly with 20mg/kg BrHPP, animals received subcutaneously the following IL2 treatment : 0.15 million units twice daily for 9 days (animal Z059), 0.3 million units twice daily for 5 days (Z135), 0.9 million units twice daily for 5 days (animal Z714) or 9 days (animal X973).

**Figure 7**

**Primate γδ cell number fold increase in response to increasing doses of Phosphostim.**
The first and second administrations of Phosphostim and IL-2 resulted in a clear dose-related elevation of peripheral

Vγ9Vδ2 T cells at Day 7 as determined by flow cytometry, which is represented in Figure 7.

**Figure 8**

**Serum cytokine (INFγ and TNFα) production in Phosphostim treated primates.**

The effects of Phosphostim treatment on the production of cytokines (INFγ and TNFα) production in the serum was studied twice:

- after the first infusion, in all treated animals: a significant production of systemic TNFα (serum concentrations around 60 and 120 pg/ml) was evidenced in both animals having received 80 mg/kg, 1 hour after BrHPP injection;
- in two females (F2032 & F2034), which received 80 mg/kg (without IL-2) during the peak (Day 7) of the $4^{th}$ injection.

Serum TNFα and INFγ concentration evolutions for both animals are shown, demonstrating that Vγ9Vδ2 T cells amplified *in vivo* upon BrHPP/IL-2 co-treatment also produce detectable amounts of systemic cytokines.

**Figure 9**

**In vitro cytotoxicity of expanded Vγ9Vδ2 T cells from mRCC patients.**

Lytic activities of BrHPP-amplified γδ T cells were measured against classical control targets (Raji and Daudi), primary autologous normal and tumor cell lines of the selected patients in a 4h standard $^{51}Cr$ release assay for the five patients.

**Figures 10 to 13**

**In vivo (monkey) dose ranging studies with HDMAPP and BrhPP**

The dose-range effect of HDMAPP and BrHPP in vivo as determined by determining numbers of γδ T cells by flow cytometry are shown in Figures 10 to 13.

Figures 10A and 10B show the absolute cell count (/$mm^3$ blood) for HDMAPP and BrHPP respectively.
Figures 11A and 11B show the percentage γδ T cells of total circulating lymphocytes for HDMAPP and BrHPP respectively.
Figures 12A and 12B show the fold γδ T cell increase in absolute cell count (/$mm^3$ blood) for HDMAPP and BrHPP respectively.
Figures 13A and 13B show the fold increase in percentage of total circulating lymphocytes for HDMAPP and BrHPP respectively.

**Figure 14**

**A. Comparison of BrHPP and HDMAPP in vitro activities**

Figure 14A shows the in vitro EC50 for the compounds BrHPP, HDMAPP and the aminobisphosphonate compound Zoledronate®. The in vitro biological activity of γδ T cell amplification from human PBMCs (in the presence of rhIL2) was assessed using a TNFα release assay.

**B. Comparison of BrHPP and HDMAPP in vivo activities**

Figure 14B shows the in vivo EC50 for the compounds: for BrHPP the EC50 is about 1 nM while for HDMAPP the EC50 is about 5 pM. By contrast, the less potent Zoledronate® showed an EC50 value of about 1 μM. The biological activity of γδ T cell amplification from human PBMCs was assessed by counting γδ T cell using flow cytometry.

**Figures 15 to 19**

**In *vivo* efficacy of human γδ T cells Nod-Scid/Tumor Model**

Tumor growth in the first few days after administration of human PBML and BrHPP treatment to a Nod-Scid mouse is shown in Figure 15.

Phenotyping and homing of human cells: In the peritoneal cavity: the weekly check of the IP phenotype of treated mice showed a human γδ T cell presence only in the BrHPP treated mice. The relative numbers of γδ T cells in the blood is shown in Figure 16 and in the peritoneal cavity in Figure 17. Human γδ T cells represent a higher percentage of the human CD3 T cells. In the mice recipient organs: phenotyping is carried out at sacrifice (4 weeks after the PBMC and BrHPP treated or not treated groups); the major human cells present in those organs are human CD3+ T cells with 99% expression of the Ab TcR. However in the tumor, human γδ cells were present only in the BrHPP treated group.

While tumor size increased in the first few days after treatment, tumor size decreased therafter quickly in the PBMC/BrHPP and IL2 treated group. Figure 19 shows that from day 7 onwards the tumor size shrank. In the PBMC group, after short arrest, the size grows and no significant difference was observed between the tumors sizes in this group and those of the negative control.

## DETAILED DESCRIPTION

*Definitions*

Within the context of the present invention, the expression "regulating the activity of γδ T cells" designates causing or favoring an increase in the number and/or biological activity of such cells in a subj ect. Regulating thus includes without limitation modulating (e.g., stimulating) expansion of such cells in a subject and/or, for instance, triggering of cytokine secretion (e.g., TNFα or IFNγ). As indicated, γδ T cells normally represent between about 1-10% of total circulating lymphocytes in a healthy adult human subj ect. The present invention can be used to significantly increase the γδ T cells population in a subject, particularly to reach 30-90% of total circulating lymphocytes, typically 40-90%, more preferably from 50-90%. In typical embodiments, the invention allows the selective expansion of γδ T cells in a subject, to reach 60-90% of total circulating lymphocytes, preferably 70-90%, more preferably from 80-90%. Regulating also includes, in addition or in the alternative, modulating the biological activity of γδ T cells in a subject, particularly their cytolytic activity or their cytokine-secretion activity. The invention defines novel conditions and strategies for increasing the biological activity of γδ T cells towards target cells.

As used herein, the term"EC50" with respect to regulating the activity of γδ T cells, refers to the efficient concentration of the subject compositions which produces 50% of its maximum response or effect with respect to such activity of γδ T cells.

As used herein, the term"EC100" with respect to regulating the activity of γδ T cells, refers to the efficient concentration of the subject compositions which produces its maximum response or effect with respect to such activity of γδ T cells.

Where hereinbefore and hereinafter numerical terms are used, they are meant to include the numbers representing the upper and lower limits. For example, "between 1 and 3" stands for a range "from and including 1 up to and including 3", and "in the range from 1 to 3" would stand for "from and including 1 up to and including 3". The same is true where instead of numbers (e.g. 3) words denoting numbers are used (e.g. "three").

Where "comprising" is used, this can preferably be replaced by "consisting essentially of", more preferably by "consisting of".

Where "about" is used in connection with a number, this preferably means the number +/-15%, more preferably the number plus 5%, most preferably the number itself without "about". For example, "about 100" would stand for "from and including 85 to and including 115". Where "about" is used in connection with numeric ranges, for example "about 1 to about 3", or "between about one and about three", preferably the definition of "about" given for a number in the last sentence is applied to each number defining the start and the end of a range separately. Preferably, where "about" is used in connection with any numerical values, the "about" can be deleted.

"Weekly" stands for "about once a week" (meaning that more than one treatment is made with an interval of about one week between treatments), the about here preferably meaning +/-1 day (that is, translating into "every 6 to 8 days"); most preferably, "weekly" stands for "once every 7 days".

"3-weekly" or "three-weekly" stands for "about once every three weeks" (meaning that more than one treatment is made with an interval of about three weeks between treatments), the about here preferably meaning +/-3 days (that is, translating into every 18 to 24 days); most preferably, "weekly" stands for "once every 21 days" (=every third week).

Whenever within this whole specification "treatment of a proliferative disease" or "treatment of a tumor", or "treatment of cancer" or the like is mentioned with reference to γδ T cell activator(s), there is meant:

a) a method of treatment (=for treating) of a proliferative disease, said method comprising the step of administering (for at least one treatment) a γδ T cell activator, (preferably in a pharmaceutically acceptable carrier material) to a warm-blooded animal, especially a human, in need of such treatment, in a dose that allows for the treatment of said disease (=a therapeutically effective amount), preferably in a dose (amount) as specified to be preferred hereinabove and hereinbelow;

b) the use of a γδ T cell activator for the treatment of a proliferative disease; or a γδ T cell activators, for use in said treatment (especially in a human);

c) the use of a γδ T cell activators, for the manufacture of a pharmaceutical preparation for the treatment of a proliferative disease; and/or

d) a pharmaceutical preparation comprising a dose of a γδ T cell activator that is appropriate for the treatment of a proliferative disease; or any combination of a), b), c) and d), in accordance with the subject matter allowable for patenting in a country where this application is filed;

e) a method of using a γδ T cell activator for the manufacture of a pharmaceutical preparation for the treatment of a proliferative disease, comprising admixing said γδ T cell activator with a pharmaceutically acceptable carrier. In cases where a tumor disease or a specific tumor (e.g. colon tumor, colon carcinoma or colon cancer; or prostate tumor, prostate carcinoma or prostate cancer) are mentioned instead of "proliferative disease", categories a) to e) are also encompassed, meaning that the respective tumor disease can be filled in under a) to e) above instead of "proliferative disease", in accordance with the patentable subject matter.

As further described herein, several γδ T cell activators have been found to regulate the activity of γδ T cells in vitro with at the nano- and pico-molar concentration level. Based on in vivo studies with first and second γδT activators, BrHPP and HDMAPP, it has now been found that these compounds are able to induce increases in biological activity as well as high rates of proliferation of γδ T cells in vivo in a primate. This enables a new strategy of stimulation of γδ T cells in vivo.

It has now also been found that a strategy of stimulation of γδ T cells can be effective in humans for the treatment of solid tumors, and furthermore also in solid tumors with metastases. A γδ T cell activating compound, BrHPP (also referred to as Phosphostim), was used in a clinical study to demonstrate that treatment of humans having metastatic renal carcinoma by introducing ex vivo activated γδ T cells could result in the effective treatment of solid tumors. Briefly, BrHPP was used in an adoptive autologous cellular immunotherapy process designed to produce large numbers of highly enriched Vγ9Vδ2 T cells from patient's cytapheresis. *Ex vivo* stimulation allows the generation of critical numbers of effector cells for therapeutic purposes. The BrHPP-stimulated γδ T cells were obtained by a 2-week manufacturing process. The initial cell preparation consists of PBMCs from blood from either fresh or frozen cytapheresis. The cells are expanded for two weeks in a closed system, with sequential addition of defined dosage IL-2 to the culture medium after a unique GMP-grade Phosphostim stimulation. The manufacturing process is much simpler than most current cellular therapy approaches using conventional CD8+ T cell lines or clones: there is no final separation or purification step nor use of feeder cells; the specific TCR-mediated signal provided by Phosphostim is sufficient to trigger the IL-2-dependent expansion of the Vγ9Vδ2 subset, which becomes dominant in the culture. Several doses of the γδ cellular product can be manufactured from one frozen cytapheresis. Typically, 100 million frozen PBMCs from cytapheresis yield 2 to 5 billions cells. The BrHPP-stimulated γδ T cells are currently being used in a Phase I clinical trial in metastatic Renal Cell Carcinoma (mRCC). The trial is currently on going at the second dose level of 4 billions cells after achieving correct tolerance of the first 1 billion cell dose. Preliminary results from the ongoing trial are promising, as the evaluable patients showed signs of anti-tumor activity in the form of stable disease.

In was also found that autologous γδ T cells could lyse, as a function of the effector to target cell ratio, tumors cells obtained from human patients having metastatic renal cell carcinoma, while not substantially causing lysis of normal (non-tumor) cells from the same patient. As described herein, BrHPP is a synthetic phosphoantigen that specifically expands Vγ9Vδ2 T cells from healthy donors' Peripheral Blood Mononuclear Cells (PBMC). These expanded Vγ9Vδ2 T cells lysed lymphoma targets and some established Renal Carcinoma Cell (RCC) lines.

In the present study, primary normal and tumor renal cells from RCC patients were established, and the effect of the BrHPP on PBMCs of these patients was investigated. This permitted assessment of the lytic potential of expanded Vγ9Vδ2 T cells against primary normal and tumor renal cells in an is autologous setting. This cytotoxic activity was compared with other autologous effectors cells, like LAK cells (Lymphokine-Activated Killer cells) for example.

The inventors performed a series of experiments suggesting that the compound BrHPP could also be used to treat solid tumors in vivo, and moreover metastatic tumors. In one set of experiments the inventors used a NOD/SCID mouse model of cancer to which human γδ T cells were introduced to demonstrate that a γδ T cell activator could prevent or arrest growth of metastatic renal cell tumor cells in culture.

The inventors further elucidated the pharmacokinetics of γδ T cells in primates in a series of experiments using several different γδ T cell activators. Provided are the findings that γδ T cells stimulated in a primate reach their peak numbers after between about 5 and 7 days following administration of the γδ T cell activator. Furthermore, results showed that γδ T cell activators can be administered repeatedly and re-stimulate γδ T cell activity in vivo. However, re-stimulation of γδ T cells is optimally not performed prior to peak expansion of γδ T, and moreover that such re-stimulation be performed once the γδ T cell population has returned to substantially the rate prior to the first stimulation. Furthermore, each administration of a compound to stimulate γδ T cells is optimally performed by a single shot, for example by infusion in the examples presented herein. In one example comparing two γδ T cell activators BrHPP and HDMAPP, dosages providing optimal γδ T cell biological activity and proliferation increases were determined. In determining the EC50 of respective compounds, the comparison revealed a correspondence of in vitro and in vivo EC50 dosages. Interestingly, assessment of the activity of the HDMAPP compound revealed that in comparison with its previously reported activity, low dose administration regimens for this compound may be used, as was confirmed in the EC50 in primates.

Thus, in a first aspect, the present invention relates to the treatment of a solid tumor, wherein a γδ T cell activator is administered to a warm-blooded animal, especially a human, preferably a human in need of such treatment, especially in a therapeutically effective amount.

A variety of cancers and other proliferative diseases including, but not limited to, the following can be treated using the methods and compositions of the invention:

- carcinoma, including that of the bladder, breast, colon, kidney, liver, lung, ovary, pancreas, stomach, cervix, thyroid and skin, including squamous cell carcinoma;
- tumors of mesenchymal origin, including fibrosarcoma and rhabdomyoscarcoma;
- other tumors, including melanoma, seminoma, teratocarcinoma, neuroblastoma and glioma;
- tumors of the central and peripheral nervous system, including astrocytoma, neuroblastoma, glioma, and schwannomas;
- tumors of mesenchymal origin, including fibrosarcoma, rhabdomyoscaroma, and osteosarcoma; and
- other tumors, including melanoma, xeroderma pigmentosum, keratoacanthoma, seminoma, thyroid follicular cancer and teratocarcinoma.

Where hereinbefore and subsequently a tumor, a tumor disease, a carcinoma or a cancer are mentioned, also metastasis in the original organ or tissue and/or in any other location are implied alternatively or in addition, whatever the location of the tumor and/or metastasis is.

In a preferred aspect, the γδ T cell activator may increase the biological activity of γδ T cells, preferably increasing the activation of γδ T cells, particularly increasing cytokine secretion from γδ T cells or increasing the cytolytic activity of γδ T cells, with or without also stimulating the expansion of γδ T cells. Thus in one aspect, the present invention relates to methods for the treatment of a disease, especially a proliferative disease, and more preferably a solid tumor, particularly a solid tumor having metastases, where a γδ T cell activator comprising a compound of the formula I, especially a γδ T cell activator according to formulas I to XVII, especially γδ T cell activator selected from the group consisting of BrHPP, CBrHPP, HDMAPP HDMAPP and epoxPP, is administered in an amount and under conditions sufficient to increase the activity γδ T cells in a subject, preferably in an amount and under conditions sufficient to increase cytokine secretion by γδ T cells and/or to increase the cytolytic activity of γδ T cells. In typical embodiments, a γδ T cell activator allows the cytokine secretion by γδ T cells to be increased at least 2, 3, 4, 10, 50, 100-fold, as determined in vitro.

Cytokine secretion and cytolytic activity can be assessed using any appropriate in vitro assay, or those provided in the examples herein. For example, cytokine secretion can be determined according to the methods described in Espinosa et al. (J. Biol. Chem., 2001, Vol. 276, Issue 21, 18337-18344), describing measurement of TNF-α release in a bioassay using TNF-α-sensitive cells. Briefly, $10^4$ γδT cells/well were incubated with stimulus plus 25 units ofIL2/well in 100 μl of culture medium during 24 h at 37 °C. Then, 50 μl of supernatant were added to 50 μl of WEHI cells plated at $3 \times 10^4$ cells/well in culture medium plus actinomycin D (2 μg/ml) and LiCl (40 mM) and incubated for 20 h at 37 °C. Viability of the TNF-α-sensitive cells and measured with a 3-(4,5-dimethylthiazol-2-yl)-2,5-diphenyltetrazoliumbromide assay. 50 μl of 3-(4,5-dimethylthiazol-2-yl)-2,5-diphenyltetrazolium bromide (Sigma; 2.5 mg/ml in phosphate-buffered saline) per well were added, and after 4 h of incubation at 37 °C, 50 μl of solubilization buffer (20% SDS, 66% dimethyl formamide, pH 4.7) were added, and absorbance (570 nm) was measured. Levels of TNF-α release were then calculated from a standard curve obtained using purified human rTNF-α (PeproTech, Inc., Rocky Hill, NJ). Interferon-γ released by activated T cells was measured by a sandwich enzyme-linked immunosorbent assay. $5 \times 10^4$ γδT cells/well were incubated with stimulus plus 25 units ofIL2/well in 100 μl of culture medium during 24 h at 37 °C. Then, 50 μl of supernatant were harvested for enzyme-linked immunosorbent assay using mouse monoclonal antibodies (BIOSOURCE, Camarillo, CA).

A preferred assay for cytolytic activity is a $^{51}Cr$ release assay. In exemplary assays, the cytolytic activity of γδ T cells is measured against autologous normal and tumor target cell lines, or control sensitive target cell lines such as Daudi and control resistant target cell line such as Raji in 4h $^{51}Cr$ release assay. In a specific example, target cells were used in amounts of $2x10^3$ cells/well and labeled with 100μCi $^{51}Cr$ for 60 minutes. Effector/Target ( E/T) ratio ranged from 30: 1 to 3.75: 1. Specific lysis (expressed as percentage) is calculated using the standard formula [(experimental-spontaneous release / total-spontaneous release) x100].

In another aspect, the present invention relates to methods for the treatment of a disease, especially a proliferative disease, and more preferably a solid tumor, particularly a solid tumor having metastases, where a γδ T cell activator, especially a γδ T cell activator according to formulas I to XVII, especially γδ T cell activator selected from the group consisting of BrHPP, CBrHPP, HDMAPP HDMAPP and epoxPP, is administered in an amount and under conditions sufficient to stimulate the expansion of the γδ T cell population in a subject, particularly to reach 30-90% of total circulating lymphocytes, typically 40-90%, more preferably from 50-90%. In typical embodiments, the invention allows the selective expansion of γδ T cells in a subject, to reach 60-90% of total circulating lymphocytes, preferably 70-90%, more preferably from 80-90%. Percentage of total circulating lymphocytes can be determined according to methods known in the art. A preferred method for determining the percentage of γδ T cells in total circulating lymphocytes is by flow cytometry, examples of appropriate protocols described in the examples herein.

In another embodiment, the present invention relates to methods for the treatment of a disease, especially a

proliferative disease, and more preferably a solid tumor, particularly a solid tumor having metastases, where a γδ T cell activator, especially a γδ T cell activator according to formulas I to XVII, especially γδ T cell activator selected from the group consisting of BrHPP, CBrHPP, HDMAPP HDMAPP and epoxPP, is administered in an amount and under conditions sufficient to stimulate the expansion of the γδ T cell population in a subject, particularly to increase by more than 2-fold the number of γδ T cells in a subject, typically at least 10-fold, more preferably at least 20-fold. In typical embodiments, the invention allows the selective expansion of γδ T cells in a subject, to increase by at least 2, 4, 10, 20, or 50-fold the number of γδ T cells in a subject, more preferably at least 100 or 200-fold. The number of γδ T cells in a subject is preferably assessed by obtaining a blood sample from a patient before and after administration of said γδ T cell activator and determining the difference in number of γδ T cells present in the sample. A preferred method for determining the number of γδ T cells by flow cytometry, examples of appropriate protocols described in the examples herein.

In another aspect, the present invention relates to methods for the treatment of a solid tumor, particularly a solid tumor having metastases, where a γδ T cell activator, especially a γδ T cell activator according to formulas I to XVII, especially γδ T cell activator selected from the group consisting of BrHPP, CBrHPP, HDMAPP HDMAPP and epoxPP, is administered in an amount and under conditions sufficient to stimulate the expansion of the γδ T cell population in a subject, particularly to reach a circulating γδ T cell count of at least 500 γδ T cells/mm3 in a subject, typically at least 1000 γδ T cells/mm3, more preferably at least 2000 γδ T cells/mm3. The circulating γδ T cell count in a subject is preferably assessed by obtaining a blood sample from a patient before and after administration of said γδ T cell activator and determining the number of γδ T cells in a given volume of sample. A preferred method for determining the number of γδ T cells by flow cytometry, examples of appropriate protocols described in the examples herein.

In a further aspect, the present invention relates to an in vivo regimen for the treatment a solid tumor having metastases, where a γδ T cell activator, especially a γδ T cell activator according to formulas I to XVII, especially γδ T cell activator selected from the group consisting of BrHPP, CBrHPP, HDMAPP HDMAPP and epoxPP, is administered to a warm-blooded animal, especially a human, in a dose that is higher (preferably at least 10%, 20%, 30% higher) than the single administration Efficient Concentration value giving half of the maximum effect (EC50) of γδ T cell biological activity or population expansion, or more preferably a dose that is at least 50%, or more preferably at least 60%, 75%, 85% or preferably between about 50% and 100% of the single administration Efficient Concentration value giving the maximum effect.

In preferred aspects, one or more (preferably at least two) further doses preferably each within the dose range mentioned herein are administered in one or preferably more than one further treatment cycle(s), especially with an interval between the treatment cycles of more than one week or preferably more than two weeks after the preceding treatment, more preferably after about two to about eight (8) weeks, most preferably about three (3) to about four (4) weeks after the preceding treatment, respectively. Generally, this treatment regimen where a dose is administered in two or more treatment cycles with periods of time between one to eight, preferably three to four weeks of time between administrations is preferred over more frequent treatments with lower doses, lower increases in γδ T cell biological activity or lower expansion of γδ T cell population.

Preferably, dosage (single administration) of a compound of formula I for treatment is between about 1 μg/kg and about 1.2 g/kg.

It will be appreciated that the above dosages related to a group of compounds, and that each particular compound may vary in optimal doses, as further described herein for exemplary compounds. Nevertheless, compounds are preferably administered in a dose sufficient to significantly increase the biological activity of γδ T cells or to significantly increase the γδ T cell population in a subject. Said dose is preferably administered to the human by intravenous (i.v.) administration during 2 to 180 min, preferably 2 to 120 min, more preferably during about 5 to about 60 min, or most preferably during about 30 min or during about 60 min.

In preferred exemplary compounds, a compound of formula II is administered in a dosage (single administration) between about 0.1 mg/kg and about 1.2 g/kg, preferably between about 10 mg/kg and about 1.2 g/kg, more preferably between about 5 mg/kg and about 100 mg/kg, even more preferably between about 5 μg/kg and 60 mg/kg. Most preferably, dosage (single administration) for three-weekly or four-weekly treatment (treatment every three weeks or every third week) is between about 0.1 mg/kg and about 1.2 g/kg, preferably between about 10 mg/kg and about 1.2 g/kg, more preferably between about 5 mg/kg and about 100 mg/kg, even more preferably between about 5 μg/kg and 60 mg/kg. This dose is preferably administered to the human by intravenous (i.v.) administration during 2 to 180 min, preferably 2 to 120 min, more preferably during about 5 to about 60 min, or most preferably during about 30 min or during about 60 min.

On other aspects, the dosage of a γδ T cell activator may be determined as a function of its maximal tolerated dose or highest tested dose in non-human animals. The present invention thus discloses an in vivo regimen for the treatment of a proliferative disease, especially a solid tumor and more particularly a solid tumor having metastases, where a γδ T cell activator, especially a γδ T cell activator according to formula I, especially γδ T cell activator selected from the group consisting of BrHPP, CBrHPP, HDMAPP HDMAPP and epoxPP, is administered in a dose that is between about 1 and about 100%, preferably between about 25 and 100%, of the (single administration) maximal tolerated dose

(MTD) to a warm-blooded animal, especially a human.

*Treatment cycles*

In further methods, the inventors have devised administration regimens providing improved regulation of γδ T cell activity based on the in vivo kinetics of γδ T cell regulating pyrophosphate compounds.

The invention provides a method of regulating the activity of γδ T cells in a mammalian subject, the method comprising administering to a subject in need thereof an effective amount of a γδ T cell activator according to a treatment cycle in which γδ T cell activity, preferably the γδ T cell rate (number of γδ T cells), is allowed to return to substantially basal rate prior to a second administration of the compound. As further described herein, in preferred embodiments, at least about one week, but more preferably at least about two weeks, are required for a patient's γδ T cell rate to return to substantially basal rate.

As further shown in the examples, the inventors have found that cycles shorter than about 7 days do not permit suitable stimulation of γδ T cell activity. The course of a preferred cycle is an at least 1-weekly cycle, but more preferably at least a 2-weekly cycle (at least about 14 days), or more preferably at least 3-weekly or 4-weekly, though cycles anywhere between 2-weekly and 4-weekly are preferred. Also effective and contemplated are cycles of up to 8-weekly, for example 5-weekly, 6-weekly, 7-weekly or 8-weekly.

In one preferred embodiment, administration of the γδ T cell activator occurs on the first day of a 2-weekly to 4-weekly cycle (that is, an about 14 to 28 day weeks repeating cycle). In a preferred embodiment, the γδ T cell activator is administered only the first day of the 2-weekly to 4-weekly, or preferably 3 weekly, cycle.

In preferred embodiments, administration of the γδ T cell activator occurs on the first day of a 1-weekly to 4-weekly cycle. In a preferred embodiment, the γδ T cell activator is administered only on the first day of the 1-weekly to 4-weekly cycle. In a preferred embodiment, the γδ T cell activator is administered only on the first day of the 1-weekly to 4-weekly cycle.

In particularly preferred embodiments, administration of the γδ T cell activator occurs on the first day of a 3-weekly to 4-weekly cycle. In a preferred embodiment, the γδ T cell activator is administered only on the first day of the 3-weekly to 4-weekly cycle. In a preferred embodiment, the γδ T cell activator is administered only on the first day of the 3-weekly to 4-weekly cycle.

As mentioned, a subject will preferably be treated for at least two cycles, or more preferably for at least three cycles. In other aspect, treatment may continue for a greater number of cycles, for example at least 4, 5, 6 or more cycles can be envisioned. At the end of each cycle, the cycle of dosing may be repeated for as long as clinically tolerated and the tumor is under control or until tumor regression. Tumor "control" is a well recognized clinical parameter, as defined above. In a preferred embodiment, the cycle of dosing is repeated for up to about eight cycles

*Co-treatment with cytokine*

In other embodiments, the methods of the invention comprises further administering a cytokine. While the compounds of the invention may be used with or without further administration, in a preferred aspect a cytokine can be administered, wherein said cytokine is capable of increasing the expansion of a γδ T cell population treated with a γδ T cell activator compound, preferably wherein the cytokine is capable of inducing an expansion of a γδ T cell population which is greater than the expansion resulting from administration of the γδ T cell activator compound in the absence of said cytokine. A preferred cytokine is an interleukin-2 polypeptide.

A cytokine having γδ T cell proliferation inducing activity, most preferably the interleukin-2 polypeptide, is administered at low doses, typically over a period of time comprised between 1 and 10 days. The γδ T cell activator is preferably administered in a single dose, and typically at the beginning of a cycle.

In preferred aspects, a cytokine, most preferably IL-2, is administered daily for up to about 10 days, preferably for a period of between about 3 and 10 days, or most preferably for about 7 days. Preferably, the administration of the cytokine begins on the same day (e.g. within 24 hours of) as administration of the γδ T cell activator. It will be appreciated that the cytokine can be administered in any suitable scheme within said regimen of between about 3 and 10 days. For example, in one aspect the cytokine is administered each day, while in other aspects the cytokine need not be administered on each day. When the cytokine is administered for about 7 to about 14 days, a 4-weekly treatment cycle is preferred. When the first component is administered for about 4 days, a 3-weekly day treatment cycle is preferred.

The present invention more specifically relates to the use of a γδ T cell activator according to claim 1 and an interleukin-2 polypeptide, for the manufacture of a pharmaceutical composition for treating a solid tumor in a subject, wherein said γδ T cell activator and interleukin-2 polypeptide are administered separately to the subject. More preferably, the interleukin-2 polypeptide is administered at low doses, typically over a period of time comprised between 1 and 10 days, and/or the γδ T cell activator is administered in a single dose at the beginning of the treatment.

The above methods and treatments may be used alone or in combination with other active agents or treatments.

For instance, for the treatment of tumors, the invention may be used in combination with other anti-tumor agents or treatments, such as chemotherapy, radiotherapy or gene therapy.

In preferred embodiments, the cytokine is administered during the first cycle of treatment with the γδT activator, and during each subsequent cycle of treatment with the γδT activator. However, it will be appreciated that the treatment regimen of the invention may be modified such that the cytokine is not administered in the first cycle of treatment. That is, the method may comprise:

(a) providing to a subject at least one first cycle of treatment, said first cycle comprising administering a synthetic γδT activator on the first day of a 1-weekly to 4-weekly cycle, or more preferably a 2-weekly to 4-weekly cycle, wherein no cytokine is administered during said first cycle;
(b) providing at least a second cycle of treatment, said second cycle comprising administering a synthetic γδT activator on the first day of a 1-weekly to 4-weekly cycle, or more preferably a 2-weekly to 4-weekly cycle, and administering a cytokine for a period of between 1 and about 10 days; and
(c) optionally repeating step (b) for any suitable number of further cycles.

In other aspects, it will be appreciated that the treatment regimen of the invention may be modified such that the cytokine is not administered in one or more of the subsequent cycles of treatment. That is, the method comprises:

(a) providing to a subject at least one first cycle of treatment, said first cycle comprising administering a synthetic γδT activator on the first day of a 1-weekly to 4-weekly cycle, or more preferably a 2-weekly to 4-weekly cycle, and administering a cytokine for a period of between 1 and about 10 days;
(b) providing at least a second cycle of treatment, said second cycle comprising administering a synthetic γδT activator on the first day of a 1-weekly to 4-weekly cycle, or more preferably a 2-weekly to 4-weekly cycle, wherein no cytokine is administered during said second cycle; and
(c) optionally repeating steps (a) or (b) for any suitable number of further cycles.

*Mode of use*

As disclosed herein, the γδ T cell activator is preferably administered as a single shot. When used in a treatment comprising more than one administration, the γδ T cell activator is preferably administered as a single shot at the beginning of a treatment cycle. As shown in the experimental section, such administration schedule provides a remarkable increase in the activity of γδ T cells in a subject. The active ingredients may be administered through different routes, typically by injection or oral administration. Injection may be carried out into various tissues, such as by intravenous, intra-peritoneal, intra-arterial, intra-muscular, intra-dermic, subcutaneous, etc.

Most preferably, the γδ T cell activator is administered by intravenous (i.v.) administration. Preferably said infusion is during 2 to 180 min, preferably 2 to 120 min, more preferably during about 5 to about 30 min, most preferably during about 10 to about 30 min, e.g. during about 30 min. As further described herein, the invention discloses that a brief stimulation of γδ T cell activity is sufficient to achieve the γδ T cell regulating effect. Thus, preferably where a γδ T cell activating compound has a short serum half-life, for example having a serum half-life of less than about 48 hours, less than about 24 hours, or less than about 12 hours, a rapid infusion is used. Said rapid infusion is preferably between about 10 minutes and 60 minutes, or more preferably about 30 minutes.

When an administration regimen comprises both a γδ T cell activator and an interleukin-2 polypeptide, said compounds are preferably separately administered. Within the context of the present invention, the term "separately administered" indicates that the active ingredients are administered at a different site or through a different route or through a different schedule to the subject. Accordingly, the ingredients are generally not mixed together prior to administration, although they may be combined in a unique package in suitable separated containers.

In a preferred embodiment, the active ingredients are administered through different schedules: the synthetic γδ T cell activator is administered as a single shot, at the beginning of the treatment, and the interleukin-2 polypeptide is administered over a prolonged period of time, typically between 1 and 10 days. As shown in the experimental section, such administration schedule provides a remarkable increase in the activity of γδ T cells in a subject.

The active ingredients may be administered through different routes, typically by injection or oral administration. Injection may be carried out into various tissues, such as by intravenous, intra-peritoneal, intra-arterial, intra-muscular, intra-dermic, subcutaneous, etc. Preferred administration routes for the synthetic activators are intravenous and intra-muscular. Preferred administration routes for the cytokine are subcutaneous, intravenous and intra-muscular.

A specific embodiment of the present invention relates to the use of (i) a synthetic γδT activator selected from a PED or a PHD compound and (ii) an interleukin-2 polypeptide, for the manufacture of a pharmaceutical composition for treating cancer, an infectious disease or an auto-immune disease in a subject, wherein said synthetic γδT activator and interleukin-2 polypeptide are administered separately to the subject. More preferably, the interleukin-2 polypeptide

is administered at low doses, typically over a period of time comprised between 1 and 10 days, and/or the synthetic γδT activator is administered in a single dose at the beginning of the treatment, preferably at a dose comprised between 0.5 and 80 mg/kg, more preferably between 1 and 60, even more preferably between 2 and 60.

*Dosage of γδ T cell activators*

As discussed, specific dosage ranges suitable for the administration of γδ T cell activators to increase the activity of γδ T cells are disclosed herein. Nevertheless, it will be appreciated that the dose of activator may be adapted by the skilled artisan, depending on the nature of the activator, its specific-activity, EC50, stability and/or pharmacokinetics, as well as on the type of subject, etc. Several methods for doing so with respect to γδ T cell activators are also provided herein. Preferably, the γδ T cell activator is administered in a human subject at a dose comprised between 0.01 and 200 mg/kg, preferably between 0.05 and 80 mg/kg, more preferably between 0.1 and 60, even more preferably between 1 and 60 mg/kg. The activator may be administered as a single dose, at the beginning of the treatment, or distributed over several days. Unexpectedly, however, the invention shows that a significantly higher effect is obtained when the activator is administered in a single dose at the beginning of the treatment. Typical dosages are comprised between 1 and 60 mg/kg, more preferably between 1 and 50 mg/kg. Appropriate dosages may be deduced from experiments conducted in animals, by normalizing with respect to the mean body weight and mean body surface. For instance, it can be calculated that efficient doses of between 2 and 300 mg/kg in a cynomolgus monkey (having a mean body weight of about 3kg and mean body surface of about 0.3 $m^2$) correspond to an efficient dosage of between 0.5 and 80 mg/kg in a human subject (having a mean body weight of about 65kg and mean body surface of about 1.8 $m^2$). It should be understood that different dosages may be used, including higher dosages, considering the low toxicity observed in vivo with the synthetic activators.

The invention further provides method which may be used to determine the appropriate dosage ranges for further γδ T cell activators based on their in vitro activities. In one aspect, the invention provides in vivo and in vitro dose-effect curves for two γδ T cell activators showing correspondence in in vitro and in vivo γδ T cell stimulating activities, particularly correspondence of EC50 values. The invention thus also provides methods of determining the dosage of a γδ T cell activator, as well as dosages for administration to human subjects for said test compounds based on the examples provided herein. An appropriate dosage range for a compound can be determined by (a) determining the in vitro γδT activating potency of a compound, preferably using an assay method as described in the examples herein, and (b) comparing said activity of the test compound to the in vitro activity obtained using a compound for which the in vivo activity is known, preferably a BrHPP or HDMAPP compound, and computing an in vivo dosage or dosage range proportional to that obtained with the compound for which the in vivo activity is known. Preferably determining the in vitro activity comprises determining the EC50. In this way, a dosage range can be determined, for example useful for selecting a starting dose for studies in non-human mammalian subjects, preferably cynomolgus monkeys. Further precision can be obtained by administering different doses within the range to subjects and assessing γδ T cell activity, for examples using the assays described herein.

The present invention also provides that a brief stimulation of γδ T cell activity is sufficient to stimulate an increase in γδ T cell activity. Thus, suitable γδ T cell activating compound include compounds having short as well as longer half-lives. In one aspect, a γδ T cell activator having a short serum half-life, for example having a serum half-life of less than about 48 hours, less than about 24 hours, or less than about 12 hours, is administered in a dose that is higher (preferably at least 110%, 120%, 130%, or 150% of the EC50) than the single administration Efficient Concentration value giving half of the maximum effect (EC50) of γδ T cell biological activity or population expansion, or more preferably a dose that is at least 50%, or more preferably at least 60%, 75%, 85% or preferably between about 50% and 100% of the single administration Efficient Concentration value giving the maximum effect (EC100). Preferably said administration is by intravenous infusion, during between about 10 minutes and about 60 minutes.

In other aspect, appropriate dosage may be determined as a function of the Maximal Tolerated Dose (MTD). The MTD is determined according to standard procedures; preferably, in warm-blooded animals the MTD in case of oral or intravenous administration is determined as the dose of a single administration where no death occurs and a loss of body weight of less than 40, preferably less than 25, percent (%) is found in the treated warm-blooded animal individual (this term here mainly referring to an animal; for humans see below). In other aspects, where dose range studies in animal have not demonstrated an MTD, the highest tested dose may be considered in place of the MTD.

The MTD may vary depending on the population of the patients which may be defined by tumor type, age range, gender, tumor stage and the like. While in animals, the most preferable way of determining the MTD can be analogous to that shown in the Examples presented below, in humans the MTD may generally be determined by starting with one single administration of a very low dose, e.g. 1/10th of the LD10 (i.e., the dose that is lethal to 10% of animals) in the most sensitive animal species in which toxicology studies have been performed. Dose escalation for the next dose level is 100%, unless grade 2 toxicity is seen according to the US National Cancer Institute Revised Common Toxicity Criteria, in which case dose escalation will be 67%. Dose escalation for subsequent dose levels is in the range of 25%

to 67%. For example, three patients are usually treated at one dose level and observed for acute toxicity for one course of treatment before any more patients are entered. If none of the three patients experience DLT (dose-limiting toxicity), then the next cohort of three patients is treated with the next higher dose. If two or more of the three patients experience DLT, then three more patients are treated at the next lower dose unless six patients have already been treated at that dose. If one of three patients treated at a dose experiences DLT, then three more patients are treated at the same level. If the incidence of DLT among those patients is one in six, then the next cohort is treated at the next higher dose. In general, if two or more of the six patients treated at a dose level experience DLT, then the MTD is considered to have been exceeded, and three more patients are treated at the next lower dose as described above. The MTD is defined as the highest dose studied for which the incidence of DLT was less than 33%. Usually dose escalation for subsequent courses in the same patient--i.e. intrapatient dose escalation--is not permitted. Alternatively, dose steps may be defined by a modified Fibonacci series in which the increments of dose for succeeding levels beyond the starting dose are 100%, 67%, 50% and 40%, followed by 33% for all subsequent levels. Finally, the MTD may be found by methods described in Simon, R., et al., J. Nat. Cancer Inst. 89(15), 1997, p. 1138-1147.

The DLT generally includes (but is not limited to) any drug-related death and most drug-related grade 3 and 4 toxicities, including febrile neutropenia (see also US National Cancer Institute Revised Common Toxicity Criteria). See especially the examples.

In the above methods and uses, the subject is preferably a human subject, such as a subject having a cancer, an infectious disease, an autoimmune disease or an allergic disease. The invention is indeed suitable to treat all conditions caused by or associated with the presence of pathological cells which are sensitive to γδ T cell lysis.

The invention is particularly suited to stimulate the anti-tumor immunity of a subject having a solid or hematopoietic tumor, such as a lymphoma, bladder cancer, multiple myeloma, renal cell carcinoma, etc.

Nevertheless, the invention is also suitable to stimulate an anti-viral immune response in a subject having an infection by a virus selected from HIV, CMV, EBV, Influenza virus, HCV, HBV, etc.

The invention is also suitable to stimulate an immune response in a subject having an infection by a pathogen causing tuberculosis, malaria, tularemia, colibacillosis, etc.

The invention is also suitable to treat (e.g., to stimulate an immune response in) a subject having an autoimmune disease, such as diabetes, multiple sclerosis, rheumatoid arthritis, etc. or a subject having an allergic disease, including asthma, airway hyper-responsiveness, etc.

Unless otherwise indicated, the dosages for administration to a warm blooded animal, particularly humans provided herein are indicated in pure form (anionic form) of the respective compound. Purity level for the active ingredient depending on the synthesis batch can be used to adjust the dosage from actual to anionic form and vice-versa.

*Synthetic γδ T lymphocyte activators*

An advantageous aspect of this invention resides in the use of a synthetic γδT lymphocytes activating compound. Indeed, the invention shows that a potent and targeted expansion and activation of γδT cells can be obtained in vivo by trigerring one single metabolic pathway, using defined activating compounds following a particular administration schedule.

The term "synthetic γδT lymphocyte activating compound", and the term "synthetic γδT cell activating compound" used interchangeable, designates a molecule artificially produced, which can activate γδT lymphocytes. More particularly, the term synthetic γδT lymphocyte activating compound designates a molecule produced ex vivo or in vitro. It is more preferably a ligand of the T receptor of γδT lymphocytes. The activator may by of various nature, such as a peptide, lipid, small molecule, etc. It may be a purified or otherwise artificially produced (e.g., by chemical synthesis, or by microbiological process) endogenous ligand, or a fragment or derivative thereof, or an antibody having substantially the same antigenic specificity. The activator is most preferably a synthetic chemical compound capable of selectively activating Vγ9Vδ2 T lymphocytes. Selective activation of Vγ9Vδ2 T lymphocytes indicates that the compound has a selective action towards specific cell populations, and essentially does not activate other T cell sub-types, such as Vδ1 T cells. Such selectivity, as disclosed in the present application, suggests that preferred compounds can cause a selective or targeted activation of the proliferation or biological activity of Vγ9Vδ2 T lymphocytes.

Preferably a synthetic γδ T lymphocyte activator is a compound capable of regulating the activity of a γδ T cell in a population of γδ T cell clones in culture. The synthetic γδ T lymphocyte is capable of regulating the activity of a γδ T cell population of γδ T cell clones in a at millimolar concentration, preferably when the γδ T cell activator is present in culture at a concentration of less than 100 mM. Optionally a synthetic γδ T lymphocyte is capable of regulating the activity of a γδ T cell in a population of γδ T cell clones at millimolar concentration, preferably when the γδ T cell activator is present in culture at a concentration of less than 10 mM, or more preferably less than 1 mM. Regulating the activity of a γδ T cell can be assessed by any suitable means, preferably by assessing cytokine secretion, most preferably TNF-α secretion as described herein. Methods for obtaining a population of pure γδ T cell clones is described in Davodeau et al, (1993) and Moreau et al, (1986), the disclosures of which are incorporated herein by reference. Preferably the

compound is capable of causing at least a 20%, 50% or greater increase in the number of γδ T cells in culture, or more preferably at least a 2-fold increase in the number of γδ T cells in culture.

In the preferred embodiment, R is selected from the group consisting of :

1)

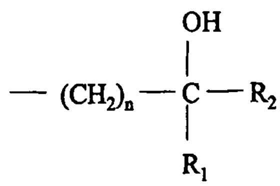

wherein n is an integer from 2 to 20, $R_1$ is a $(C_1-C_3)$alkyl group, and $R_2$ is an halogenated $(C_1-C_3)$alkyl, a $(C_1-C_3)$alkoxy-$(C_1-C_3)$alkyl, an halogenated $(C_2-C_3)$acyl or a $(C_1-C_3)$alkoxy-$(C_2-C_3)$acyl. Preferably, $R_1$ is a methyl or ethyl group, and $R_2$ is an halogenated methyl ($-CH_2-X$, X being an halogen), an halogenated $(C_2-C_3)$acetyl, or $(C_1-C_3)$alkoxy- acetyl. The halogenated methyl or acetyl can be mono-, di-, or tri-halogenated. Preferably, n is an integer from 2 to 10, or from 2 to 5. In a more preferred embodiment, n is 2. In a most preferred embodiment, n is 2, $R_1$ is a methyl and $R_2$ is an halogenated methyl, more preferably a monohalogenated methyl, still more preferably a bromide methyl. In a particularly preferred embodiment, n is 2, $R_1$ is a methyl, R2 is a methyl bromide. In a most preferred embodiment, R is 3-(bromomethyl)-3-butanol-1-yl.

2)

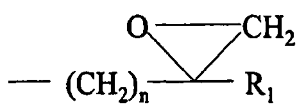

wherein n is an integer from 2 to 20, and $R_1$ is a methyl or ethyl group. Preferably, n is an integer from 2 to 10, or from 2 to 5. In a more preferred embodiment, n is 2 and R1 is a methyl.

3)

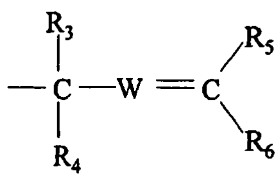

wherein $R_3$, $R_4$, and $R_5$ , identical or different, are a hydrogen or $(C_1-C_3)$alkyl group, W is -CH- or -N-, and $R_6$ is an $(C_2-C_3)$acyl, an aldehyde, an $(C_1-C_3)$alcohol, or an $(C_2-C_3)$ester. More preferably, $R_3$ and $R_5$ are a methyl and $R_4$ is a hydrogen. More preferably, $R_6$ is $-CH_2-OH$, -CHO, $-CO-CH_3$ or- $CO-OCH_3$. Optionally, the double-bond between W and C is in conformation trans (E) or cis (Z). More preferably, the double-bond between W and C is in conformation trans (E).

The group Y can allow to design a prodrug. Therefore, Y is enzymolabile group which can be cleaved in particular regions of the subject. The group Y can also be targeting group. In a preferred embodiment, Y is $O^-Cat+$, a group -A-R, or a radical selected from the group consisting of a nucleoside, a monosaccharide, an epoxyde and a halohydrin. Preferably, Y is an enzymolabile group. Preferably, Y is $O^-Cat+$, a group -A-R, or a nucleoside. In a first preferred embodiment, Y is $O^-Cat+$. In a second preferred embodiment, Y is a nucleoside.

In a preferred embodiment, $Cat^+$ is $H^+$, $Na^+$, $NH_4^+$, $K^+$, $Li^+$, $(CH_3CH_2)_3NH^+$.

In a preferred embodiment, A is O, CHF, $CF_2$ or $CH_2$. More preferably, A is O or $CH_2$.

In a preferred embodiment, B is O or NH. More preferably, B is O.

In a preferred embodiment, m is 1 or 2. More preferably, m is 1.

The synthetic γδT lymphocyte activators comprise the compounds of formula (II):

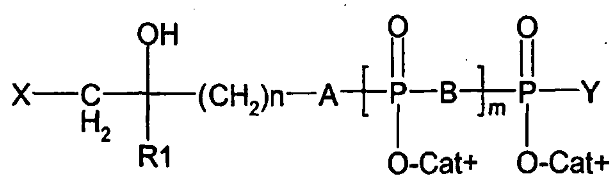
(II)

in which X is an halogen (preferably selected from I, Br and Cl), B is O or NH, m is an integer from 1 to 3, R1 is a methyl or ethyl group, Cat+ represents one (or several, identical or different) organic or mineral cation(s) (including the proton), and n is an integer from 2 to 20, A is O, NH, CHF, $CF_2$ or $CH_2$, and Y is $O^-Cat+$, a nucleoside, or a radical -A-R, wherein R is selected from the group of 1), 2) or 3). Preferably, Y is $O^-Cat+$, or a nucleoside. More preferably, Y is $O^-Cat+$, Preferably, R1 is a methyl. Preferably, A is O or $CH_2$. More preferably, A is O. Preferably, n is 2. Preferably, X is a bromide. Preferably, B is O. Preferably, m is 1 or 2. More preferably, m is 1.

For example, synthetic γδT lymphocyte activators comprise the compounds of formula (III) or (IV):

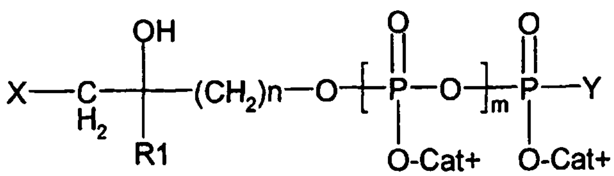
(III)

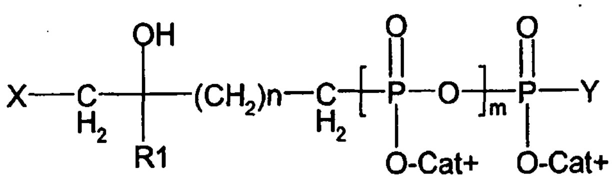
(IV)

wherein X, R1, n, m and Y have the aforementioned meaning.

In one preferred embodiment, synthetic γδT lymphocyte activators comprise the compounds of formula (V):

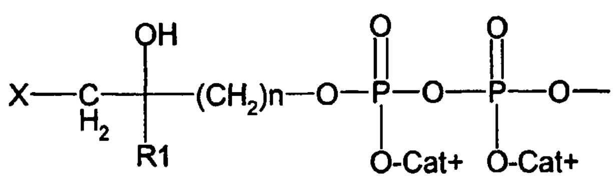
(V)

in which X is an halogen (preferably selected from I, Br and Cl), R1 is a methyl or ethyl group, Cat+ represents one (or several, identical or different) organic or mineral cation(s) (including the proton), and n is an integer from 2 to 20. Preferably, R1 is a methyl. Preferably, n is 2. Preferably, X is a bromide.

In a most preferred embodiment, synthetic γδT lymphocyte activators comprise the compound of formula (VI):

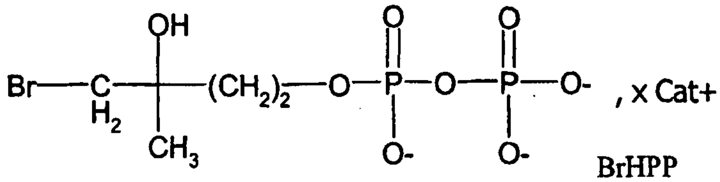

(VI)

BrHPP

Preferably x Cat+ is 1 or 2 $Na^+$.

In an other most preferred embodiment, synthetic γδT lymphocyte activators comprise the compound of formula (VII):

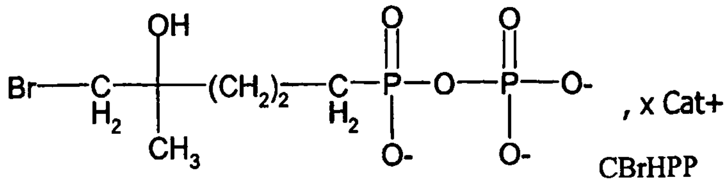

(VII)

CBrHPP

Preferably × Cat+ is 1 or 2 $Na^+$.

In one particular embodiment, synthetic γδT lymphocyte activators comprise the compounds of formula (VIII):

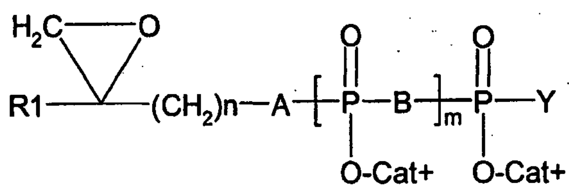

(VIII)

in which R1 is a methyl or ethyl group, Cat+ represents one (or several, identical or different) organic or mineral cation (s) (including the proton), B is O or NH, m is an integer from 1 to 3, and n is an integer from 2 to 20, A is O, NH, CHF, $CF_2$ or $CH_2$, and Y is $O^-$Cat+, a nucleoside, or a radical -A-R, wherein R is selected from the group of 1), 2) or 3). Preferably, Y is $O^-$Cat+, or a nucleoside. More preferably, Y is $O^-$Cat+. Preferably, R1 is a methyl. Preferably, A is O or $CH_2$. More preferably, A is O. Preferably, n is 2. Preferably, B is O. Preferably, m is 1 or 2. More preferably, m is 1.

For example, synthetic γδT lymphocyte activators comprise the compounds of formula (IX) or (X):

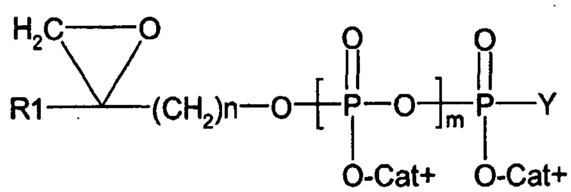

(IX)

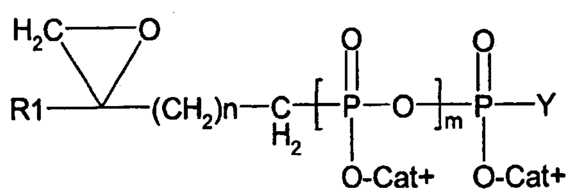

(X)

wherein R1, n, m and Y have the above mentioned meaning.

In one preferred embodiment, synthetic γδT lymphocyte activators comprise the compounds of formula (XI):

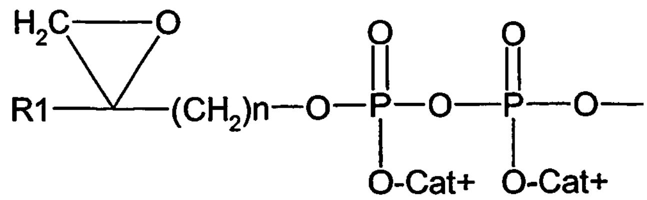

(XI)

in which R1 is a methyl or ethyl group, Cat+ represents one (or several, identical or different) organic or mineral cation(s) (including the proton), and n is an integer from 2 to 20. Preferably, R1 is a methyl. Preferably, n is 2.

In a most preferred embodiment, synthetic γδT lymphocyte activators comprise the compound of formula (XI):

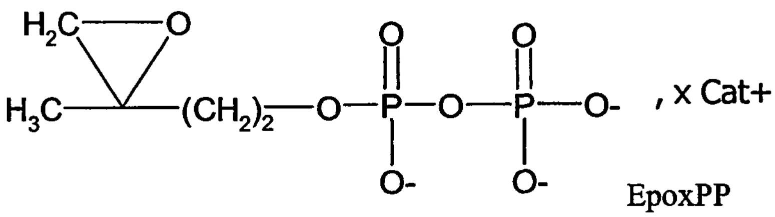

(XI)

EpoxPP

Preferably × Cat+ is 1 or 2 $Na^+$.

In one particular embodiment, synthetic γδT lymphocyte activators comprise the compounds of formula (XII):

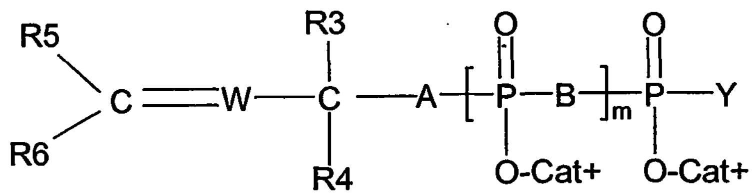

(XII)

in which $R_3$, $R_4$, and $R_5$ , identical or different, are a hydrogen or $(C_1$-$C_3)$alkyl group, W is -CH- or -N-, $R_6$ is an $(C_2$-$C_3)$ acyl, an aldehyde, an $(C_1$-$C_3)$alcohol, or an $(C_2$-$C_3)$ester, Cat+ represents one (or several, identical or different) organic or mineral cation(s) (including the proton), B is O or NH, m is an integer from 1 to 3, A is O, NH, CHF, $CF_2$ or $CH_2$, and Y is $O^-$Cat+, a nucleoside, or a radical -A-R, wherein R is selected from the group of 1), 2) or 3). Preferably, Y is $O^-$Cat+, or a nucleoside. More preferably, Y is $O^-$Cat+. Preferably, A is O or $CH_2$. More preferably, A is O. More preferably, $R_3$ and $R_5$ are a methyl and $R_4$ is a hydrogen. More preferably, $R_6$ is $-CH_2$-OH, - CHO, $-CO-CH_3$ or $-CO-OCH_3$. Preferably, B is O. Preferably, m is 1 or 2. More preferably, m is 1. Optionally, the double-bond between W and C is in conformation trans (E) or cis (Z). More preferably, the double-bond between W and C is in conformation trans (E).

For example, synthetic γδT lymphocyte activators comprise the compounds of formula (XIII) or (XIV):

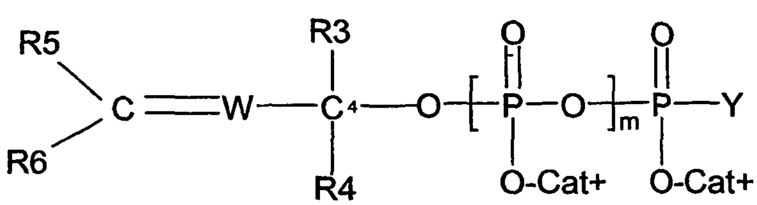

(XIII)

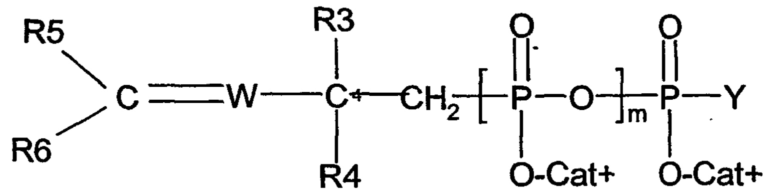

(XIV)

wherein R3, R4, R5, R6, W, m, and Y have the above mentionned meaning. Preferably, W is -CH-. Preferably, R3 and R4 are hydrogen. Preferably, R5 is a methyl. Preferably, R6 is $-CH_2-OH$.

In a most preferred embodiment, synthetic γδT lymphocyte activators comprise the compound of formula (XV):

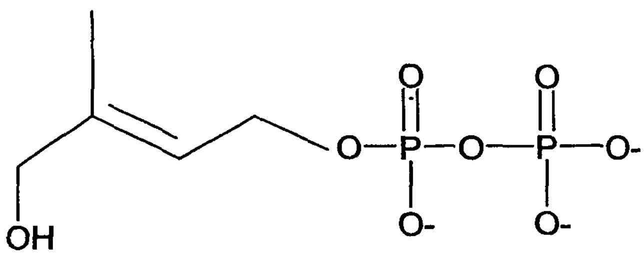

(XV) HDMAPP

In an other most preferred embodiment, synthetic γδT lymphocyte activators comprise the compound of formula (XVI):

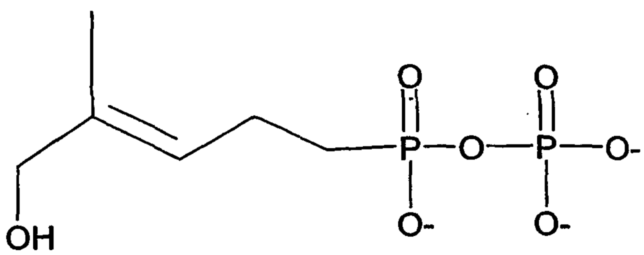

(XVI) CHDMAPP

Specific examples of compounds include :

(E)1-pyrophosphonobuta-1,3-diene
(E)1-pyrophosphonopenta-1,3-diene
(E)1-pyrophosphono-4-methylpenta-1,3-diene
(E,E)1-pyrophosphono-4,8-dimethylnona-1,3,7-triene
(E,E,E)1-pyrophosphono-4,8,12-trimethyltrideca-1,3,7,11-tetraene
(E,E)1-triphosphono-4,8-dimethylnona-1,3,7-triene
4- triphosphono-2-methylbutene
α,β-di-[3-methylpent-3-enyl]-pyrophosphonate
1-pyrophosphono-3-methylbut-2-ene
α,γ-di-[3-methylbut-2-enyl]-triphosphonate
α,β-di-[3-methylbut-2-enyl]-pyrophosphonate
allyl-pyrophosphonate
allyl-triphosphonate
α,γ-di-allyl-pyrophosphonate
α,β-di-allyl-triphosphonate
(E,E)4-[(5'-pyrophosphono-6'-methyl-penta-2',4'-dienyloxymethyl)-phenyl]-phenyl-methanone
(E,E)4-[(5'-triphosphono-6'-methyl-penta-2',4'-dienyloxymethyl)-phenyl]-phenyl-methanone
(E,E,E)[4-(9'-pyrophosphono-2',6'-dimethyl-nona-2',6',8'-trienyloxymethyl)-phenyl]-phenyl-methanone

(E,E,E)[4-(9'-pyrophosphono-2',6',8'-trimethyl-nona-2',6',8'-trienyloxymethyl)-phenyl]-phenyl-methanone
5-pyrophosphono-2-methypentene
5-triphosphono-2-methypentene
α,γ-di-[4-methylpent-4-enyl]-triphosphonate
5-pyrophosphono-2-methypent-2-ene
5-triphosphono-2-methypent-2-ene
9-pyrophosphono-2,6-dimethynona-2,6-diene
9-triphosphono-2,6-dimethynona-2,6-diene
α,γ-di-[4,8-dimethylnona-2,6-dienyl]-triphosphonate
4-pyrophosphono-2-methybutene
4-methyl-2-oxa-pent-4-enyloxymethylpyrophosphate
4-methyl-2-oxa-pent-4-enyloxymethyltriphosphate
α,β-di-[4-methyl-2-oxa-pent-4-enyloxymethyl]-pyrophosphate
a,y-di-[4-methyl-2-oxa-pent-4-enyloxymethyl]-triphosphate

Phosphohalohydrins (R=1$^{st}$))

3-(halomethyl)-3-butanol-1-yl-diphosphate
3-(halomethyl)-3-pentanol-1-yl-diphsophate
4-(halomethyl)-4-pentanol-1-yl-diphosphate
4-(halomethyl)-4-hexanol-1-yl-diphosphate
5-(halomethyl)-5-hexanol-1-yl-diphosphate
5-(halomethyl)-5-heptanol-1-yl-diphosphate
6-(halomethyl)-6-heptanol-1-yl-diphosphate
6-(halomethyl)-6-octanol-1-yl-diphosphate
7-(halomethyl)-7-octanol-1-yl-diphosphate
7-(halomethyl)-7-nonanol-1-yl-diphosphate
8-(halomethyl)-8-nonanol-1-yl-diphosphate
8-(halomethyl)-8-decanol-1-yl-diphosphate
9-(halomethyl)-9-decanol-1-yl-diphosphate
9-(halomethyl)-9-undecanol-1-yl-diphosphate
10-(halomethyl)-10-undecanol-1-yl-diphosphate
10-(halomethyl)-10-dodecanol-1-yl-diphosphate
11-(halomethyl)-11-dodecanol-1-yl-diphosphate
11-(halomethyl)-11-tridecanol-1-yl-diphosphate
12-(halomethyl)-12-tridecanol-1-yl-diphosphate
12-(halomethyl)-12-tetradecanol-1-yl-diphosphate
13-(halomethyl)-13-tetradecanol-1-yl-diphosphate
13-(halomethyl)-13-pentadecanol-1-yl-diphosphate
14-(halomethyl)-14-pentadecanol-1-yl-diphosphate
14-(halomethyl)-14-hexadecanol-1-yl-diphosphate
15-(halomethyl)-15-hexadecanol-1-yl-diphosphate
15-(halomethyl)-15-heptadecanol-1-yl-diphosphate
16-(halomethyl)-16-heptadecanol-1-yl-diphosphate
16-(halomethyl)-16-octadecanol-1-yl-diphosphate
17-(halomethyl)-17-octadecanol-1-yl-diphosphate
17-(halomethyl)-17-nonadecanol-1-yl-diphosphate
18-(halomethyl)-18-nonadecanol-1-yl-diphosphate
18-(halomethyl)-18-eicosanol-1-yl-diphosphate
19-(halomethyl)-19-eicosanol-1-yl-diphosphate
19-(halomethyl)-19-heneicosanol-1-yl-diphosphate
20-(halomethyl)-20-heneicosanol-1-yl-diphosphate
20-(halomethyl)-20-docosanol-1-yl-diphosphate
21-(halomethyl)-21-docosanol-1-yl-diphosphate
21-(halomethyl)-21-tricosanol-1-yl-diphosphate

More particularly,

3-(bromomethyl)-3-butanol-1-yl-diphosphate (BrHPP)
5-bromo-4-hydroxy-4-methylpentyl pyrophosphonate (CBrHPP)
3-(iodomethyl)-3-butanol-1-yl-diphosphate (IHPP)
3-(chloromethyl)-3-butanol-1-yl-diphosphate (ClHPP)
3-(bromomethyl)-3-butanol-1-yl-triphosphate (BrHPPP)
3-(iodomethyl)-3-butanol-1-yl-triphosphate (IHPPP)
α,γ-di-[3-(bromomethyl)-3-butanol-1-yl]-triphosphate (diBrHTP)
α,γ-di-[3-(iodomethyl)-3-butanol-1-yl]-triphosphate (diIHTP)

Phosphoepoxydes (R=2$^{nd}$))

3,4-epoxy-3-methyl-1-butyl-diphosphate (Epox-PP)
3,4,-epoxy-3-methyl-1-butyl-triphosphate (Epox-PPP)
α,γ-di-3,4,-epoxy-3-methyl-1-butyl-triphosphate (di-Epox-TP)
3,4-epoxy-3-ethyl-1-butyl-diphosphate
4,5-epoxy-4-methyl-1-pentyl-diphosphate
4,5-epoxy-4-ethyl-1-pentyl-diphosphate
5,6-epoxy-5-methyl-1-hexyl-diphosphate
5,6-epoxy-5-ethyl-1-hexyl-diphosphate
6,7-epoxy-6-methyl-1-heptyl-diphosphate
6,7-epoxy-6-ethyl-1-heptyl-diphosphate
7,8-epoxy-7-methyl-1-octyl-diphosphate
7,8-epoxy-7-ethyl-1-octyl-diphosphate
8,9-epoxy-8-methyl-1-nonyl-diphosphate
8,9-epoxy-8-ethyl-1-nonyl-diphosphate
9,10-epoxy-9_methyl-1-decyl-diphosphate
9,10-epoxy-9-ethyl-1-decyl-diphosphate
10,11-epoxy-10-methyl-1-undecyl-diphosphate
10,11-epoxy-10-ethyl-1-undecyl-diphosphate
11,12-epoxy-11-methyl-1-dodecyl-diphosphate
11,12-epoxy-11-ethyl-1-dodecyl-diphosphate
12,13-epoxy-12-methyl-1-tridecyl-diphosphate
12,13-epoxy-12-ethyl-1-tridecyl-diphosphate
13,14-epoxy-13-methyl-1-tetradecyl-diphosphate
13,14-epoxy-13-ethyl-1-tetradecyl-diphosphate
14,15-epoxy-14-methyl-1-pentadecyl-diphosphate
14,15-epoxy-14-ethyl-1-pentadecyl-diphosphate
15,16-epoxy-15-methyl-1-hexadecyl-diphosphate
15,16-epoxy-15-ethyl-1-hexadecyl-diphosphate
16,17-epoxy-16-methyl-1-heptadecyl-diphosphate
16,17-epoxy-16-ethyl-1-heptadecyl-diphosphate
17,18-epoxy-17-methyl-1-octadecyl-diphosphate
17,18-epoxy-17-ethyl-1-octadecyl-diphosphate
18,19-epoxy-18-methyl-1-nonadecyl-diphosphate
18,19-epoxy-18-ethyl-1-nonadecyl-diphosphate
19,20-epoxy-19-methyl-1-eicosyl-diphosphate
19,20-epoxy-19-ethyl-1-eicosyl-diphosphate
20,21-epoxy-20-methyl-1-heneicosyl-diphosphate
20,21-epoxy-20-ethyl-1-heneicosyl-diphosphate
21,22-epoxy-21-methyl-1-docosyl-diphosphate
21,22-epoxy-21-ethyl-1-docosyl-diphosphate

More particularly,

3,4-epoxy-3-methyl-1-butyl-diphosphate (Epox-PP)
3,4,-epoxy-3-methyl-1-butyl-triphosphate (Epox-PPP)
α,γ-di-3,4,-epoxy-3-methyl-1-butyl-triphosphate (di-Epox-TP)
uridine 5'-triphosphate -(3,4-époxy methyl butyl) (Epox-UTP)

Phosphoepoxydes (R=3rd))

(E)-4-hydroxy-3-methyl-2-butenyl pyrophosphate (HDMAPP)
(E)-5-hydroxy-4-methylpent-3-enyl pyrophosphonate (CHDMAPP)

These compounds may be produced according to various techniques known per se in the art, some of which being disclosed in PCT Publications nos. WO 00/12516, WO 00/12519, WO 03/050128, and WO 03/009855.

In a most preferred embodiment, the synthetic γδT lymphocyte activating compound is selected from the group consisting of HDMAPP, CHDMAPP, Epox-PP, BrHPP and CBrHPP, more preferably HDMAPP, CHDMAPP, BrHPP and CBrHPP, still more preferably HDMAPP.

Alternatively, although potentially less efficient, other activators for use in the present invention are phosphoantigens disclosed in WO 95/20673, isopentenyl pyrophosphate (IPP) (US 5,639,653) and 3-methylbut-3-enyl pyrophosphonate (C-IPP).

Compounds comprising a nucleoside as Y group can be prepared, for example, by the following reactions. Depending on the type and reactivity of the functional groups provided by Y, the professional is able to adapt the following examples, if necessary including the phases of protection/non-protection of the sensitive functional groups or those that can interact with the coupling reaction.

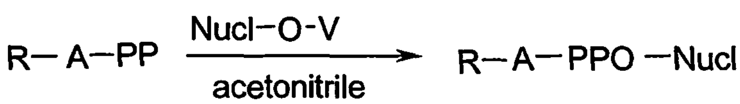


Reaction A

or

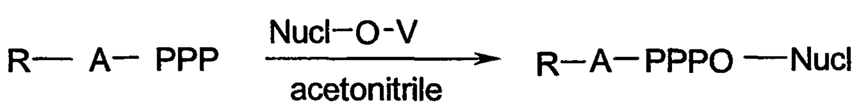


Reaction B

where -O-V is a good group beginning with V chosen, for example, from among tosyle, mesyle, triflyle, brosyle or bromium, PP represents the pyrophosphate group, PPP represents the triphosphate group, R-A- has the above mentionned meaning and Nucl is a nucleoside. Preferably, Nucl-O-V is selected from the group consisting of : 5'-O-Tosyladenosine, 5'-O-Tosyluridine, 5'-O-Tosylcytidine, 5'-O-Tosylthymidine or 5'-O-Tosyl-2'-deoxyadenosine.

For example, for the compound with R of group 1), the reaction procedure can be the following:

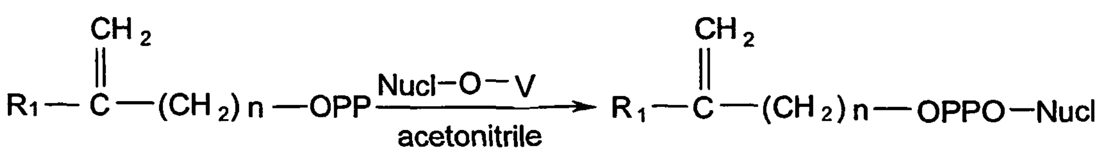


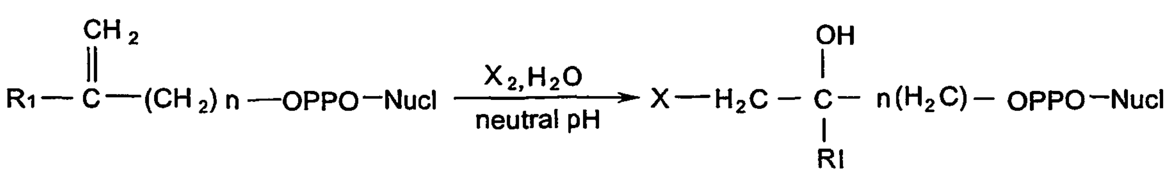

where -O-V is a good group beginning with V chosen, for example, from among tosyle, mesyle, triflyle, brosyle or bromium, PP represents the pyrophosphate group and Nucl is a nucleoside. Preferably, Nucl-O-V is selected from the group consisting of : 5'-O-Tosyladenosine, 5'-O-Tosyluridine, 5'-O-Tosylcytidine, 5'-O-Tosylthymidine or 5'-O-Tosyl-2'-deoxyadenosine as described in Davisson et al, (1987). Neutral pH is a nucleophile substitution reaction that can be carried out in conditions similar to those described by Davisson et al, (1987); and Davisson et al. (1986).

This reaction can also be used to prepare compound comprising a monosaccharide as group Y. In this case, Nucl-O-V is replaced by MonoSac-O-V, wherein Monosac is monosaccharide. For example, it is possible to use the MonoSac-O-Y group corresponding to compound Methyl-6-O-tosyl-alpha-D-galactopyranoside as described in publication Nilsson and Mosbach, (1980), or the commercially available mannose triflate compound.

This reaction can further be used to prepare compound comprising a oligosaccharide as group Y. In this case, Nucl-O-V is replaced by oligoSac-O-V, wherein oligoSac is an oligosaccharide. For example, it is possible to use the oligoSac-O-Y group corresponding to compound $6^A$-O-p-Toluenesulfonyl-β-cyclodextrin as described in publication (Organic syntheses, Vol. 77, p 225-228).

This reaction can be used to prepare compound comprising a polysaccharide as group Y. In this case, Nucl-O-V is replaced by polySac-O-V, wherein polySac is a polysaccharide. For example, it is possible to use the polySac-O-Y group corresponding to tosylated polysaccharide as described in publication Nilsson et al., (1981); and Nilsson and Mosbach, (1980). This coupling technique based on the activation of the hydroxyl groups of a polysaccharide support by tosylation allows for covalent coupling in an aqueous or an organic medium.

This reaction can also be used for preparing compound comprising an aldehyde derivative as group Y by choosing, instead of Nucl, a derivative including a protected aldehyde function in the form of an acetal or any other group protecting this function.

Alternatively, compounds comprising a nucleoside as Y group can be prepared by the following reaction:

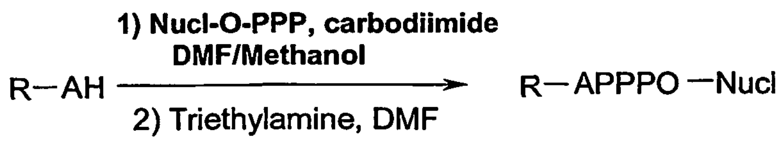


Reaction C

where PPP represents the triphosphate group, R-A has the above mentionned meaning, DMF is dimethylformamide, and Nucl is a nucleoside. This reaction can be carried out in conditions similar to those described by Knorre et al.(1976), or by Bloom et al., United States Patent No. 5,639,653 (1997), from alcohol and a nucleotide with formula Nucl-O-PPP.

For example, for the compound with R of group 1), the reaction procedure can be the following:

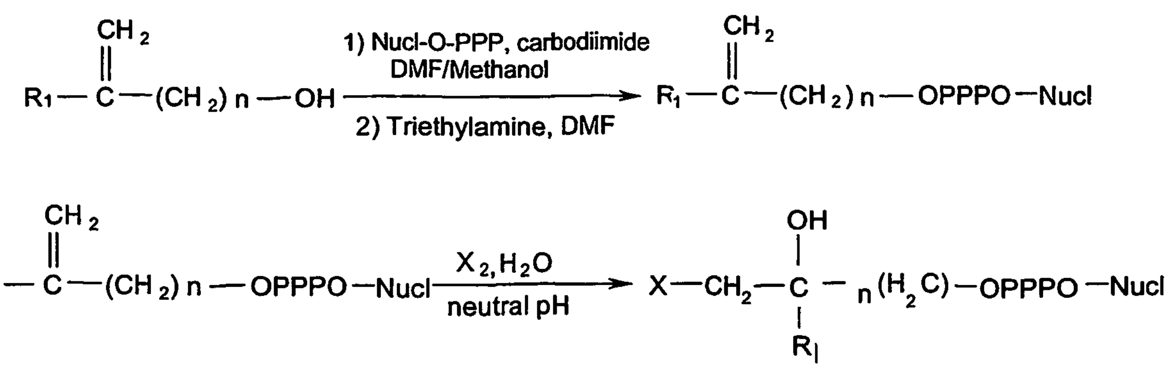


where PPP represents the triphosphate group, DMF is dimethylformamide, and Nucl is a nucleoside.

This reaction can also be applied to the preparation of oligonucleotides 5'-triphosphate ?-esters as indicated by the authors of publication Knorre et al. (1976).

Compounds comprising a nucleic acid as Y group, more particularly a ribonucleic acid, can be prepared in conditions similar to those described in publication F. Huang et al (1997). The authors describe a universal method from catalytic RNA that is applicable to any molecule comprising a free terminal phosphate group. Compounds structurally related to the phosphohalohydrine group such as isopentenyl pyrophosphate or thiamine pyrophosphate are used or mentioned by these authors (see p. 8968 of F. Huang et al (1997)). It should also be noted that the experimental conditions for the coupling procedure (in particular pH conditions) described in the section « Reaction of Isolate 6 pppRNA

with phosphate containing Nucleophiles » on page 8965 are compatible with the presence of a halohydrine function.

Compounds comprising an amino acid, a peptide or a protein derivative as Y group can be obtained using the well known reactivity of their primary amine or thiol function on an epoxyde function ($S_N2$ reaction). This type of coupling classically involves an intermediate group still called "linker" bearing an epoxyde function. An example of a reaction procedure using this type of coupling is provided below.

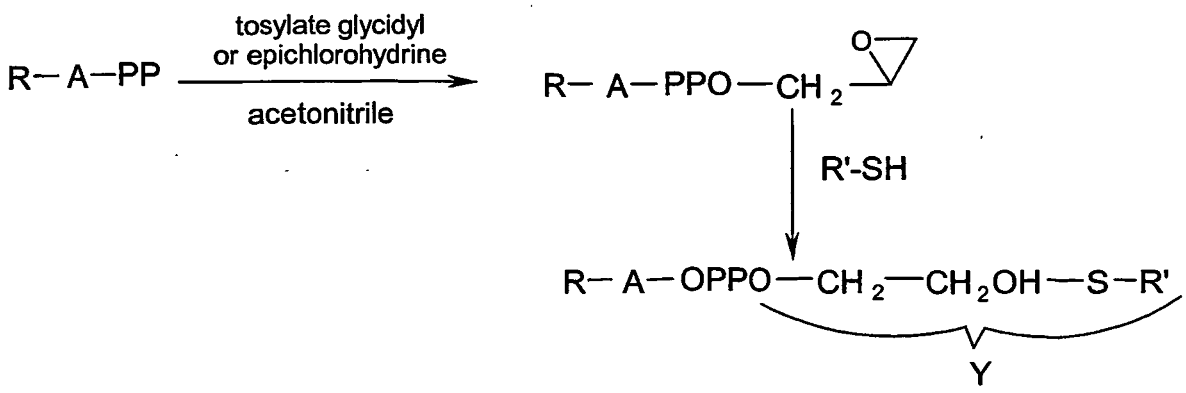


Reaction D

where PP represents the pyrophosphate group, R-A has the above mentionned meaning and R'-SH is an amino acid, a peptide or a protein derivative. The first phase can be carried out in conditions similar to those described by Davisson et al. (1987) and Davisson et al, (1986), from the tetrabutylammonium salt of the initial compound and commercially available compounds such as glycidyl tosylate or epichlorohydrine. This reaction can also be carried out with thriphosphate compounds. Alternatively, a primary amine R'-$NH_2$ can be used instead of R'-SH. Without the reaction with R'-SH, the first reaction can be used to prepare compound comprising an epoxyde derivative.

Alternatively, compounds comprising an amino acid, a peptide or a protein derivative as Y group can be prepared by the following reaction:

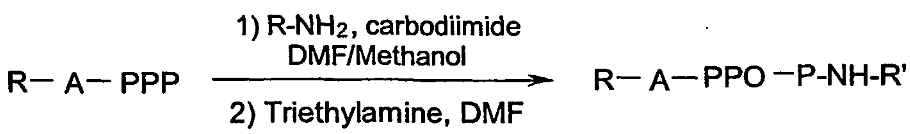


Reaction E

where PPP represents the triphosphate group, PP represents the pyrophosphate group, P represents the phosphate group, R-A has the above mentionned meaning and R'-NH is an amino acid, a peptide or a protein derivative. The reaction can be carried out in conditions similar to those described by Knorre et al. (1976), from compound (R-A-PPP) and an amino acid, peptide or a protein with formula R-$NH_2$. This reaction involves the protection of the sensitive functions of compound R-$NH_2$ or can react with the carbodiimide (in particular, the carboxyl function).

Tri or tetra-n-butylammonium salts of phosphoric, pyrophosphoric, triphosphoric, tetra-phosphoric or polyphosphoric acid can be prepared from commercially available corresponding acids. Derivatives with a related structure such as derivatives of methanetrisphosphonic acid described in publication Liu et al (1999), can also be prepared according to the reaction procedure.

The above mentionned reactions can be extrapolated to a very large spectrum of molecules or biomolecules by using the reactivity of the hydroxyl, amine, phosphate or thiol functions. Thereby, inositol derivatives can be prepared according to reactions A or B by activation of the hydroxyl function. Derivatives of folic acid (vitamin B9) or tetrahydrofolic acid can be prepared according to reactions D or E by calling on the reactivity of the primary amine function.

Of course, other types of coupling can be considered and the professional can have access to a large choice of reactions.

Thereby, coupling by phosphorylation of carboxylic acid or phenol groups can be used for the formation of fatty acid, lipid or certain flavonoid derivatives.

The cytokines

As indicated above, the method is based on the use of particular combinations of active agents, according to particular schedules. The invention more preferably uses a cytokine in combination with a synthetic activator, the cytokine being an interleukin-2 polypeptide.

The interleukin-2 polypeptide may be of human of animal origin, preferably of human origin. It may comprise the sequence of a wild-type human (or animal) IL-2 protein, or any biologically active fragment, variant or analogue thereof, i.e., any fragment, variant or analogue capable of binding to an IL-2 receptor and of inducing activation of γδT cells in the method of this invention.

The sequence of reference, wild-type human interleukin-2 proteins is available in the art, such as in Genbank, under accession numbers NP000577 ; AAK26665 ; PO1585 ; XP035511, for instance.

The term "variant" designates, in particular, any natural variants, such as those resulting from polymorphism (s), splicing(s), mutation(s), etc. Such naturally-occurring variants may thus comprise one or several mutation, deletion, substitution and/or addition of one or more amino acid residues, as compared to a reference IL-2 protein sequence.

The term "variant" also includes IL-2 polypeptides originating from various mammalian species, such as for instance rodent, bovine, porcine, equine, etc. More preferably, the IL-2 polypeptide is of human origin, i.e., comprises the sequence of a human IL-2 protein or a variant, fragment or analogue thereof.

The term "variant" also includes synthetic IL-2 variants, such as any synthetic polypeptide comprising one or several mutation, deletion, substitution and/or addition of one or more amino acid residues, as compared to a reference IL-2 protein sequence, and capable of binding to an IL-2 receptor and of inducing activation of γδT cells in the method of this invention. Preferred synthetic IL-2 variants have at least 75% identity in amino acid sequence with the primary sequence of an IL-2 reference protein, more preferably at least 80%, even more preferably at least 85 or 90%. The identity between sequences may be determined according to various known methods such as, typically, using the CLUSTAL method.

Variants also include IL-2 polypeptides encoded by a nucleic acid sequence that hybridize, under conventional, moderate stringency, with the nucleic acid sequence encoding a reference IL-2 protein, or a fragment thereof. Hybridization conditions are, for instance incubation at 40-42˚C for 12 hours in 50% formamide, 5 X SSPE, 5 X Denhardt's solution, 0.1% SDS.

The IL-2 polypeptide may also be any fragment of a reference IL-2 protein which retain the ability to bind to an IL-2 receptor and to induce activation of γδT cells in the method of this invention. Such fragments contain, at least, one functional domain of IL-2, such as the receptor binding site. Fragments contain preferably at least 40%, 50% or, preferably, at least 60% of the complete reference sequence.

Analogues designate polypeptides using the same receptor as Interleukin-2 and thus mediating similar activation signal in a γδ T lymphocyte.

The interleukin-2 polypeptide may further comprise heterologous residues added to the natural sequence, such as additional amino acids, sugar, lipids, etc. This may also be chemical, enzymatic or marker (e.g., radioactive) groups. The added residues or moiety may represent a stabilizing agent, a transfection-facilitating agent, etc.

The IL-2 polypeptides may be in soluble, purified form, or conjugated or complexed with an other molecule, such as a biologically active peptide, protein, lipid, etc. The IL-2 polypeptide may be produced according to techniques known in the art, such as by chemical synthesis, enzymatic synthesis, genetic (e.g., recombinant DNA) synthesis, or a combination thereof. An IL-2 polypeptide of pharmaceutical grade may also be obtained from commercial sources.

The interleukin-2 polypeptide is preferably administered at low doses, i.e. at doses that are sufficient to target in vivo cells that express the high affinity receptor for IL2, defined as the tri-molecular complex CD25/CD122/CD130. Practically, in human, such doses have been experimentally defined in clinical trials as being comprised between 0.2 and 2 million units per square meters, when injected subcutaneously (see for example buzio et al 2001).

The IL-2 polypeptide is preferably administered by injection of between 0.1 and 3 Million Units per day, over a period of 1 to 10 days. Preferably, daily doses of between 0.2 and 2 MU per day, even more preferably between 0.2 and 1.5 MU, further preferably between 0.2 and 1 MU, are being administered. The daily dose may be administered as a single injection or in several times, typically in two equal injections. The IL-2 treatment is preferably maintained over between 1 and 9 days, even more preferably during 3 to 7 days. Optimum effect seems to be achieved after 5 days treatment.

## PREFERRED EMBODIMENTS OF THE INVENTION

In preferred embodiments, compounds BrHPP, CBrHPP, HDMAPP, CHDMAPP and epoxPP are used according to the methods of the invention.

*BrHPP and EpoxPP*

The synthesis of BrHPP is described in Example 1 and in Espinosa (2001), the disclosure of which is incorporated herein by reference. The synthesis of EpoxPP is described in European Patent No. 1109818B1, the disclosure of which is incorporated herein by reference.

(1) The present invention relates especially to the treatment of a disease, especially a tumor, especially a solid tumor, more especially one of the preferred diseases as defined above or below, characterized in that a compound of formula II, III or VIII, especially BRHPP or EpoxPP, is administered more than once, with a two-weekly up to eight-weekly, preferably between three-weekly or four-weekly interval to a human in a dose that is calculated according to the formula (A)

$$\text{single dose (mg/kg)}=(0.1 \text{ to } y) * N \qquad (A)$$

where N (a whole or fractional number) is the number of weeks between treatments (about two to about eight weeks), that is N is about 2 to about 8, preferably between about 3 to 4; more preferably, the treatment dose is calculated according to the formula B,

$$\text{single dose (mg/kg)}=(5 \text{ to } 100) * N; \qquad (B)$$

even more preferably according to the formula C,

$$\text{single dose (mg/kg)}=(10 \text{ to } 100) * N; \qquad (C)$$

or still more preferably according to the formula D,

$$\text{single dose (mg/m2)}=(5 \text{ to } 60) * N \qquad (D)$$

where, in each of formulae A to D, N is about 2 to about 8 or preferably about 3 to 4 (corresponding to intervals of about 2 to about 8 weeks and about 3 to about 4 weeks between treatments);
the compound of formula II or III, especially BRHPP, administration preferably taking place:

(a) about three-weekly to about four weekly, preferably three-weekly or four-weekly, in a human in a dose that lies between about 0.1 mg/kg and about 1.2 g/kg, preferably between about 10 mg/kg and about 1.2 g/kg, more preferably between about 5 mg/kg and about 100 mg/kg, even more preferably between about 5 mg/kg and 60 mg/kg, or preferably about 20 mg/kg; or
(b) about four-weekly to about eight weekly, preferably about five-weekly, six-weekly, seven-weekly or eight-weekly, in a human in a dose that lies dose is between about between about 0.1 mg/kg and about 1.2 g/kg, preferably between about 10 mg/kg and about 1.2 g/kg, more preferably between about 5 mg/kg and about 100 mg/kg, even more preferably between about 5 mg/kg and 60 mg/kg, or preferably about 20 mg/kg;

the administration preferably taking place by i.v. infusion during 2 to 120 min, more preferably during about 5 to about 30 min, most preferably during about 10 to about 30 min, e.g. during about 30 min.

(2) The present invention preferably relates also to the treatment of a solid tumor disease, most preferably a tumor disease having metastases, said tumor being selected from a gastrointestinal, e.g. colorectal; lung tumor, especially a non-small cell lung carcinoma; a breast tumor; an epidermoid tumor; a renal; a genitourinary, e.g. prostatic; a pancreatic; and a brain tumor (and/or any metastasis thereof), most preferably a gastrointestinal tumor, especially a colorectal cancer, more especially a gastrointestinal cancer, especially a colorectal cancer; or a tumor of the genitourinary tract, especially a prostate cancer; where a compound of formula II, III or VIII, especially BRHPP or

EpoxPP, is administered to a warm-blooded animal, especially a human.

(3) The present invention also preferably relates to an in vivo regimen for stimulating a γδ T cell in an individual, preferably a regimen for treatment of a solid tumor; wherein a composition is administered to an individual such that a compound of formula II, III or VIII, especially BRHPP or EpoxPP is administered once in a dose that is

(a) between about the EC50 value and the EC100 value, more preferably at least 110%, 120%, 150% or 175% of the EC50, to a human
(b) between about 0.1 mg/kg and about 100 mg/kg, to a human

and, if required, one or more (preferably at least two, at least three, at least four, at least five, at least six, at least eight or at least ten) further doses each within the dose range mentioned above for the first dose are administered in further treatment cycles, preferably each dose after a period of time that allows for sufficient recovery of the γδ T cell population to basal levels in the treated individual from each preceding dose administration, especially more than one week, more than two weeks after the preceding treatment, more especially two to eight weeks, most especially three to four weeks after the preceding treatment, especially three weeks after that treatment.
More preferably, under (1) to (3) a compound of formula II, III or VIII, especially BRHPP or EpoxPP is administered three-weekly to a human in a dose that lies between about 0.1 mg/kg and about 1.2 g/kg, preferably between about 10 mg/kg and about 1.2 g/kg, more preferably between about 5 mg/kg and about 100 mg/kg, even more preferably between about 5 mg/kg and 60 mg/kg, or preferably about 20 mg/kg;; or a compound of formula II, III or VIII, especially BRHPP or EpoxPP is administered four-weekly (every 4 weeks) in a dose that is between about 0.1 mg/kg and about 1.2 g/kg, preferably between about 10 mg/kg and about 1.2 g/kg, more preferably between about 5 mg/kg and about 100 mg/kg, even more preferably between about 5 mg/kg and 60 mg/kg, or preferably about 20 mg/kg;. This dose is preferably administered to the human by intravenous (i.v.) administration during 2 to 120 min, more preferably during about 5 to about 30 min, most preferably during about 10 to about 30 min, e.g. during about 30 min.
More preferably, said treatment is repeated until disease progression, unacceptable toxicity, 1 or preferably 2 cycles beyond determination of a complete response, or patient withdrawal of consent for any reason is encountered.

(4) The present invention preferably also relates to an in vivo regimen for the treatment of a solid tumor disease, especially (i) of a solid tumor selected from a gastrointestinal, e.g. colorectal; lung tumor, especially a non-small cell lung carcinoma; a breast tumor; an epidermoid tumor; a renal; a genitourinary, e.g. prostatic; a pancreatic; and a brain tumor (and/or any metastasis thereof), most preferably a gastrointestinal tumor, especially a colorectal cancer, more especially a gastrointestinal cancer, especially a colorectal cancer; or a tumor of the genitourinary tract, especially a prostate cancer; especially where such tumor is metastatic, wherein a compound of formula II, III or VIII, especially BRHPP or EpoxPP, is administered between once-weekly and eight-weekly to a warm-blooded animal in a dose that is below 80%, more preferably below 50% of the maximal tolerable dose (MTD) or highest dose tested in non-human animals.
Preferably, in the case of weekly treatment of a human with said compound of formula II, III or VIII, especially BRHPP or EpoxPP, the dose is in the range of about 1 to about 60%, preferably about 10 to about 60%, e.g. about 5 to about 35% of the MTD, for example in the range of about 30 to about 35% of the MTD. Preferably, for BRHPP the dose is in the range of about 5 to about 60%, preferably about 10 to about 60%, especially in the range of about 10 to about 45%, most especially in the range of about 30 to about 45% of the MTD.

(5) The present invention preferably also relates to an in vivo regimen for the treatment of a disease, especially a solid tumor disease selected from a gastrointestinal, e.g. colorectal; lung tumor, especially a non-small cell lung carcinoma; a breast tumor; an epidermoid tumor; a renal; a genitourinary, e.g. prostatic; a pancreatic; and a brain tumor (and/or any metastasis thereof), most preferably a gastrointestinal tumor, especially a colorectal cancer, more especially a gastrointestinal cancer, especially a colorectal cancer; or a tumor of the genitourinary tract, especially a prostate cancer; especially where such tumor is metastatic, wherein a compound of formula II, III or VIII, especially BRHPP or EpoxPP, is administered between once-weekly and eight-weekly to a warm-blooded animal in a dose that is between the Efficient Concentration value giving half the maximum effect (EC50) and the Efficient Concentration value giving the maximal effect (EC100), or that is between the EC50 and 200% of the EC50, or preferably at least 110%, 120%, 130%, 150%, 160%, 175% or 200% of the EC50 value.

*CBrHPP*

The synthesis of CBrHPP can be carried according to any suitable method. In a preferred example, 3-methylbut-3-enyl pyrophosphonate (C-IPP) is prepared according to the methods of PCT patent publication no. WO 03/050128,

Brondino et al, (1996), or
Valentijn et al.(1991), and is converted to CBrHPP according to the methods of Espinosa et al (2001a).

(1) The present invention relates especially to the treatment of a disease, especially a tumor, especiall a solid tumor, more especially one of the preferred diseases as defined above or below, characterized in that a CBrHPP compound is administered more than once, with a two-weekly up to eight-weekly, preferably between three-weekly and four-weekly interval to a human in a dose that is calculated according to the formula (A)

$$\text{single dose (mg/kg)}=(0.1 \text{ to } y) * N \qquad \text{(A)}$$

where N (a whole or fractional number) is the number of weeks between treatments (about two to about eight weeks), that is N is about 2 to about 8, preferably between about 3 to 4; more preferably, the treatment dose is calculated according to the formula B,

$$\text{single dose (mg/kg)}=(5 \text{ to } 100) * N; \qquad \text{(B)}$$

even more preferably according to the formula C,

$$\text{single dose (mg/kg)}=(10 \text{ to } 100) * N; \qquad \text{(C)}$$

or still more preferably according to the formula D,

$$\text{single dose (mg/m2)}=(5 \text{ to } 60) * N \qquad \text{(D)}$$

where, in each of formulae A to D, N is about 2 to about 8 or preferably about 3 to 4 (corresponding to intervals of about 2 to about 8 weeks and about 3 to about 4 weeks between treatments);
the CBrHPP administration preferably taking place:

(a) about three-weekly to about four weekly, preferably three-weekly or four-weekly, in a human in a dose that lies between about 0.1 mg/kg and about 1.2 g/kg, preferably between about 10 mg/kg and about 1.2 g/kg, more preferably between about 5 mg/kg and about 100 mg/kg, even more preferably between about 5 mg/kg and 60 mg/kg, or preferably about 20 mg/kg; or
(b) about four-weekly to about eight weekly, preferably about five-weekly, six-weekly, seven-weekly or eight-weekly, in a human in a dose that lies dose is between about 0.1 mg/kg and about 1.2 g/kg, preferably between about 10 mg/kg and about 1.2 g/kg, more preferably between about 5 mg/kg and about 100 mg/kg, even more preferably between about 5 mg/kg and 60 mg/kg, or preferably about 20 mg/kg;

the administration preferably taking place by i.v. infusion during 2 to 120 min, more preferably during about 5 to about 30 min, most preferably during about 10 to about 30 min, e.g. during about 30 min.

(2) The present invention preferably relates also to the treatment of a tumor disease, most preferably a tumor disease having metastases, said tumor being selected from a gastrointestinal, e.g. colorectal; lung tumor, especially a non-small cell lung carcinoma; a breast tumor; an epidermoid tumor; a renal; a genitourinary, e.g, prostatic; a pancreatic; and a brain tumor (and/or any metastasis thereof), most preferably a gastrointestinal tumor, especially a colorectal cancer, more especially a gastrointestinal cancer, especially a colorectal cancer; or a tumor of the genitourinary tract, especially a prostate cancer; wherein CBrHPP is administered to a warm-blooded animal, especially a human.

(3) The present invention also preferably relates to an in vivo regimen for stimulating a γδ T cell in an individual, preferably a regimen for treatment of a tumor disease, preferably a solid tumor, or an autoimmune disorder or an infectious disease; wherein CBrHPP is administered once in a dose that is

(a) between about the EC50 value and the EC100 value, more preferably at least 110%, 120%, 150% or 175% of the EC50, to a human
(b) between about 0.1 mg/kg and about 100 mg/kg, to a human

and, if required, one or more (preferably at least two, at least three, at least four, at least five, at least six, at least eight or at least ten) further doses each within the dose range mentioned above for the first dose are administered in further treatment cycles, preferably each dose after a period of time that allows for sufficient recovery of the γδ T cell population to basal levels in the treated individual from each preceding dose administration, especially more than one week, more than two weeks after the preceding treatment, more especially two to eight weeks, most especially three to four weeks after the preceding treatment, especially three weeks after that treatment.
More preferably, under (1) to (3) CBrHPP is administered three-weekly to a human in a dose that lies between about 0.1 mg/kg and about 1.2 g/kg, preferably between about 10 mg/kg and about 1.2 g/kg, more preferably between about 5 mg/kg and about 100 mg/kg, even more preferably between about 5 mg/kg and 60 mg/kg, or preferably about 20 mg/kg; or CBrHPP is administered four-weekly (every 4 weeks) in a dose that is between about 0.1 mg/kg and about 1.2 g/kg, preferably between about 10 mg/kg and about 1.2 g/kg, more preferably between about 5 mg/kg and about 100 mg/kg, even more preferably between about 5 mg/kg and 60 mg/kg, or preferably about 20 mg/kg. This dose is preferably administered to the human by intravenous (i.v.) administration during 2 to 120 min, more preferably during about 5 to about 30 min, most preferably during about 10 to about 30 min, e.g. during about 30 min. More preferably, said treatment is repeated until disease progression, unacceptable toxicity, 1 or preferably 2 cycles beyond determination of a complete response, or patient withdrawal of consent for any reason is encountered.

(4) The present invention preferably also relates to an in vivo regimen for the treatment of a tumor disease, especially (i) of a solid tumor selected from a gastrointestinal, e.g. colorectal; lung tumor, especially a non-small cell lung carcinoma; a breast tumor; an epidermoid tumor; a renal; a genitourinary, e.g. prostatic; a pancreatic; and a brain tumor (and/or any metastasis thereof), most preferably a gastrointestinal tumor, especially a colorectal cancer, more especially a gastrointestinal cancer, especially a colorectal cancer; or a tumor of the genitourinary tract, especially a prostate cancer; especially where such tumor is metastatic, wherein CBrHPP, is administered between once-weekly and eight-weekly to a warm-blooded animal in a dose that is below 80%, more preferably below 50% of the maximal tolerable dose (MTD).
Preferably, in the case of weekly treatment of a human with said CBrHPP compound, the dose is in the range of about 1 to about 60%, preferably about 10 to about 60%, e.g. about 5 to about 35% of the MTD, for example in the range of about 30 to about 35% of the MTD. Preferably, for CBrHPP the dose is in the range of about 5 to about 60%, preferably about 10 to about 60%, especially in the range of about 10 to about 45%, most especially in the range of about 30 to about 45% of the MTD. In a special case, the dose can be between about 2 and about 18 mg/m2 for CBrHPP.

(5) The present invention preferably also relates to an in vivo regimen for the treatment of a disease, especially a solid tumor disease selected from a gastrointestinal, e.g. colorectal; lung tumor, especially a non-small cell lung carcinoma; a breast tumor; an epidermoid tumor; a renal; a genitourinary, e.g. prostatic; a pancreatic; and a brain tumor (and/or any metastasis thereof), most preferably a gastrointestinal tumor, especially a colorectal cancer, more especially a gastrointestinal cancer, especially a colorectal cancer; or a tumor of the genitourinary tract, especially a prostate cancer; especially where such tumor is metastatic, wherein CBrHPP is administered between once-weekly and eight-weekly to a warm-blooded animal in a dose that is between the Efficient Concentration value giving half the maximum effect (EC50) and the Efficient Concentration value giving the maximal effect (EC100), or that is between 110% and 200% of the EC50, or preferably at least 110%, 120%, 130%, 150%, 160%, 175% or 200% of the EC50 value.

*HDMAPP*

Since the isolation of HDMAPP from E. coli cells deficient in the lytB component of the nonmevalonate (MEP) pathway, described in Hintz et al (2001), the chemical synthesis of HDMAPP has been achieved by a number of laboratories. The synthetic HDMAPP and the natural compound isolated from *E. coli lytB* mutants displayed identical activities in stimulating Vγ9/Vδ2 T cells. The reactivity of human peripheral blood mononuclear cells towards HDMAPP was restricted Vγ9/Vδ2 T cells, leading to up-regulation of activation markers on the cell surface, secretion of pro-inflammatory cytokines, and expansion of the Vγ9/Vδ2 subpopulation in the presence of co-stimulation provided by IL-2. HDMAPP was reported to have an $EC_{50}$ value of approx. 0.1 nM (compared to IPP with an $EC_{50}$ of approx. 1 μM), leading to the assumption that HDMAPP exclusively accounted for the known Vγ9/Vδ2 T cell reactivity towards pathogenic bacteria such as *Brucella, Campylobacter, Ehrlichia, E. coli, Francisella, Listeria, Mycobacterium, Pseudomonas, Salmonella*,

and *Yersinia*, as well as to the protozoan parasites *Plasmodium* and *Toxoplasma.*

In vitro in vivo pharmacodynamics of Vγ9/Vδ2 $^{+}$T cell stimulation as elucidated by the inventors have now shown that HDMAPP is surprisingly effective in regulating γδ T cell activity, and may be administered to mammals in a low dose administration regimens.

Preferred methods for the synthesis of HDMAPP are described in Example 2 and in Wolff et al, Tetrahedron Letters (2002) 43:2555 and Hecht et al, Tetrahedron Letters (2002) 43: 8929.

(1) The present invention relates especially to the treatment of a disease, especially a tumor, especially a solid tumor, more especially one of the preferred diseases as defined above or below, characterized in that a compound of formula XII, especially HDMAPP, is administered more than once, with a two-weekly up to eight-weekly, preferably between three-weekly and four-weekly interval to a human in a dose that is calculated according to the formula (A)

$$\text{single dose (mg/kg)=(0.1 to y) * N} \qquad \text{(A)}$$

where N (a whole or fractional number) is the number of weeks between treatments (about two to about eight weeks), that is N is about 2 to about 8, preferably between about 3 to 4; more preferably, the treatment dose is calculated according to the formula B,

$$\text{single dose (mg/kg)=(0.001 to 100) * N;} \qquad \text{(B)}$$

even more preferably according to the formula C,

$$\text{single dose (mg/kg)=(0.01 to 5) * N;} \qquad \text{(C)}$$

or still more preferably according to the formula D,

$$\text{single dose (mg/m2)=(0.02 to 2.5) * N} \quad \text{(D)}$$

where, in each of formulae A to D, N is about 2 to about 8 or preferably about 3 to 4 (corresponding to intervals of about 2 to about 8 weeks and about 3 to about 4 weeks between treatments);
the a compound of formula XII, especially HDMAPP, administration preferably taking place:

(a) about three-weekly to about four weekly, preferably three-weekly or four-weekly, in a human in a dose that lies between about 1 μg/kg and about 100 mg/kg, preferably between about 10 μg/kg and about 20 mg/kg, more preferably between about 20 μg/kg and about 5 mg/kg, even more preferably between about 20 μg/kg and 2.5 mg/kg, or preferably about 0.5 mg/kg, or preferably about 0.5 mg/kg; or
(b) about four-weekly to about eight weekly, preferably about five-weekly, six-weekly, seven-weekly or eight-weekly, in a human in a dose that lies dose is between about 1 μg/kg and about 100 mg/kg, preferably between about 10 μg/kg and about 20 mg/kg, more preferably between about 20 μg/kg and about 5 mg/kg, even more preferably between about 20 μg/kg and 2.5 mg/kg, or preferably about 0.5 mg/kg, or preferably about 0.5 mg/kg;

the administration preferably taking place by i.v. infusion during 2 to 120 min, more preferably during about 5 to about 30 min, most preferably during about 10 to about 30 min, e.g. during about 30 min.

(2) The present invention preferably relates also to the treatment of a tumor disease, most preferably a tumor disease having metastases, said tumor being selected from a gastrointestinal, e.g. colorectal; lung tumor, especially a non-small cell lung carcinoma; a breast tumor; an epidermoid tumor; a renal; a genitourinary, e.g. prostatic; a pancreatic; and a brain tumor (and/or any metastasis thereof), most preferably a gastrointestinal tumor, especially a colorectal cancer, more especially a gastrointestinal cancer, especially a colorectal cancer; or a tumor of the genitourinary tract, especially a prostate cancer; a said compound of formula XII, especially HDMAPP, is administered to a warm-blooded animal, especially a human.

(3) The present invention also preferably relates to an in vivo regimen for stimulating a γδ T cell in an individual, preferably a regimen for treatment of a tumor disease, preferably a solid tumor, or an autoimmune disorder or an infectious disease; wherein a compound of formula XII, especially HDMAPP is administered once in a dose that is

(a) between about the EC50 and the EC100, more preferably at least 110%, 120%, 150% or 175% of the EC50, to a human
(b) between about 10 μg/kg and about 20 mg/kg, to a human

and, if required, one or more (preferably at least two, at least three, at least four, at least five, at least six, at least eight or at least ten) further doses each within the dose range mentioned above for the first dose are administered in further treatment cycles, preferably each dose after a period of time that allows for sufficient recovery of the γδ T cell population to basal levels in the treated individual from each preceding dose administration, especially more than one week, more than two weeks after the preceding treatment, more especially two to eight weeks, most especially three to four weeks after the preceding treatment, especially three weeks after that treatment.
More preferably, under (1) to (3) a compound of formula XII, especially HDMAPP is administered three-weekly to a human in a dose that lies between about 1 μg/kg and about 100 mg/kg, preferably between about 10 μg/kg and about 20 mg/kg, more preferably between about 20 μg/kg and about 5 mg/kg, even more preferably between about 20 μg/kg and 2.5 mg/kg, or preferably about 0.5 mg/kg, or preferably about 0.5 mg/kg; or a compound of formula XII, especially HDMAPP is administered four-weekly (every 4 weeks) in a dose that is between about 1 μg/kg and about 100 mg/kg, preferably between about 10 μg/kg and about 20 mg/kg, more preferably between about 20 μg/kg and about 5 mg/kg, even more preferably between about 20 μg/kg and 2.5 mg/kg, or preferably about 0.5 mg/kg, or preferably about 0.5 mg/kg. This dose is preferably administered to the human by intravenous (i.v.) administration during 2 to 120 min, more preferably during about 5 to about 30 min, most preferably during about 10 to about 30 min, e.g, during about 30 min.
More preferably, said treatment is repeated until disease progression, unacceptable toxicity, 1 or preferably 2 cycles beyond determination of a complete response, or patient withdrawal of consent for any reason is encountered.

(4) The present invention preferably also relates to an in vivo regimen for the treatment of a tumor disease, especially (i) of a solid tumor selected from a gastrointestinal, e.g. colorectal; lung tumor, especially a non-small cell lung carcinoma; a breast tumor; an epidermoid tumor; a renal; a genitourinary, e.g. prostatic; a pancreatic; and a brain tumor (and/or any metastasis thereof), most preferably a gastrointestinal tumor, especially a colorectal cancer, more especially a gastrointestinal cancer, especially a colorectal cancer; or a tumor of the genitourinary tract, especially a prostate cancer; especially where such tumor is metastatic, wherein a compound of formula XII, especially HDMAPP, is administered between once-weekly and eight-weekly to a warm-blooded animal in a dose that is below 80%, more preferably below 50% of the maximal tolerable dose (MTD).
Preferably, in the case of weekly treatment of a human with said a compound of formula XII, especially HDMAPP, the dose is in the range of about 1 to about 60%, preferably about 10 to about 60%, e.g. about 5 to about 35% of the MTD, for example in the range of about 30 to about 35% of the MTD. Preferably, for HDMAPP the dose is in the range of about 5 to about 60%, preferably about 10 to about 60%, especially in the range of about 10 to about 45%, most especially in the range of about 30 to about 45% of the MTD. In a special case, the dose can be between about 2 and about 18 mg/m2 for HDMAPP.

(5) The present invention preferably also relates to an in vivo regimen for the treatment of a disease, especially a solid tumor disease selected from a gastrointestinal, e.g. colorectal; lung tumor, especially a non-small cell lung carcinoma; a breast tumor; an epidermoid tumor; a renal; a genitourinary, e.g. prostatic; a pancreatic; and a brain tumor (and/or any metastasis thereof), most preferably a gastrointestinal tumor, especially a colorectal cancer, more especially a gastrointestinal cancer, especially a colorectal cancer; or a tumor of the genitourinary tract, especially a prostate cancer; especially where such tumor is metastatic, wherein a compound of formula XII, especially HDMAPP, is administered between once-weekly and eight-weekly to a warm-blooded animal in a dose that is between the Efficient Concentration value giving half the maximum effect (EC50) and the Efficient Concentration value giving the maximal effect (EC100), or that is between 110% and 200% of the EC50, or preferably at least 110%, 120%, 130%, 150%, 160%, 175% or 200% of the EC50 value.

*CHDMAPP*

The synthesis of CHDMAPP can be carried according to any suitable method. Examples include the methods

of Nakamura et al (1973), Zoretic and Zhang (1996) or Umbreit and Sharpless (1977), to produce a E-hydroxydimethylallyl type synthon prior to phosphorylation ou phosphonation. Phosphorylation or phosphonation can then be carried out according to methods described in PCT patent publication no. WO 03/050128, Brondino et al, (1996), or Valentijn et al.(1991).

(1) The present invention relates especially to the treatment of a disease, especially a tumor, especially a solid tumor, more especially one of the preferred diseases as defined above or below, characterized in that a CHDMAPP compound is administered more than once, with a two-weekly up to eight-weekly, preferably between three-weekly and four-weekly interval to a human in a dose that is calculated according to the formula (A)

$$\text{single dose (mg/kg)}=(0.1 \text{ to } y) * N \qquad \text{(A)}$$

where N (a whole or fractional number) is the number of weeks between treatments (about two to about eight weeks), that is N is about 2 to about 8, preferably between about 3 to 4; more preferably, the treatment dose is calculated according to the formula B,

$$\text{single dose (mg/kg)}=(5 \text{ to } 100) * N; \qquad \text{(B)}$$

even more preferably according to the formula C,

$$\text{single dose (mg/kg)}=(10 \text{ to } 100) * N; \qquad \text{(C)}$$

or still more preferably according to the formula D,

$$\text{single dose (mg/m2)}=(5 \text{ to } 60) * N \qquad \text{(D)}$$

where, in each of formulae A to D, N is about 2 to about 8 or preferably about 3 to 4 (corresponding to intervals of about 2 to about 8 weeks and about 3 to about 4 weeks between treatments);
the CHDMAPP administration preferably taking place:

(a) about three-weekly to about four weekly, preferably three-weekly or four-weekly, in a human in a dose that lies between about 1 μg/kg and about 100 mg/kg, preferably between about 10 μg/kg and about 20 mg/kg, more preferably between about 20 μg/kg and about 5 mg/kg, even more preferably between about 20 μg/kg and 2.5 mg/kg, or preferably about 0.5 μg/kg, or preferably about 0.5 mg/kg; or
(b) about four-weekly to about eight weekly, preferably about five-weekly, six-weekly, seven-weekly or eight-weekly, in a human in a dose that lies dose is between about 1 μg/kg and about 100 mg/kg, preferably between about 10 μg/kg and about 20 mg/kg, more preferably between about 20 μg/kg and about 5 mg/kg, even more preferably between about 20 μg/kg and 2.5 mg/kg, or preferably about 0.5 mg/kg, or preferably about 0.5 mg/kg;

the administration preferably taking place by i.v. infusion during 2 to 120 min, more preferably during about 5 to about 30 min, most preferably during about 10 to about 30 min, e.g. during about 30 min.

(2) The present invention preferably relates also to the treatment of a tumor disease, most preferably a tumor disease having metastases, said tumor being selected from a gastrointestinal, e.g. colorectal; lung tumor, especially a non-small cell lung carcinoma; a breast tumor; an epidermoid tumor; a renal; a genitourinary, e.g. prostatic; a pancreatic; and a brain tumor (and/or any metastasis thereof), most preferably a gastrointestinal tumor, especially a colorectal cancer, more especially a gastrointestinal cancer, especially a colorectal cancer; or a tumor of the genitourinary tract, especially a prostate cancer; where CHDMAPP is administered to a warm-blooded animal, especially a human.

(3) The present invention also preferably relates to an in vivo regimen for stimulating a γδ T cell in an individual,

preferably a regimen for treatment of a tumor disease, preferably a solid tumor, or an autoimmune disorder or an infectious disease; wherein CHDMAPP is administered once in a dose that is

(a) between about the EC50 and the EC100, more preferably at least 110%, 120%, 150% or 175% of the EC50, to a human
(b) between about 10 μg/kg and about 20 mg/kg, to a human

and, if required, one or more (preferably at least two, at least three, at least four, at least five, at least six, at least eight or at least ten) further doses each within the dose range mentioned above for the first dose are administered in further treatment cycles, preferably each dose after a period of time that allows for sufficient recovery of the γδ T cell population to basal levels in the treated individual from each preceding dose administration, especially more than one week, more than two weeks after the preceding treatment, more especially two to eight weeks, most especially three to four weeks after the preceding treatment, especially three weeks after that treatment.
More preferably, under (1) to (3) CHDMAPP is administered three-weekly to a human in a dose that lies between about 1 μg/kg and about 100 mg/kg, preferably between about 10 μg/kg and about 20 mg/kg, more preferably between about 20 μg/kg and about 5 mg/kg, even more preferably between about 20 μg/kg and 2.5 mg/kg, or preferably about 0.5 mg/kg, or preferably about 0.5 mg/kg; or CHDMAPP is administered four-weekly (every 4 weeks) in a dose that is between about 1 μg/kg and about 100 mg/kg, preferably between about 10 μg/kg and about 20 mg/kg, more preferably between about 20 μg/kg and about 5 mg/kg, even more preferably between about 20 μg/kg and 2.5 mg/kg, or preferably about 0.5 mg/kg, or preferably about 0.5 mg/kg. This dose is preferably administered to the human by intravenous (i.v.) administration during 2 to 120 min, more preferably during about 5 to about 30 min, most preferably during about 10 to about 30 min, e.g. during about 30 min.
More preferably, said treatment is repeated until disease progression, unacceptable toxicity, 1 or preferably 2 cycles beyond determination of a complete response, or patient withdrawal of consent for any reason is encountered.

(4) The present invention preferably also relates to an in vivo regimen for the treatment of a tumor disease, especially (i) of a solid tumor selected from a gastrointestinal, e.g. colorectal; lung tumor, especially a non-small cell lung carcinoma; a breast tumor; an epidermoid tumor; a renal; a genitourinary, e.g. prostatic; a pancreatic; and a brain tumor (and/or any metastasis thereof), most preferably a gastrointestinal tumor, especially a colorectal cancer, more especially a gastrointestinal cancer, especially a colorectal cancer; or a tumor of the genitourinary tract, especially a prostate cancer; especially where such tumor is metastatic, wherein CHDMAPP is administered between once-weekly and eight-weekly to a warm-blooded animal in a dose that is below 80%, more preferably below 50% of the maximal tolerable dose (MTD).
Preferably, in the case of weekly treatment of a human with said CHDMAPP the dose is in the range of about 1 to about 60%, preferably about 10 to about 60%, e.g. about 5 to about 35% of the MTD, for example in the range of about 30 to about 35% of the MTD. Preferably, for CHDMAPP, the dose is in the range of about 5 to about 60%, preferably about 10 to about 60%, especially in the range of about 10 to about 45%, most especially in the range of about 30 to about 45% of the MTD. In a special case, the dose can be between about 2 and about 18 mg/m2 for CHDMAPP.

(5) The present invention preferably also relates to an in vivo regimen for the treatment of a disease, especially a solid tumor disease selected from a gastrointestinal, e.g. colorectal; lung tumor, especially a non-small cell lung carcinoma; a breast tumor; an epidermoid tumor; a renal; a genitourinary, e.g. prostatic; a pancreatic; and a brain tumor (and/or any metastasis thereof), most preferably a gastrointestinal tumor, especially a colorectal cancer, more especially a gastrointestinal cancer, especially a colorectal cancer; or a tumor of the genitourinary tract, especially a prostate cancer; especially where such tumor is metastatic, wherein CHDMAPP is administered between once-weekly and eight-weekly to a warm-blooded animal in a dose that is between the Efficient Concentration value giving half the maximum effect (EC50) and the Efficient Concentration value giving the maximal effect (EC100), or that is between 110% and 200% of the EC50, or preferably at least 110%, 120%, 130%, 150%, 160%, 175% or 200% of the EC50 value.

## EXAMPLES

### EXAMPLE 1

### Synthesis of BrHPP

All glassware and equipment were dried for several hours prior to use. Unless otherwise stated, the reagents

and starting material were from Fluka. Trisodium (*R*,*S*)-3-(bromomethyl)-3-butanol-1-yl-diphosphate (BrHPP) was produced as white amorphous powder by the following procedure. Tosyl chloride (4.8 g, 25 mmol) and 4-(*N*,*N*-dimethylamino-) pyridine (3.4 g, 27.5 mmol; Aldrich) were mixed under magnetic stirring with 90 ml of anhydrous dichloromethane in a 250-ml three-necked flask cooled in an ice bath. A solution of 3-methyl-3-butene-1-ol (2.2 g, 25 mmol) in about 10 ml of anhydrous dichloromethane was then slowly introduced with a syringe through a septum in the flask, and the ice bath was then removed. The reaction was monitored by silica gel TLC (pentane/ethyl acetate, 85:15 (v/v)). After 2 h with constant stirring, the mixture was precipitated by dilution into 1 liter of hexane and filtered, and the filtrate was concentrated under reduced pressure. This filtration/suspension step was repeated using diethyl ether, and the resulting oil was purified by liquid chromatography on silica gel (pentane/ethyl acetate, 85:15 (v/v)), yielding a yellow oil of 3-methyl-3-butene-1-yl-tosylate (5.6 g, 23.5 mmol, 94% yield) kept under dry $N_2$ at 4 ˚C (positive mode ESI-MS: *m*/*z* 241 $[M + H]^+$; *m*/*z* 258 $[M + NH_4]^+$; *m*/*z* 263 $[M + Na]^+$; $MS^2$ of *m*/*z* 258: *m*/*z* 190 ($C_5H_8$ loss)).

Disodium dihydrogen pyrophosphate (51.5 mmol, 11.1 g) dissolved in 100 ml of deionized water (adjusted to pH 9 with $NH_4OH$) was passed over a cation exchange DOWEX 50WX8 (42 g, 200 meq of form $H^+$) column and eluted with 150 ml of deionized water (pH 9). The collected solution was neutralized to pH 7.3 using tetra-*n*-butyl ammonium hydroxide and lyophilized. The resulting hygroscopic powder was solubilized with anhydrous acetonitrile and further dried by repeated evaporation under reduced pressure. The resulting Tris (tetra-n-butyl ammonium) hydrogenopyrophosphate (97.5% purity by HPAEC; see below) was stored (concentration, ~0.5 M) at -20˚C in anhydrous conditions under molecular sieves. 100 ml of a solution containing 50 mmol of Tris (tetra-*n*-butyl ammonium) hydrogenopyrophosphate (0.5 M, 2.5 eq) in anhydrous acetonitrile under magnetic stirring in a 250-ml three-necked flask cooled in an ice bath were slowly mixed with 20 mmol (4.8 g) of 3-methyl-3-butene-1-yl-tosylate introduced via a septum with a syringe. After 20 min, the ice bath was withdrawn, and the reaction was left under agitation at room temperature for 24 h. The reaction was analyzed by HPAEC (see below), evaporated, and diluted into 50 ml of a mixture composed of a solution (98 % volume) of ammonium hydrogenocarbonate (25 mM) and 2-propanol (2 volume %). The resulting mixture was passed over a cation exchange DOWEX 50WX8 ($NH_4^+$, 750 meq) column formerly equilibrated with 200 ml of the solution (98 % volume) of ammonium hydrogenocarbonate (25 mM) and 2-propanol (2 volume %). The column was eluted with 250 ml of the same solution at a slow flow and collected in a flask kept in an ice bath. The collected liquid was lyophilized, and the resulting white powder was solubilized in 130 ml of ammonium hydrogenocarbonate (0.1 M) and completed by 320 ml of acetonitrile/2-propanol (v/v). After agitation, the white precipitate of inorganic pyro- and mono-phosphates was eliminated by centrifugation (2100 × *g*, 10 ˚C, 8 min). This procedure was repeated three times, the supernatant was collected and dried, and the resulting oil was diluted in 120 ml of water. Remainders of unreacted tosylates were extracted three times by chloroform/methanol (7:3 (v/v)) in a separatory funnel, and the water phase was finally lyophilized. The resulting white powder was again washed twice by acetonitrile/chloroform/methanol (50:35:15 (v/v)) and dried under gentle $N_2$ flow. 11.25 mmol of pure 3-methyl-3-butene-1-yl-pyrophosphate triammonium salt were obtained by this procedure (75% yield) and were then dissolved in 200 ml of water for oxidation. For 6 mmol of 3-methyl-3-butene-1-yl-pyrophosphate, an aqueous solution of $Br_2$ (0.1 M) kept at 4 ˚C was added dropwise until appearance of a persistent yellowish color, yielding after evaporation 5.8 mmol (2.3 g) of an acidic solution (pH 2.1) of BrHPP, which was immediately neutralized by passing over DOWEX 50WX8-200 ($NH_4^+$, 48 meq). The ammonium salt of BrHPP obtained after lyophilization was dissolved in water and separated from bromides by passing through Dionex OnGuard-Ag (2 meq/unit) cartridges and an on-line column of (100 meq, 21 g) DOWEX 50WX8-200 ($Na^+$) eluted by milli-Q water. Colorless stock solutions of BrHPP ($Na^+$) were filtered over Acrodisc 25 membranes of 0.2 μM and kept as aliquots at -20 ˚C.

*HPLC*- Final purification of BrHPP was achieved by HPLC (Spectra system P1000 XR device) on an analytic Symmetry 5 μ C18 column (Waters) eluted at 1 ml/min and 20 ˚C with the ternary gradient indicated below. Upstream of detectors, a split of eluent distributes 190 μl/min in the online MS detector (see below), and the remaining 810 μl/min was sent to the Waters 996 photodiode array detector. Single wavelength detection at λ = 226 nm was of 7 milliabsorbance units for 6 μg of BrHPP injected in 25 μl (Rheodyne injector). The gradient program was as follows: solvent A, acetonitrile; solvent B, 50 mM ammonium acetate; solvent C, water; 0-7 min, 5% B in C; 7.1-11 min, 100% C; 12-15 min, 100% A; 15-17 min, 100% C.

## EXAMPLE 2

### Synthesis of HDMAPP using the method of Hecht et al (2002)

#### (E)-4-Chloro-2-methylbut-2-en-1-ol

TiCl4 (285mg, 1.5mmol, 164.5 μL) is dissolved in 3 mL of dry CH2CL2 under N2. The solution is cooled to -80 to -90C, and a solution of 84 mg of commercially available 2-methyl-2-cinyloxirane (98.2 μL, 1 mmol) in 0.4 mL of CH2CL2 is added in dropes with stirring. After 90 min. the reaction mixture is quenched by adding 5 mL of IN HCl. After

warming to room temperature, the phases are separated and the aqueous layer is extracted four times with 20 mL of diethyl ether. The combined organic phases are dried over $MgSO_4$. Evaporation of the solvent and purification by flash chromatography (pentanes/diethyl ether 1:1 v/v) affords 93 mg of pure product.

**(E)-1-Hydroxy-2-methylbut-2-enyl 4-diphosphate from (E)-4-Chloro-2-methylbut-2-en-1-ol**

A solution containing 227 mg (0.25 mmol) of tris (tetra-n-butylammonium) hydrogen pyrophosphate in 300 μL of MeCN is added slowly at room temperature to a solution of (E)-4-Chloro-2-methylbut-2-en-1-ol (25 mg, 0.21 mmol) in 250 μL of MeCN affording an orange-red solution. After 2 h, the solvent is removed under reduced pressure. The orange-colored oil is dissolved in 3 mL of $H_2O$, and the solution is passed through a column of DOWEX 50 WX8 (1x4 cm, $NH_4^+$ form) that has been equilibrated with 20 mL of 25 mM $NH_4HCO_3$. The column is developed with 20 mL of 25 mM $NH_4HCO_3$. Fractions are combined and lyophilized to yield 0.19 mmol of pure product (90%).

**EXAMPLE 3**

**BrHPP non-GLP studies**

*3.1. Material and methods*

3.1.1. Animals

- Group 1 : 5 purpose bred healthy male cynomolgus monkeys (M. fascicularis), supplied by C.R.P. Le Vallon, Femey S.E., Mahebourg, Mauritius. At the beginning of the study, body weights range from 3.7 to 4.6 kg.
- Group 2 : 10 purpose bred healthy cynomolgus monkeys (5 males and 5 females), supplied by C.R.P. Le Vallon. At the beginning of the study, body weights range from 1.8 to 3.5 kg and ages from 2 to 3 years.

Husbandry conditions conformed to the European requirements, comprising monitored temperature, humidity, air change and lighting cycle. Group 1 animals were housed in Biomatech (Chasse sur Rhône, France), and group 2 animals were housed in MDS, (Les Oncins, France).
All experiments were subjected to local ethical committee before processing.

3.1.2. Phosphoantigens

The synthesis and characterization of trisodium (R,S)-3-(bromomethyl)-3-butanol-1-yl-diphosphate BrHPP has been described previously (see above, as carried out in Espinosa, 2001). The lot used for the experiments described here was manufactured and characterized under GMP conditions by PCAS-SELOC (Limay, France). Sterilization and clinical unit preparation was conducted under GLP by AXCELL BIOTECHNOLOGIES (Saint-Genis l'Argentière, France). Titration of BrHPP in sterile aqueous solution was achieved by High Performance Anion-Exchange Chromatography with conductimetric detection (DIONEX DX600 system).

3.1.3. Drug administration and blood sampling

- Group 1 : animals were anaesthetised with intra-muscular injection of 6mg/kg ZoletilND 100 (Tiletamine-Zolazepam, Virbac, Carros, France) before any injection or blood taking.
- Group 2 : injections and blood taking were performed on manually restrained non-anaesthetised animals.
- 4-day BrHPP alone treatment (group 1) : 2 animals received 1mg/kg of BrHPP in 10ml saline on day 0 (microflex infusion set introduced into the external saphenous vein) and then 4mg/kg, 16mg/kg and 32mg/kg in 20ml saline on days 1, 2 and 3 by the same way. (Duration of infusion : 10 to 15 min).
- BrHPP/IL2 co-treatments (group 1) : 5 animals received either 20 mg/kg once or 4 mg/kg (16.7 mg/kg or 3.3 mg/kg of BrHPP anionic form) 5 times daily of BrHPP in final 50 ml saline by the same way as above (duration of infusion : 30 min). IL2 (18 million UI per vial, Proleukin®, Chiron, US) was resuspended in 1ml sterile water and diluted to 10ml with 4% HSA for a final concentration of 1.8 million UI/ml. In a first cycle of treatment, all animals received the same dose of IL2 consisting of 5 days of twice daily injections of 0.9 million units IL2. In a second cycle of treatment, animals received subcutaneously the following IL2 treatment : 0.15 million units twice daily for 9 days (animal Z059),

0.3 million units twice daily for 5 days (Z135), 0.9 million units twice daily for 5 days (animal Z714) or 9 days (animal X973).

A single animal received 80 mg/kg (66.6 mg/kg anionic form) BrHPP + 9 days of IL2 co-treatment consisting of a single daily subcutaneous injection of 0.6 million units.

- BrHPP/IL2 co-treatments (group 2) : BrHPP was diluted in saline to the appropriate final concentration (depending on the dose to inject and on the last recorded body weight) so as to inject always approximately 50ml in 30min (microflex infusion set introduced into cephalic or external saphenous vein). All animals received 0.6 million IU IL2 per day for 7 days. IL2 was administered subcutaneously as 2 separate injections of 0.3 million IU IL2 in sterile water, 8-hour apart. Control animals received IL2 only.

Twice weekly blood samples (1 to 4 ml) were withdrawn from femoral vessels/artery into EDTA containing tubes. Tubes were shipped overnight at room temperature (RT) before flow cytometry analyses.

3.1.4. Flow cytometry

Peripheral γδ lymphocytes were analysed twice weekly by flow cytometry on total monkey blood, after double staining with anti-CD3-PE antibody and anti-Vgamma9-FITC antibodies and/or anti Vd2 antibodies (CD3-PE : SP34 clone, BD Biosciences Pharmingen, Le Pont de Claix, France). Anti Vgamma 9, clone 7B6 is a monoclonal raised to human Vgamma 9 but that cross-reacts with cynomolgus cells. It was purified by affinity chromatography on protein A and coupled to FITC as previously described. We checked that this antibody stained most of Vd2 positive cells, stained by commercial (Endogen, Woburn, MA) TCR2732 clone (as previously described for other Vg9 antibodies in Rhesus monkey by Shen, 2002, data not shown).

Briefly, 50μl monkey blood is incubated 15 min at RT with 5μl anti-CD3-PE and 6μl anti-delta2-FITC or 10μl anti-gamma9-FITC antibodies. Antibodies are washed with 3ml 1X PBS, centrifuged for 4 min at 1300rpm at RT and supernatant is discarded. Red cells are lysed with the OptiLyse C reagent (Immunotech-Beckman-Coulter, Marseilles, France) according to the manufacturer's instructions. At the final step, stained white blood cells are recovered by centrifugation and resuspended in 300μl PBS + 0.2% PFA. Immediately before analysis, 50μl calibrated Flow CountTM Fluorospheres (Immunotech-Beckman-Coulter, Marseilles, France) are added to the cells for absolute number counting of the populations of interest.

For group 2, lymphocyte subsets were also analysed in parallel by dual color flow cytometry with CD20-FITC (2H7 clone); CD3-PE (SP34 clone); CD4-FITC (M-T477 clone); CD8-FITC (SK1 clone) (all purchased from BD Biosciences, Le Pont de Claix, France).

Flow cytometry was performed on a Epics XL-MCL apparatus (Beckman-Coulter, Roissy, France) with the Expo32 software.

3.1.5. Cytokine detection

Serum cytokines (TNFa and INFg) were detected and quantified with the BIOSOURCE CytoscreenTM ELISA monkey TNFa and CytoscreenTM ELISA monkey INFg respectively (purchased from CliniSciences, Montrouge, France) according to the manufacturer's instructions.

3.1.6. Haematology and serum clinical chemistry

Classical blood parameters follow-up (red blood cell, platelets total and differential white blood cells counts, haemoglobin, mean corpuscular haemoglobin, mean corpuscular haemoglobin concentration) were performed at the sites of monkey handling, just before and after each administration and twice weekly.

On all animals in group 2, 48 or 72 hours after each injection, 16 blood chemistry parameters were measured (sodium, potassium, chloride, calcium, inorganic phosphorus, glucose, urea, total cholesterol, total bilirubin, total protein, albumin, globulin, creatinin, alkaline phosphatase, aspartate aminotransferase and alkaline aminotransferase).

3.1.7. Vital parameters follow up

The animals were observed daily and during and after each injection for any change in vital and clinical parameters (general behaviour, skin, hair, respiratory system, central nervous system). Animals were regularly weighed, every 3 days (group 1) or weekly (group 2). Body temperature (on vigil animals of group 2) was measured before and at the end of each BrHPP/IL2 (or IL2 alone) infusion and once daily during the 5 days following administration. Heart

rate and blood pressure were recorded for all animals in group 2, before and at the end of each administration. All animals were observed at least twice daily for signs of morbidity/mortality.

3.1.8. Biopsy preparations

5 animals in group 2 were sacrificed 9 days after the second administration, by intravenous injection of sodium pentobarbitone, exsanguinated, and were submitted to full necropsy procedures for organ toxicology assessment of the drug. Samples of organs were weighed and collected in RPMI medium (Gibco-BRL - Life Science) for further processing. Lymphoid organ samples (thymus, tonsils, bone marrow, mesenteric, inguinal and tracheobronchial lymph nodes) were carefully mechanically dissociated with sterile syringe plungers, washed several times in RPMI medium and filtered twice through nylon membranes (Scrynel NYHC 100μm nylon, purchased from VWR International, France). Cells were then double stained with anti-CD3-PE and anti-gamma9-FITC antibodies and analysed by flow cytometry as described above.

*3.2. Results*

3.2.1. BrHPP alone does not induce reproducible expansion of g9d2 cells in vivo

We tested first if BrHPP injection alone was able to support activation of g9d2 positive T cells in vivo. In a first series of experiment, 4 monkeys were treated by five daily injection of 0.2 mg/kg (0.17 mg/kg anionic form) of BrHPP, and 4 animals were treated with saline. Injection of this dose of BrHPP did not result in any toxicity in the treated non human primates as assessed by vital signs or rectal temperature. Follow up of Vg9Vd2 T cells showed a slight increase in % of Vg9Vd2 in two treated animals and none in the placebo group but this expansion was not significant (data not shown).
To test if higher doses of the molecule could induce a more consistent activation or expansion, another two animals received four daily injections with increasing dosing of BrHPP (1mg/kg, 4 mg/kg, 16 mg/kg and 32 mg/kg, or the equivalent respective doses of 0.83mg/kg, 3.33 mg/kg, 13.33 mg/kg and 26.6 mg/kg anionic form BrHPP).
Again no toxicity nor fever was observed in the two treated animals. Follow up of Vg9Vd2 reavealed a decrease at day two post first injection, but the two animals returned to basal level at day 9 (figure 1). Survey of CD25 and CD69 did not give significant results, but these markers may not be optimal for study of non human primate cells.
At least two possibities can explain the lack of objective response of Vg9Vd2 cells upon BrHPP treatment i) as the BrHPP is a small molecule containing pyrophosphate, it may be excreted or degraded so rapidly that it does not allow sufficient contact with the target cells to allow activation of the cells ii) Expansion of g9d2 cells in vivo requires, as in vitro, the presence of cytokine, particularly IL2.
Although we do not have at present an analytical method sensitive enough to follow the molecule in vivo, it was likely that the serum concentration in vivo would be sufficient to trigger Vg9Vd2 cells. In vitro, we and others (Lang, 1995) have shown that the activation of Vg9Vd2 cells occurs within minutes after the addition of the molecule. Two mechanisms may prevent the molecule to be active in vivo i) the likely rapid renal clearance of such a small molecule and ii) a degradation process in vivo. The study of the half life of the molecule in primate sera showed that the half life of the molecule at 37°C is about one hour. The main mechanism of degradation of the molecule is the removal of the first phosphate, rendering the molecule biologically inactive. Nevertheless, the concentration of the molecule, injected by intravenous route in relatively high amount, should be maintained beyond the 20nM EC50 for a significant time, if no additional, blood cell or organ related degradation process occurs in vivo.
To try to reveal the activation of the cells, we tested the concomitant injection of low doses of rhIL2 with BrHPP.

3.2.2. BrHPP + IL2 induces reproducible, transient expansion of Vg9Vd2 cells in vivo in cynomolgus.

4 animals and one control (group 1 animals) were treated with the combination of BrHPP or saline with low doses of rhIL2. This group of animals was composed of the two previous animals that did not show any augmentation of Vg9Vd2 upon injection of BrHPP alone and three new animals. Among the four BrHPP treated animals 2 animals underwent 5 daily injection of 4mg/kg of BrHPP, and two animals underwent a single injection of 20 mg/kg (16.7 mg/kg anionic form). The BrHPP treated animals and control were administered the same rhIL2 regimen consisting of 0.9 million units twice daily by subcutaneous injection for 5 days. The control animal was treated first with saline and IL2, and then 14 days later with a single shot of BrHPP and a second course of 5 days IL2.
Haematological follow up of the animals revealed increase of lymphocyte counts by 2 to 4 in the BrHPP treated animals and control as well, consistent with the IL2 administration.
A specific expansion both in percent increase among CD3 positive cells and absolute numbers of Vg9Vd2 was observed in all BrHPP treated animals (fig2). This increase was transient, as the expansion is seen at day 7 with already a slight

increase at day 3, and levels being back to around before treatment level at day 10. The control animal treated with IL2 alone, showed a slight increase in absolute numbers but no increase in percentage, suggesting that peripheral Vg9Vd2, as other lymphocyte subset, slightly increase upon IL2 administration. Administration of BrHPP on the control animal, after this cycle of IL2 alone, results in an expansion consistent with that of treated animals, therefore validating this animal as a control.
Absolute increase of Vg9Vd2 was higher in the two animals receiving a single dose of BRHPP (x30 and x40) as compared to animals receiving the same dose but split in 5 daily injections (x8 and x18) (figure 1c). This may be due either to the deleterious effect of multiple injection or lower individual dose injected. As single injection was at least as efficient and more practical, next experiments were performed with single injection of the product.

3.2.3. Dose range effect of BrHPP in cynomolgus monkeys

To evaluate the dose range effect of the molecule, a new experiment was set on 10 new animals (group 2 animals). Subgroups of two animals, one male and one female, were treated with increasing doses of BrHPP (0, 0.2, 4, 20, 80 mg/kg), and co-treated with IL2 for 7 days.
Again, animals treated with IL2 alone did experience a slight increase of Vg9Vd2 cells, of the same order of magnitude as compared to other lymphocyte subset.
0.2 mg/kg dose was undistinguishable from the control animals, both in terms of % and absolute numbers of Vg9Vd2. A dose range effect was observed both in % and absolute numbers of Vg9Vd2 from 4 to 80 mg/kg (figure 3a) at day 7, without apparently reaching a plateau. Again % and absolute counts came rapidly back to pre-treatment level.
As a plateau was not reached during this treatment, same animals were treated a second time 21 days post first injection, after Vg9Vd2 came back to pre-treatment level, with doses ranging from 20 mg/kg, 80 mg/kg, 120 mg/kg and 160 mg /kg. To minimize potential effect of the first treatment on the second treatment, the new doses (120 and 160 mg/kg) were given to animals that seldom responded to the first treatment (ie animals treated with 0.2, and 4mg/kg during the first course of treatment, figure 3b).
The time line of the proliferation after this second injection was about the same as described before. It should be noted that at the highest concentration, the level of circulating Vg9Vd2 reached about 80 % of circulating CD3 positive cells (figure 3b), with absolute numbers reaching a mean of 10,000 Vg9Vd2 cells/mm3. The numbers and percentage of Vg9Vd2 declined after the peak between day 5 and day 7 , although the time to go back to pre-inj ection level appears slower for the two highest doses.
Pooled data from injection 1 and injection 2 gave a clear dose range effect both in terms of percentage and absolute numbers of Vg9Vd2 cells at the peak of response (figure 3c and 3d). This dose range effect is observed despite some individual heterogeneity, particularly in the 120mg/kg group, as demonstrated by the error bars, with a no effect dose situated between 0.2 and 4 mg/kg, still higher doses would have been necessary to establish a plateau.

3.2.4. Influence of IL2 schedule

5 animals from group 1 were submitted to injections of BrHPP followed by different doses and time schedule of IL2. As shown in figure 6, even doses as low as 0.15 million units twice daily are sufficient to support expansion of Vγ9Vδ2 T cells, apparently equivalent to higher doses (0.3 and 0.9 million units twice daily). The length of the Vγ9Vδ2 expansion does not seem to increase when IL2 treatment is given for more than five days, although more animals should be tested to clearly establish it. Interestingly, an animal subjected to 0.6 million units subcutaneous injection of IL2 as a single daily injection also developed an expansion of Vγ9Vδ2 cells (data not shown).

3.2.5. Phosphoantigen + IL2 induced expansion can be reproduced at least five times successively

A memory type response to a recall injection of phosphoantigen containing preparation (ie BCG) was recently suggested in a primate model. We tested if such a response could be obtained with a pure preparation of synthetic ligand, and IL2 cotreatment.
Animals from group 1 were treated a second time with a single shot of 20 mg/kg and 7 days IL2 and Vg9Vd2 increase was monitored by flow cytometry. Fold increase obtained with the two injections is represented in figure 4a. The timing of the amplification of Vg9Vd2 was the same as in the first injection. The two animals that were treated with a single shot of BrHPP during the first injection showed a slightly reduced amplification rate in the second injection. The two animals that were treated with 5 daily 4mg/kg of BrHPP injections showed a slightly higher amplification rate at the second injection, most probably reflecting the better effectiveness of single injections over fractionated injections as discussed before.
As mentioned before, animals of group 2 underwent two successive injections during the dose range assay. Interestingly, the two animals that were treated first with 80 mg/kg, and then with 20 mg/kg showed a decreased expansion, as

compared to all animals tested with a first injection of BrHPP at 20 mg/kg (fig 4b). Animals treated first with 20 mg and then with 80 mg showed expansions comparable to animals treated with 80 mg/kg at the first injection. Taken together, these results suggest that response of Vg9Vd2 to BrHPP + IL2 expansion can be repeated twice, although may be with lower expansion rate.

To get further insight into the possible influence of successive injections on the Vg9Vd2 response, 5 animals from group 2 (one animal per subgroup) were treated with a third cycle of BrHPP injection 21 days after the second injection and a fourth cycle after another 21 days.

Again the kinetic of Vγ9Vδ2 expansion was the same as described previously, with a peak between day 5 and day 9. As shown in figure 3c, one animal gave a high increase at the third injection (x 28). Interestingly, this animal was the only one which did not come back to the basal level after the second injection of 120mg/kg (animal 2034, fig3B). The control animal which received only IL2 in the first two courses of treatment gave an increase of around 40, compatible with the values obtained in four animals treated with this dose in the first two treatment cycles (fig 4b). The three other animals gave amplification lower than 40, lower than amplification in animals treated with the same dose at injection 1 or 2. The five animals treated with a fourth injection of 80 mg/kg BrHPP and IL2 gave a detectable increase of Vg9Vd2 although lower than that obtained in the third injection (fig 4b).

In conclusion, it is shown that induction of proliferation of Vg9Vd2 by BrHPP + IL2 co-treatment can be reproduced several times. In these experiments, we could achieve expansion 4 successive times, with injections of BrHPP separated by 20 days or higher.

3.2.6. Short term production of cytokine by g9d2 T cells in vivo upon challenge with Phosphoantigens

Vg9Vd2 cells are known producers of TNFa and IFNg in vitro upon phosphoantigen challenge. In order to evaluate if these cells could be a source of these cytokines in vivo, sera of some animals was collected shortly after BrHPP injection.

Samples of sera were collected from group 2 animals during the first dose range assay (0 to 80 mg/kg) just before injection then 1 and 4 hours post injection, and assayed by ELISA specific for TNFa and IFNg.

Assessment of IFNγ did not give significant results in all treated animals.

TNFα was detectable in the sera of animals treated at the highest dose (80 mg/kg) one hour after injection of BrHPP (fig. 5a). The serum level of TNFa rapidly decreased as it was not further detectable 4 hours post BrHPP injection and remains undetectable during the increase of Vg9Vd2 in blood. This suggests that TNFa is rapidly produced from intracellular, pre-formed pool by Vg9Vd2, and then is seldom produced after the initial activation by the drug.

To test the capacity of cells to produce cytokines after expansion with the drug, two animals of group 2 were injected a fifth time at 80 mg/kg with BrHPP (without IL2) at day 7 post fourth 80 mg/kg injection, where the level of Vg9Vd2 was at the peak. As shown in figure 5B, both TNFa and IFNg was detectable in the sera of treated animals. Production of TNFa followed the same kinetic as compared to the first experiment, with a level becoming undetectable at 4 hours post injection. On the reverse, IFNg became detectable at one hour post injection, and increased at 4 hours post injection, suggesting a slower, but more sustained production of this cytokine.

3.2.7. Absence of toxicity of injection of BrHPP alone or in association with IL2

No alteration of clinical or blood chemical parameters (see material and methods) were seen in any of the animals treated either with BrHPP alone or in association with IL2. From an haematologic point of view, in all animals treated with IL2, a transient increase (2 to 5 times) in lymphocytes was observed. In animals treated with the highest dose of BrHPP, a lymphocytosis was observed, corresponding to the peak of Vg9Vd2 in the periphery.

It should be noted that rectal temperature was not significantly affected in any animal treated, despite the presence of detectable level of TNFα and IFNγ inflammatory cytokines in sera of some treated animals.

**EXAMPLE 4**

**Phosphostim (BrHPP) I.V. Pharmaco-Dynamic Study in Cynomolgus Monkeys**

The objective of this GLP study was to explore further the pharmaco-dynamic properties of Phosphostim (BrHPP, 200mg; GMP batch) alone and in combination with IL-2 following several i.v. administrations to cynomolgus monkeys. In particular, this study was planned to evaluate :

- A dose-effect relationship between Phosphostim injection and γδ T cell peripheral amplification;
- The pharmaco-dynamic effect of a second, third and up to fifth administration of Phosphostim in male and female animals;

- The functionality of *in vivo* induced γδ T cells, by dosing systemic cytokines after Phosphostim treatment;
- The effect of treatment with IL-2 alone and to validate the selected dose of 0.6 million IU/day/animal.

Phosphostim was administered i.v., as slow infusions (50 ml in 30 minutes), at various dose levels to five groups of two animals (one male + one female). Males received two successive injections of BrHPP (Day 0 and Day 22) before sacrifice and females four injections (Days 0, 22, 52 and 84). Two females, selected upon their level of response to the $4^{th}$ treatment, received a supplementary injection of BrHPP alone at Day 91, during γδ cell peripheral increase, for systemic cytokine dosages.

The administration schedule of the test product can be summarized as follows in Table 1:

Table 1

| Group # | BrHPP Dose Levels (mg/kg)*** | | | | |
|---|---|---|---|---|---|
| | Day 0 $1^{st}$ injection | Day 22 $2^{nd}$ injection | Day 52 $3^{rd}$ injection | Day 84 $4^{th}$ injection | Day 91 $5^{th}$ injection¥ |
| 1 | 0* | 0* | 80** | 80** | 80*** |
| 2 | 0.2 | 160 | 80** | 80** | 80*** |
| 3 | 4 | 120 | 80** | 80** | - |
| 4 | 20 | 80 | 80** | 80** | - |
| 5 | 80 | 50 | 80** | 80** | - |

* administration of Ringer lactate physiological solution alone as a control
** females only
*** selected females, upon their level of response to the $4^{th}$ injection
¥ without IL-2 co-treatment

The dose levels in Table 1 are can be converted to BrHPP pure (anionic) form equivalent by multiplying each dose by 0.6. Thus, the doses of 0.2, 4, 20, 50, 80, 120 and 160 mg/kg in Table 1 are equivalent respectively to 0.12, 2.4, 12, 30, 48, 72 and 96 mg/kg of anionic form BrHPP.

IL-2 was administered s.c. at the following frequency: from Day 0 to Day 6 and from Day 22 to Day 28 for all animals; from Day 52 to 58 and from Day 84 to Day 90 for all the females. IL-2 was administered as two separate s.c. injections approximately 8 hours apart of 0.3 million IU, corresponding to 0.6 million IU/day/animal.

*Results*

The first and second administrations of Phosphostim and IL-2 resulted in a clear dose-related elevation of peripheral Vγ9Vδ2 T cells at Day 7, which is represented in Figure 7.

The first slightly efficient tested dose was 4 mg/kg (2.4 mg/kg anionic form BrHPP), 20 mg/kg (12 mg/kg anionic form BrHPP) inducing 20-fold γδ cell number increase. The estimated EC50 value of BrHPP *in vivo* was around 120 mg/kg (72 mg/kg anionic form BrHPP) and induced a 100-fold increase in circulating γδ cell count. At the highest tested dose, circulating Vγ9Vδ2 T cells were found 200-fold more numerous than before treatment and represented the majority of peripheral lymphocytes.

This study confirmed that IL-2 alone induced no specific amplification of γδ T cells *in vivo.*

The effects of Phosphostim treatment on the production of cytokines (INFγ and TNFα) production in the serum was studied twice:

- after the first infusion, in all treated animals: a significant production of systemic TNFα (serum concentrations around 60 and 120 pg/ml) was evidenced in both animals having received 80 mg/kg, 1 hour after BrHPP injection;
- in two females (F2032 & F2034), which received 80 mg/kg (48 mg/kg anionic form BrHPP) (without IL-2) during the peak (Day 7) of the $4^{th}$ injection. Serum TNFα and INFγ concentration evolutions for both animals are shown on the following curves (see Figure 8).

Thus, Vγ9Vδ2 T cells amplified *in vivo* upon BrHPP/IL-2 co-treatment also produce detectable amounts of systemic cytokines.

**EXAMPLE 5**

**Supplementary Pharmaco-Dynamics Studies in the Primate**

We have thus performed other non-GLP pharmaco-dynamics studies in male cynomolgus monkeys in order to further document the features of *in vivo* γδ T cells response to consecutive injections of BrHPP, varying either the dose or the time-laps between injections. We have demonstrated that, in males:

- at least 3 successive injections, 8 weeks apart, of either 20 or 80 mg/kg (12 or 48 mg/kg anionic form equilvalent) BrHPP induce significant increases of γδ cell rate and absolute count in the periphery;
- at least 4 successive injections, 4 weeks apart, of 20mg/kg (12 mg/kg anionic form equilvalent) BrHPP also induce detectable increases of γδ cell rate and absolute count.

Thus we confirm that under some circumstances, at least four successive peripheral amplifications of γδ T cells induced by BrHPP/IL-2 co-treatment can be obtained, indifferently in male and female cynomolgus monkeys.

**EXAMPLE 6**

**Two-Phase Repeated Dose Toxicity Studies in Cynomolgus Monkeys Following I.V. Administration of Phosphostim and BrHPP**

The objective of the first phase of this non GLP study was to determine BrHPP Maximum Tolerated Dose (MTD) in a group of two cynomolgus monkeys (one male, one female) by escalating doses. The second objective of this study was to characterize the toxicity of daily i.v. administration of Phosphostim for two weeks in two cynomolgus monkeys (one male, one female) at the MTD determined during the first phase of the study. The animals were treated at increasing dose-levels every 3 or 4 days (phase 1) and then at the estimated Maximum Tolerated Dose (MTD) daily for 2 weeks (phase 2).

A total of four cynomolgus monkeys (two males and two females) were divided into 2 groups and treated with BrHPP, by intravenous administration over a 1-hour infusion period as follows:

- Phase 1 (escalating dose):

  Group 1 (one male and one female) were administered BrHPP and/or Phosphostim (BrHPP, 200mg) i.v. at increasing dose-levels (160, 400, 600, 900 and 1200 mg/kg) every 3 or 4 days.
  Anionic form BrHPP equivalent to these doses are respectively: 96, 240, 360, 540 and 720 mg/kg.
  BrHPP was given as a solution after reconstitution in water for injection finally diluted in Ringer lactate under a dose volume of 4, 10 or 15 mL/kg.

- Phase 2 (fixed dose):

  Group 2 (one male and one female) were administered BrHPP i.v. daily at 900 mg/kg/day (540 mg/kg/day in anionic form BrHPP equivalent) for 2 weeks.

The animals were checked daily for mortality and clinical signs. Body weight was recorded pre-study and on the day of each administration in phase 1 or twice weekly throughout phase 2, and including on the day of necropsy for phase 2. Food consumption was estimated daily, starting at least 7 days pre-phase. Hematological, blood biochemical and/or peripheral blood lymphocyte subset analysis investigations were performed pre-study, on the day of each administration in phase 1, on day 7 in phase 2 and at the end of both phases. Electrocardiographic and blood pressure recordings were performed pre-study, on the day of each administration in phase 1 and on day 1 and at the end of treatment in phase 2. Blood, for determination of plasma levels of the test item, was sampled on days 1 and 14 in phase 2. On completion of each phase, animals were submitted to a complete macroscopic post-mortem examination and specified tissues preserved. In phase 2, selected body organs were weighed and microscopic examination of selected tissues was performed for all animals.

*Results*

No unscheduled death occurred and no relevant clinical signs were noted during the treatment phases. BrHPP treatment had no effect on the body weight evolution of animals in either phase and food consumption was unaffected.

BrHPP did not affect blood pressure in either phase. No treatment related hematological or blood chemistry changes were noted in phase 1. In Phase 2, increased lymphocyte count was noted in both animals, which may be attributed to the pharmacological activity of BrHPP.

A slightly lower absolute thymus weight was noted in the female.

No relevant findings were found either during Phase 1 or Phase 2 after macroscopic post-mortem examination. Lymphoid depletion was seen in the thymus and mesenteric lymph node of the female, whereas the opposite trend (increase of follicular germinal center in the mesenteric lymph node) was noted in the male in phase 2.

**EXAMPLE 7**

**Two Week Repeated Dose Toxicity Study in Rats Following I.V. Administration of Phosphostim**

The objective of this GLP study was to evaluate the potential toxicity of BrHPP following daily i.v. administration in rats for two weeks. 116 Sprague-Dawley rats were allocated into four groups: one control group (Group 1) with 10 males and 10 females and three treatment groups (Groups 2 to 4) with 10 males and 10 females. Group 2, 3 and 4 were administered respectively 80, 150 and 300 mg/kg/day (respectively 48, 90 and 180 mg/kg in anionic form equivalent) of BrHPP. Each treatment group also had a satellite group of 6 females and 6 males. Satellite animals were allocated for toxico-kinetics purposes.

BrHPP was administered daily by slow intravenous injection (0.4 mL/min) as a solution in sterile water for injection and Ringer lactate at the dose levels of 80, 150 or 300 mg/kg/day. The control group received the vehicle (together with 1% sodium to reach an osmolarity similar to the high-dose group dosage forms) under the same experimental conditions. A constant dosage-volume of 5 mL/kg was used.

The animals were checked daily for mortality and clinical signs. Body weight and food consumption were recorded twice a week. Ophthalmologic examination was performed before and at the end of the treatment period. Blood samples for determination of levels of BrHPP were taken from satellite animals on Day 1 and at the end of the treatment period. Vaginal lavage was performed in females during pre-treatment and at the end of the treatment period for monitoring of estrous cycle. Hematological, blood biochemical and urinalysis investigations were performed on all principal animals at the end of week 2. On completion of the study, all animals were killed and subjected to a macroscopic post mortem examination and specified organs were weighed and preserved. Microscopic examination was performed on selected tissues from animals of the control and the 300 mg/kg/day groups.

*Results*

No unscheduled death occurred during the study. Vocalization during dosing was noted on many occasions in all animals given 150 and 300 mg/kg/day (respectively 90 and 180 mg/kg in anionic form equivalent). Dose-related higher mean body weight gain was noted in females treated with BrHPP.
Similar mean food consumption was noted between control and BrHPP-treated animals. No relevant ophthalmologic findings were noted in animals given 300 mg/kg/day (180 mg/kg in anionic form equivalent) at the end of the treatment period. No changes of toxicological significance were noted in any hematological, biochemical or urinary parameter. No relevant differences in organ weights from controls were noted in any BrHPP-treated group at the end of the treatment period. No relevant findings were noted in any BrHPP-treated group at the end of the treatment period from the macroscopic post-mortem examination. Upon microscopic examination, slight changes were observed in the tail vein of both control and BrHPP-treated animals, and were attributed to the mechanical injury due to daily intravenous administrations. No relevant findings were seen in other organs and tissues of animals given 300 mg/kg/day. Additional results regarding blood levels of BrHPP, monitoring of the estrous cycle and toxico-ldnetics are currently being analyzed.

*Conclusion*

BrHPP No Observed Adverse Effect Level (NOAEL) was considered to be 300 mg/kg/day (180 mg/kg in anionic form equivalent), the highest tested dose under these study experimental conditions.

**EXAMPLE 8**

**Analysis of BrHPP-amplified γδ T cells cytotoxicity towards autologous primary tumor cells of RCC patients**

The aim of the present study was

- to establish primary normal and tumor renal cells from RCC patients

- to investigate the effect of the BrHPP on PBMCs of these patients
- to investigate the lytic potential of expanded Vγ9Vδ2 T cells against primary normal and tumor renal cells in an autologous setting. This cytotoxic activity was compared with other autologous effectors cells, like LAK cells (Lymphokine-Activated Killer cells) for example.

### *8.1 Material and methods*

#### *Patients*

All mRCC patients (N=12) included in this study presented a renal clear cells carcinoma and underwent partial or total nephrectomy. None of the patients has received any previous treatment. Seven out of the 12 patients were not included after the initial phase of testing for the main part of the study. Informed consent was obtained from the mRCC patients.

*Expansion of peripheral blood gamma delta T cells*

Blood samples from 12 RCC patients (50 ml) were collected just before or no more than 2 months after the nephrectomy. PBMCs were isolated by centrifugation on Ficoll-Hypaque density gradient (Amersham Biosciences). PBMCs from one healthy volunteer was used as control.
Ten million PBMCs were cultured at $2\times10^6$/ml in 24 well plate in RPMI 1640 supplemented with 10% v/v fetal calf serum (Fetal Clone irradiated, Hyclone).
Polyclonal Vγ9Vδ2 T cell lines were specifically expanded in presence of 3 μM BrHPP molecule (batch INPA-0214) and 100IU/ml IL2 during 15 days.
Every three days, the volume corresponding to half culture medium was replaced by fresh medium containing only 200IU/ml IL2

*Obtention of LAKs cells*

Five millions of PBMCs from mRCC patients were cultured in 6-well plate in presence of 1000UI/ml IL-2 for three days. Their cytotoxic activity was assessed in a 4-hour $^{51}$Cr release assay in parallel with autologous expanded Vγ9Vδ2 T cells to compare the cytotoxic efficiency of both populations.

*Establishment and culture of tumoral/normal cell lines*

The autologous primary tumor cell lines were derived from the tumor fragments by enzymatic digestion using Collagenase (300U/ml), Deoxyribonuclase I (500U/ml), Hyaluronidase (3000U/ml) (Sigma Aldrich). The same protocol was applied to a renal normal fragment, taken at distance from the tumor, to derive short-term normal renal cells. These cells were cultured in Dulbecco modified Eagle medium supplemented with 10% FCS and Ultroser (Gibco BRL; Scotland).

*Cell type characterization*

PBMCs from patients are either frozen or used directly in culture as mentioned above.
Before in vitro culture, phenotype of $2x10^5$ PBMCs from RCC patients was analyzed using the following combinations of fluorescein isothiocyanate (FITC), phycoerythrin (PE) and phycoreythrin-cyanin 5 (PC5) conjugated antibodies to determine the NK, αβ and γδ T cells population (all purchased from Beckman-Coulter): CD3-PC5 /V82-FITC /IgG1-PE, CD8-PE, CD56-PE. At day 15 of the culture, a triple staining was performed on the expanded Vγ9Vδ2 T cells bulk. The following antibodies combinations were performed:

> CD3-PCS/Vδ2-FITC/ CD8-PE (IgG1), CD16-PE (IgG1) CD2-PE (IgG1), CD56-PE (IgG1), CD69-PE (IgG1), HLA-DR-PE (IgG1), CD45RO-PE (IgG1), NKG2D-PE (IgG1), NKG2A-PE (IgG2b), CD94-PE (IgG1), CD158a, b, e-PE (IgG1).

Background levels were measured using isotypic controls. Compensation was set up with single stained samples, low forward scatter elements were excluded from analysis and 10,000 events were collected and analyzed using the Cell Quest software (Becton Dickinson).
Primary normal and tumor renal cells were phenotyped by indirect single color fluorescence. Cells ($2x10^5$) were incubated for 30 minutes at 4°C with following antibodies:

anti-HLA-A2, anti-HLA-BC (clone B1.23.2), anti-HLA-ABC (clone W6/32), CD54, anti-MICA (clone BAM 195 provided by Prof. A. Moretta (Genova,Italy)), G250-FITC (provided by Dr. Hirsch F. Hôpital Paul Brousse, Paris), anti-human fibroblast (clone AS02 Dianova, Hamburg).
Cells were washed twice with phosphate-buffered saline (PBS) and then incubated for 20 minutes at 4°C with phycoerythrin (PE)-conjugated goat anti-mouse Ig.

*Assay for cytolytic activity*

Expanded γδ effector T cells were tested for cytotoxicity against autologous normal and tumor target cell lines, control sensitive target cell line Daudi and control resistant target cell line Raji in 4h $^{51}Cr$ release assay. LAK effector T cells for the same patient were included in the assay to compare their lytic potential against the one of γδ T cells.

- Target cells were used in amounts of $2x10^3$ cells/well and labeled with 100μCi $^{51}Cr$ for 60 minutes. Effector/Target (E/T) ratio ranged from 30: 1 to 3.75: 1.
- Specific lysis (expressed as percentage) was calculated using the standard formula ((experimental-spontaneous release / total-spontaneous release) x100). Data are the mean of triplicate wells.

*8.2 Results*

*Selective expansion of TCR Vδ2-bearing T cells in PBMCs of patients with RCC*

First, the ability of BrHPP to expand resting γδ T cells from peripheral blood of mRCC patients was compared to γδ T cells expansion of one healthy volunteer. Seven patients could not be included in the study since no blood has been collected (BAZ, DEN,GOU and SAN) or since the pathology is not a clear cell (or conventional cell) renal carcinoma (COU, FAB and ROU).
For the evaluable patients, PBMCs were stimulated as described in materials and methods. From twelve patients analyzed, seven of them (7/12 or 58%), namely BEL, FOUR, MOR, POU, QUI, VAG and ZEN presented a Vγ9Vδ2 T cells amplification. These mRCC patients had from 1,6% to 4.2% γδ T cells at baseline that expanded from 73.3% to 94.8% after stimulation with BrHPP (n=7).
Five patients, namely CHA, CHAR, PAS, SAU and SCH were not included in the cytotoxicity assay since it was impossible to expand specifically their Vγ9Vδ2 population *in vitro.* These "BrHPP non responder patients" had from 0.6% to 3.8% γδ T cells at baseline that expanded from 4.7% to 19.6% after stimulation (n=5).
When blood sample before nephrectomy was available, the experiment was performed with this sample. It has been checked that same amplification results could be obtained with a blood sample collected after nephrectomy (data not shown).

*Cell type characterization of "BrHPP responder patients*"

*Effector cells*

Phenotypic analysis of expanded Vγ9Vδ2 T cells from mRCC patients that respond to BrHPP was carried out. Data from patients QUI and FOUR are not shown for reasons detailed below. Most of these cells had the phenotype of Ag experienced cells (CD45RO+), expressed adhesion molecules like LFA-1 or CD2 and expressed the costimulatory molecule NKG2D. Some γδ T cells maintained an activated phenotype as confirmed by the expression of CD69 and HLA-DR. Different subpopulations expressing either CD8, CD56 or CD16 could be also identified. Whereas the inhibitory heterodimer complex CD94/NKG2A was expressed by almost half of γδ T cells, receptors belonging to the Killer Ig-like Receptor family (CD158a; b; e) were expressed on very restricted subsets.

*Target cells*

Phenotypic analysis of the tumor and normal renal cells from responder patients was performed after short term *in vitro* culture. Patient QUI was excluded since no normal renal cells have been obtained. All the six other patients expressed high level of MHC class I molecule on both normal and tumoral cells and were positive for the adhesion molecule CD54. As expected, G250 was specifically expressed on tumoral cells but at various levels. The marker AS02 indicated the level of fibroblastic contamination in the culture. That ranged from 4,7% to 58,3% for the normal cells and from 6,7% to 53,2% for the tumoral counterparts for the patients BEL, MOR, POU,VAG and ZEN. It is important to note that, except for ZEN, the fibroblast (which is a cell type resistant to γδ T cell lysis) contamination level is higher in the tumoral culture that in the corresponding normal cell culture. Patient FOUR showed a large contamination, since in the

tumoral culture only the fibroblasts were growing (98,5% of AS02 positive cells). Patient FOUR was then also excluded from the main part of the study. MICA expression level, read out by BAM195 staining, is rather low on all the cells tested, except for BEL normal renal cells.

*In vitro cytotoxicity of expanded Vγ9Vδ2 T cells from mRCC patients*

Lytic activities of amplified γδ T cells were measured against classical control targets (Raji and Daudi), primary autologous normal and tumor cell lines of the selected patients in a 4h standard $^{51}Cr$ release assay for the five patients BEL, MOR, POU,VAG and ZEN. Individual data and the mean of these five patients results are shown in Figures 9A and 9B.

- Vγ9Vδ2 T cells expanded by BrHPP exhibited 36.7 +/- 5.4 % (range 29.1 to 43.1 %) of specific lysis against primary tumor cell lines for the 30: 1 ratio, whereas the primary normal cell lines were lysed at 12.5 +/- 5.0% (range 7.0 to 18.9%) at the same ratio. These results are the mean of the five patients tested.
- The lysis level of tumoral cells by autologous effector cells is significantly higher compared to the normal control. In a bidirectional paired student's t test, the p value is: p = 0.0002 at 30:1 E/T ratio, p = 0.01 at 15:1 E/T ratio and p = 0.04 at 7.5:1 E/T ratio.

In all cultures analyzed, γδ effectors T cells presented a strong lytic activity against control sensitive target cell line Daudi (68.3 +/- 14.2%) and very low activity against control negative target cell line Raji (12.9 +/- 5.0%).
For patient ZEN, lysis by autologous LAK cells was investigated. As expected LAK cells showed a lytic activity against Daudi and Raji cell lines but also the normal and tumoral cells with compared efficiency. These results have to be confirmed with other patients but they underline the lack of specificity of LAK cells mediated lysis.

*8.3 Discussion*

In this study, we have shown that peripheral Vγ9Vδ2 T cells from RCC patients can be expanded *in vitro* by BrHPP stimulation in 58% of the cases (7 patients out of 12). These cells displayed a selective lysis against autologous renal tumor cells and not against renal normal cells as read out by cytotoxic assay.
However, this lytic activity of expanded γδ T cells toward primary tumor cells may have been minimized due to a variable level of contamination by fibroblasts in these cultures: the percentages of fibroblasts ranged from 4.7% to 58.3% for normal cells and ranged from 6.7% to 53.2% for tumor cells. This contamination of primary cell culture modified the E/T ratio of the cytotoxic assay.
In order to realize this test in better conditions, we proposed to sort the fibroblasts with the specific antibody (clone AS02, Dianova) and then to culture the purified renal tumor cells.
We have obtained confirmation that expanded γδ T cells did not lyse fibroblasts. The culture of tumor cells from one patient of this study (patient FOUR) presented 98.5% of contamination by fibroblasts. The cytotoxic assay with the expanded autologous Vγ9Vδ2 T cells showed that these cells were lysed at 4.0% at the 30:1 ratio whereas Daudi were lysed at 75.4% at the same ratio.
To conclude, this study showed that for all the evaluable patients the primary tumor cells but not normal cells could be specifically lysed by autologous *in vitro* BrHPP stimulated γδ T cells.

**Example 9**

**In vitro and in vivo dosage response comparison of BrHPP and HDMAPP compounds**

***9.1 Materials and methods***

*9.1.1 In vivo*

*Animals*

Eight purpose bred healthy male cynomolgus monkeys (*M. fascicularis*). At the beginning of the study, body weights ranged from 3.5 to 4 kg and ages from 37 to 41 months.
Husbandry conditions conformed to the European requirements, comprising monitored temperature, humidity, air change and lighting cycle. Animals were housed individually in stainless steel cages. Food was provided *ad libitum* and composed of expanded complete primate diet (U.A.R., Villemoisson, Epinay/Orge, France) supplemented daily with fresh fruits.
Animals were anaesthetised with intra-muscular injection of 6mg/kg Zoletil™ 100 (Tiletamine-Zolazepam, Virbac, Carros,

France) before any perfusion.

*HDMAPP/IL2*

HDMAPP : initially at 21.5mM.
HDMAPP was produced according to the methods described herein. It was sterilized by filtration on 0.22μM micro-filters. HDMAPP solutions in water are stored frozen.
IL2: Proleukin® from Chiron (Emeryville, USA), at 18M UI per vial, stored at -20°C, reconstituted with 1ml sterile water for injection. This starting solution at 18M IU/ml is diluted qsp 10ml water, and doses of 300μl (0.6M IU) are injected daily. Each diluted solution batch is stored at 4°C for up to 3 days.

*Injections/Blood samplings*

HDMAPP was administered i.v. to male cynomolgus monkeys by 30-minute perfusions in a total volume of 50 ml with Ringer Lactate as vehicle.
Injected HDMAPP doses : 2.5mg/kg, 0.5mg/kg, 0.1mg/kg and 0.02mg/kg.
The animals were co-treated subcutaneously with 0.6M IU IL2 in sterile water per day for 5 days. Blood was drawn pre-dose and at day 4, 5, 7, 11 and 14 after HDMAPP perfusion for flow cytometry analysis of blood cellular populations of interest.
Blood samples (1 to 4 ml) were withdrawn from femoral vessels/artery into heparin-lithium containing tubes. Tubes were shipped overnight at room temperature (RT) before flow cytometry analyses.

*Flow cytometry*

Blood samples (1 to 4ml) were withdrawn from femoral vessels/artery into heparin-lithium containing tubes. Tubes were shipped overnight at room temperature (RT) before flow cytometry analyses.
Peripheral γδ lymphocytes were analysed by flow cytometry on total monkey blood, after triple staining with anti-Vgamma9FITC, anti-CD3PE and anti-CD69PC5 antibodies (Vgamma9-FITC : 7B6 clone, produced, purified and FITC-coupled at Innate Pharma; CD3-PE : SP34 clone, BD Biosciences Pharmingen, Le Pont de Claix, France; CD69PC5 : FN50 clone, Immunotech-Beckman-Coulter, Marseilles, France).
Briefly, 50μl monkey blood was incubated 15 min at RT with 10/μl anti-gamma9-FITC, 5μl anti-CD3-PE and 5μl anti-CD69PC5 antibodies. Antibodies were washed with 3ml 1X PBS, centrifuged for 4 min at 1300rpm at RT and supernatant was discarded. Red cells were lysed with the OptiLyse C reagent (Immunotech-Beckman-Coulter, Marseilles, France) according to the manufacturer's instructions. At the final step, stained white blood cells were recovered by centrifugation and resuspended in 300μl 1X PBS + 0.2% PFA. Immediately before analysis, 50μl calibrated Flow Count™ Fluorospheres (Immunotech-Beckman-Coulter, Marseilles, France) were added to the cells for absolute number counting of the populations of interest.
Flow cytometry was performed on a Epics XL-MCL apparatus (Beckman-Coulter, Roissy, France) with the Expo32 software.

*9.1.2 In vitro*

In vitro proliferation assays and in vitro TNFα release assay are performed essentially as described in Example 3.

***9.2 Results***

*9.2.1 In vivo*

HDMAPP injections on the 8 test animals were carried out according to the following schedule in Table 2:

Table 2

| Animals\Dates | Day 20 of month 1 | Day 31 of month 2 | Day 18 of month 4 | Day 30 of month 5 |
|---|---|---|---|---|
| **AD235** | 2.5mg/kg | 2.5mg/kg | 2.5mg/kg | 2.5mg/kg* |
| **AD384** | | 0.5mg/kg | 0.5mg/kg | |

(continued)

| Animals\Dates | Day 20 of month 1 | Day 31 of month 2 | Day 18 of month 4 | Day 30 of month 5 |
|---|---|---|---|---|
| **AD101** | | 0.1mg/kg | 0.1mg/kg | |
| **AC903** | | 2.5mg/kg | 0.5mg/kg | |
| **AD270** | | 0.5mg/kg | 0.5mg/kg | |
| **AD299** | | 0.1mg/kg | 0.02mg/kg | |
| **AD602** | | | 0.02mg/kg | |
| **AD219** | | | 0.02mg/kg | |
| * The result of this injection was not used for the calculation of the dose-range effect (see below). | | | | |

The effect of successive i.v. injections of various HDMAPP doses to cynomolgus monkeys was monitored. Individual curves of percentages of peripheral γδ T cells and absolute blood count of the same cells was determined for each animal.

*In vivo* amplification was assessed after 4 successive injections, 5 weeks apart, of 2.6mg/kg HDMAPP. The 4 successive HDMAPP treatments led respectively to approx. 60, 55, 60 and 40% γδ cells among $CD3^+$ at day 5).

*In vivo* amplification was assessed after 4 successive injections of 20 or 80mg/kg Phosphostim®, 4 or 8 weeks apart. The 4 successive BrHPP treatments led respectively to repeated increases of γδ cells as described in previous examples herein.

The dose-range effect of HDMAPP and BrHPP in vivo as determined by determining numbers of γδ T cells by flow cytometry are shown in Figures 10 to 13. HDMAPP was tested in four doses as described in Table 2, and as shown, these data were obtained from various numbers of injections (2.5mg/kg : 3 injections; 0.5mg/kg : 5 injections; 0.1mg/kg : 3 injections; 0.02mg/kg: 3 injections). BrHPP was tested in seven doses: 0, 0.12, 2.4, 12, 48, 72 and 96 mg/kg. The figures present γδ T cells counted at days 5 and 7 for HDMAPP and day 7 for BRHPP. As discussed above, the peak of γδ T cell expansion is found to be at day 5 after injection. For each dose range, results are expressed in (a) fold γδ T cell increase in absolute cell count ($/mm^3$ blood), (b) absolute cell count ($/mm^3$ blood), (c) percentage γδ T cells of total circulating lymphocytes, and (d) fold increase in percentage of total circulating lymphocytes.
Figures 10A and 10B show the absolute cell count ($/mm^3$ blood) for HDMAPP and BrHPP respectively.
Figures 11A and 11B show the percentage γδ T cells of total circulating lymphocytes for HDMAPP and BrHPP respectively.
Figures 12A and 12B show the fold γδ T cell increase in absolute cell count ($/mm^3$ blood) for HDMAPP and BrHPP respectively.
Figures 13A and 13B show the fold increase in percentage of total circulating lymphocytes for HDMAPP and BrHPP respectively.

*9.2.1 Comparison of BrHPP and HDMAPP in vitro and in vivo bioactivity*

BrHPP and HDMAPP bioactivity were compared using in vitro and in vivo assays. The in vitro biological activity of γδ T cell amplification from human PBMCs (in the presence of rhIL2) was assessed using a TNFα release assay. In vivo activity was determined as described above and shown in figures 10 to 13. For purposes of comparison, the aminobisphosphonate compound Zoledronate®, the most potent aminobisphosphonate evaluated so far by the inventors, was included in the in vivo comparison.

The in vitro comparison is shown in figure 14. Figure 14 shows the in vitro EC50 for the compounds: for BrHPP the EC50 is about 30 nM while for HDMAPP the EC50 is about 0.6 nM, approximately a 50-fold difference in potency.

The in vivo comparison is shown in figure 15. Figure 15 shows the in vivo EC50 for the compounds: for BrHPP the EC50 is about 1 nM while for HDMAPP the EC50 is about 5 pM, approximately a 2-log difference in potency. By contrast, the less potent Zoledronate® showed an EC50 value of about 1 μM.

**EXAMPLE 10**

**In *vivo* efficacy of human γδ T cells Nod-Scid/Tumor Model**

The aim of this project based on the use of the Nod-SCID/tumor mice model, was to study the proliferation of human γδ T cells after an in vivo single stimulation of human MNC with BrHPP and to study the in vivo anti-tumoral efficacy of the developed γδ T cells.

*Materials and methods*

Preliminary studies to set up the Nod-SCID/tumor and to stimulate human MNC with BrHPP in the Nod-SCID were carried out as follows:

* The following renal tumor cells lines purchased from the ATCC (786-O, Kaci1, G401 and G402) were used for the establishment of the model. 786-0 and Kaci-1 cells lines were renal clear cell carcinoma (RCC), G401 rhabdoid and G402 leimyeloblastoma.
8-12 weeks Nod-SCID mice were used as recipient of the cells lines.
Each mouse received, at the left flank's upper part, 2-5 $x10^6$ cells by subcutaneous injection at day D0. The appearance and the growing of the tumors evolution were checked weekly. The tumors appearance and development depend on the cell line and on the number of engrafted cells. For example, after the engraftment of $2x10^6$ cells, 3 to 4 weeks were needed for the 786-O and Kaci-1, while 2 weeks were needed for the G401 and 402.
* At day 0, mice were injected IP $50*10^6$ PBMC (collected from healthy donor, établissement français du sang), then stimulated (IP) 40mg/kg BrHPP mixed with 1000 IU of IL2. At D5 and each every three days, mice were treated with 500 UI of IL2. Human γδ T cells were developed in the peritoneal cavity of Nod-SCID/human (hu) BrHPP treated mice.

*Anti-tumoral efficacy*

Taking advantage of the tumoral development in the Nod-SCID mice and the in vivo stimulation of the human γδ T cells, the anti-tumoral effect of the γδ T cells was studied in vivo. The tumoral model was established by engrafting $2x10^6$ of 786-0 cells lines, three weeks later, when the volume of the solid tumor reached more than > 30 mm3 (calculated with the formula $A^2$ X B/2 where A and B represent respectively the length and breadth of the tumor). Mice, randomized, received IP PBMC and treated for the stimulation with BrHPP. Several groups were constituted:

A- Negative control group: Mice in the group received only tumoral cell line
B- PBMC group: Mice were injected IP with $50X10^6$ PBMC
C- Study group: Mice after receiving the $50X10^6$ PBMC were treated with BrHPP and IL2.
For each group, parameters were observed as follows:

- Weekly check of the tumor size and volume
- Weekly phenotype of human T cells in the peritoneal cavity and blood collected cells for the percentage of human γδ T cells versus ab human T cells.
- Serological TNF and INF check
- At sacrifice:

  1- phenotype study of the homing of human leukocytes cells and their phenotype (IP, liver, lung, spleen, bone morrow, blood, thymus and tumor).
  2- Immunohistochemistry (IHC) study in the tumor of the recipient mice.

*Results*

- Tumor growth: Human IL2 treatment of the 786-O tumor did not induce any activity; no difference was observed between the tumor size in this group and the non treated mice group.
  Tumor growth in the first few days after BrHPP treatment is shown in Figure 15. While tumor size increased in the first few days after treatment, tumor size decreased therafter quickly in the PBMC/BrHPP and IL2 treated group. Figure 19 shows that from day 7 onwards the tumor size shrank. In the PBMC group, after short arrest, the size grows and no significant difference was observed between the tumors sizes in this group and those of the negative control.
- Phenotyping and homing of human cells: In the peritoneal cavity: the weekly check of the IP phenotype of treated mice showed a human γδ T cell presence only in the BrHPP treated mice. The relative numbers of γδ T cells in the blood is shown in Figure 16 and in the peritoneal cavity in Figure 17. Human γδ T cells represent a higher percentage of the human CD3 T cells. In the mice recipient organs: phenotyping is carried out at sacrifice (4 weeks after the PBMC and BrHPP treated or not treated groups); the major human cells present in those organs are human CD3+ T cells with 99% expression of the Ab TcR. However in the tumor, human γδ cells were present only in the BrHPP

treated group.

- IHC: 4 μm slides of the tumor collected at the sacrifice confirmed the γδ T cells presence only in the tumors from PBMC BrHPP treated mice. The frequency of the human γδ T cells inside the tumor looks to be correlated with the delay of the BrHPP treatment.

*Conclusion*

Data obtained here demonstrated the γδ efficacy in vivo. γδ T cells exercise their activity in the 2 weeks after the BrHPP stimulation. This effect is prolonged and stable, in the group of mice where the tumor was cleared, no reappearance of new tumor was observed 12 weeks after the treatment. In 4 of 10 mice, the tumor was not totally cleared; but even in this group, tumor growth was stopped and the tumor size stayed stable after 12 weeks. The TIL in the BrHPP-untreated mice consists of ab T cells; however we did not observe any tumoral activity in this group.

**EXAMPLE 11**

**Administration of BrHPP for treatment of advanced/metastatic solid tumors in humans**

Phosphostim is based on a new chemical entity, the drug substance Bromohydrin Pyrophosphate (BrHPP), which is a specific agonist of immune competent cells namely the Vγ9Vδ2 T cell subpopulation bearing anti-tumor activity. Phosphostim (BrHPP, 200 mg) is the intravenous formulation of BrHPP for cancer immunotherapy, which will be used in combination with low dose of IL-2 (s.c. 1 M IU/$m^2$/day) in a first clinical trial in patients with advanced/metastatic solid tumors.

**Clinical trial**

Phosphostim has never been previously administered to humans. The main objective of the planned phase I clinical trial with Phosphostim is to evaluate the safety and tolerance of a Phosphostim alone and in combination with a fixed dose of IL-2. This trial is a single arm, open-label, national, multi-center, dose-escalation trial in sequential cohorts of patients with advanced/metastatic solid tumors. A traditional dose escalation design (Fibronacci) will be used, with cohorts of 3-6 patients at each dose level. All patient cohorts will receive repeated cycles of treatment every 3 weeks. The first cycle will consist of one administration by infusion of Phosphostim alone and from the second cycle onwards, 1 million IU/$m^2$/day of IL-2 will be added (for a total duration of 7 days). Phosphostim starting dose will be 200 mg/$m^2$ (5 mg/kg) corresponding to 118 mg-equivalent of BrHPP anionic form. The pharmaco-kinetics and pharmaco-dynamics properties of Phosphostim alone and in combination with IL-2 will also be evaluated in this study.

Phosphostim (BrHPP, 200 mg) is a freeze-dried apyrogenic sterile white powder to be reconstituted in solution for infusion.

Each vial of Phosphostim (BrHPP, 200mg) contains 200 mg of BrHPP anionic form and 50mg the excipient alpha-lactose monohydrate (USP).

Phosphostim (BrHPP, 200 mg) has a shelf life of 6 months at -20˚C. Additional stability studies are currently on-going. Until these studies are completed, Phosphostim should be stored at -20˚C ±5˚C, protected from light.

Phosphostim is for immediate and single use following first opening and reconstitution. Phosphostim is reconstituted immediately prior to use with 2 ml of water for injections to make a 100 mg/ml solution. The needed quantities of reconstituted product are diluted in a total volume 100 ml of ringer lactate buffer infusion vehicle. The diluted solution is clear and colorless.

Phosphostim is administered intravenously over 1 hour.

REFERENCES

Alison, T.J., Winter, C. C., Fournie, J. J., Bonneville, M., and Garboczi, D. N. (2001). Structure of a human gammadelta T-cell antigen receptor. Nature 411, 820-824.

Bank, I., Book, M., Huszar, M., Baram, Y., Schnirer, I., and Brenner, H. (1993). V delta 2+ gamma delta T lymphocytes are cytotoxic to the MCF 7 breast carcinoma cell line and can be detected among the T cells that infiltrate breast tumors. Clin Immunol Immunopathol 67, 17-24.

Behr, C., Poupot, R., Peyrat, M. A., Poquet, Y., Constant, P., Dubois, P., Bonneville, M., and Fournie, J. J. (1996).

Plasmodium falciparum stimuli for human gammadelta T cells are related to phosphorylated antigens of mycobacteria. Infect Immun 64, 2892-2896.

Belmant, C., Espinosa, E., Halary, F., Apostolou, I., Sicard, H., Peyrat, M. A., Vercellone, A., Kourilsky, P., Gachelin, G., Poupot, R., et al. (1999). Conventional and non-conventional recognition of non-peptide antigens by T lymphocytes. C R Acad Sci III 322, 919-924.

Belmant, C., Espinosa, E., Halary, F., Tang, Y., Peyrat, M. A., Sicard, H., Kozikowski, A., Buelow, R., Poupot, R., Bonneville, M., and Fournie, J. J. (2000). A chemical basis for selective recognition of nonpeptide antigens by human delta T cells. Faseb J 14, 1669-1670.

Brondino et al, Journal of Fluorine Chemistry, 76, p 193-200 (1996).

Bukowski, J. F., Morita, C. T., Tanaka, Y., Bloom, B. R., Brenner, M. B., and Band, H. (1995). V gamma 2V delta 2 TCR-dependent recognition of non-peptide antigens and Daudi cells analyzed by TCR gene transfer. J Immunol 154, 998-1006.

Buzio, C., Andrulli, S., Santi, R., Pavone, L., Passalacqua, R., Potenzoni, D., Ferrozzi, F.,Giacosa, R., and Vaglio, A. (2001). Long-term immunotherapy with low-dose interleukin-2 and interferon-alpha in the treatment of patients with advanced renal cell carcinoma. Cancer 92, 2286-2296.

Choudhary, A., Davodeau, F., Moreau, A., Peyrat, M. A., Bonneville, M., and Jotereau, F. (1995). Selective lysis of autologous tumor cells by recurrent gamma delta tumor-infiltrating lymphocytes from renal carcinoma. J Immunol 154, 3932-3940.

Constant, P., Poquet, Y., Peyrat, M. A., Davodeau, F., Bonneville, M., and Fournie, J. J. (1995). The antituberculous Mycobacterium bovis BCG vaccine is an attenuated mycobacterial producer of phosphorylated nonpeptidic antigens for human gamma delta T cells. Infect Immun 63, 4628-4633.

Davisson et al., J. Org. Chem., 1987, 52, p 1794-1801.

Davisson et al., J. Org. Chem., 1986,51, p 4768-4779.

Davodeau et al, (1993) J. Immunology 151(3): 1214-1223.

Espinosa, E., Belmant, C., Pont, F., Luciani, B., Poupot, R., Romagne, F., Brailly, H., Bonneville, M., and Fournie, J. J. (2001 a). Chemical synthesis and biological activity of bromohydrin pyrophosphate, a potent stimulator of human gamma delta T cells. J Biol Chem 276, 18337-18344.

Espinosa, E., Belmant, C., Sicard, H., Poupot, R., Bonneville, M., and Fournie, J. J. (2001b). Y2K+1 state-of-the-art on non-peptide phosphoantigens, a novel category of immunostimulatory molecules. Microbes Infect 3, 645-654.

Ferrarini, M., Heltai, S., Pupa, S. M., Mernard, S., and Zocchi, R. (1996). Killing of laminin receptor-positive human lung cancers by tumor infiltrating lymphocytes bearing gammadelta(+) t-cell receptors. J Natl Cancer Inst 88, 436-441.

Ferrarini, M., Pupa, S. M., Zocchi, M. R., Rugarli, C., and Menard, S. (1994). Distinct pattern of HSP72 and monomeric laminin receptor expression in human lung cancers infiltrated by gamma/delta T lymphocytes. Int J Cancer 57, 486-490.

Feurle, J., Espinosa, E., Eckstein, S., Pont, F., Kunzmann, V., Fournie, J. J., Herderich, M., and Wilhelm, M. (2002). Escherichia coli produces phosphoantigens activating human gamma delta T cells. J Biol Chem 277, 148-154.

Fisch, P., Moris, A., Rammensee, H. G., and Handgretinger, R. (2000). Inhibitory MHC class I receptors on gammadelta T cells in tumour immunity and autoimmunity. Immunol Today 21, 187-191.

Fournie, J. J., and Bonneville, M. (1996). Stimulation of gamma delta T cells by phosphoantigens. Res Immunol, 66th Forum in Immunology, 147, 338-347.

Fujimiya, Y., Suzuki, Y., Katakura, R., Miyagi, T., Yamaguchi, T., Yoshimoto, T., and Ebina, T. (1997). In vitro interleukin 12 activation of peripheral blood CD3(+)CD56(+) and CD3(+)CD56(-) gammadelta T cells from glioblastoma patients. Clin Cancer Res 3, 633-643.

Girardi, M., Oppenheim, D. E., Steele, C. R., Lewis, J. M., Glusac, E., Filler, R., Hobby, P., Sutton, B., Tigelaar, R. E., and Hayday, A. C. (2001). Regulation of cutaneous malignancy by gammadelta T cells. Science 294, 605-609.

Gober, H. J., Kistowska, M., Angman, L., Jeno, P., Mori, L., and De Libero, G. (2003). Human T cell receptor gammadelta cells recognize endogenous mevalonate metabolites in tumor cells. J Exp Med 197, 163-168.

Hayday, A. C. (2000). [gamma] [delta] cells: a right time and a right place for a conserved third way of protection. Annu Rev Immunol 18, 975-1026.

Hintz et al., (2001). FEBS Lett. Dec 7;509(2):317-22.

Hintz et al (2001) (FEBS Letters 509:317-322.

Hecht et al, Tetrahedron Letters (2002) 43: 8929.

Jomaa, H., Feurle, J., Luhs, K., Kunzmann, V., Tony, H. P., Herderich, M., and Wilhelm, M. (1999a). Vgamma9/Vdelta2 T cell activation induced by bacterial low molecular mass compounds depends on the 1-deoxy-D-xylulose 5-phosphate pathway of isoprenoid biosynthesis. FEMS Immunol Med Microbiol 25, 371-378.

Jomaa, H., Wiesner, J., Sanderbrand, S., Altincicek, B., Weidemeyer, C., Hintz, M., Turbachova, I., Eberl, M., Zeidler, J., Lichtenthaler, H. K., et al. (1999b). Inhibitors of the nonmevalonate pathway of isoprenoid biosynthesis as antimalarial drugs. Science 285, 1573-1576

Kato, Y., Tanaka, Y., Miyagawa, F., Yamashita, S., and Minato, N. (2001). Targeting of tumor cells for human gammadelta T cells by nonpeptide antigens. J Immunol 167, 5092-5098.

Kim, S., Iizuka, K., Aguila, H. L., Weissman, I. L., and Yokoyama, W. M. (2000). In vivo natural killer cell activities revealed by natural killer cell-deficient mice. Proc Natl Acad Sci U S A 97, 2731-2736.

Knorre et al., Febs letters, 1976, 70, 105-108.

Kobayashi, H., Tanaka, Y., Yagi, J., Toma, H., and Uchiyama, T. (2001). Gamma/delta T cells provide innate immunity against renal cell carcinoma. Cancer Immunol Immunother 50,115-124

Kunzmann, V., Bauer, E., Feurle, J., Weissinger, F., Tony, H. P., and Wilhelm, M. (2000). Stimulation of gammadelta T cells by aminobisphosphonates and induction of antiplasma cell activity in multiple myeloma. Blood 96, 384-392.

Kunzmann, V., Bauer, E., and Wilhelm, M. (1999). Gamma/delta T-cell stimulation by pamidronate. N Engl J Med 340, 737-738.

Lang, F., Peyrat, M. A., Constant, P., Davodeau, F., David-Ameline, J., Poquet, Y., Vie, H., Fournie, J. J., and Bonneville, M. (1995). Early activation of human V gamma 9V delta 2 T cell broad cytotoxicity and TNF production by nonpeptidic mycobacterial ligands. J Immunol 154, 5986-5994.

Liu et al., Angew. Chem. Int. Ed. 1999, 38, No 9, p 1245-1247.

Mitropoulos, D., Kooi, S., Rodriguez-Villanueva, J., and Platsoucas, C. D. (1994). Characterization of fresh (uncultured) tumour-infiltrating lymphocytes (TIL) and TIL-derived T cell lines from patients with renal cell carcinoma. Clin Exp Immunol 97, 321-327.

Miyagawa, F., Tanaka, Y., Yamashita, S., and Minato, N. (2001). Essential requirement of antigen presentation by monocyte lineage cells for the activation of primary human gamma delta T cells by aminobisphosphonate antigen. J Immunol 166, 5508-5514.

Moreau et al, (1986) J. Clin. Invest. 78:874.

Morita, C. T., Beckman, E. M., Bukowski, J. F., Tanaka, Y., Band, H., Bloom, B. R., Golan, D. E., and Brenner, M. B. (1995). Direct presentation of nonpeptide prenyl pyrophosphate antigens to human gamma delta T cells. Immunity 3, 495-507.

Nakamura et al, Tetrahedron Letters n°2, p 111-112 (1973).

Nilsson et al., Acta Chemica Scandinavia B 35, 1981, p 19-27.

Nilsson and Mosbach, Eur. J. Biochem., 1980, vol. 112, p 397-402.

Poccia, F., Cipriani, B., Vendetti, S., Colizzi, V., Poquet, Y., Battistini, L., Lopez-Botet, M., Fournie, J. J., and Gougeon, M. L. (1997a). CD94/NKG2 inhibitory receptor complex modulates both anti-viral and anti-tumoral responses of polyclonal phosphoantigen-reactive V gamma 9V delta 2 T lymphocytes. J Immunol 159, 6009-6017.

Poccia, F., Malkovsky, M., Gougeon, M. L., Bonneville, M., Lopez-Botet, M., Fournie, J. J., and Colizzi, V. (1997b). Gammadelta T cell activation or anergy during infections: the role of nonpeptidic TCR ligands and HLA class I molecules. J Leukoc Biol 62, 287-291.

Poquet, Y., Kroca, M., Halary, F., Stenmark, S., Peyrat, M. A., Bonneville, M., Fournie, J. J., and Sjostedt, A. (1998). Expansion of Vgamma9 Vdelta2 T cells is triggered by Francisella tularensis-derived phosphoantigens in tularemia but not after tularemia vaccination. Infect Immun 66, 2107-2114.

Rohmer, M., Knani, M., Simonin, P., Sutter, B., and Sahm, H. (1993). Isoprenoid biosynthesis in bacteria: a novel pathway for the early steps leading to isopentenyl diphosphate. Biochem J 295 ( Pt 2), 517-524.

Rojas, R. E., Torres, M., Fournie, J. J., Harding, C. V., and Boom, W. H. (2002). Phosphoantigen presentation by macrophages to mycobacterium tuberculosis-reactive Vgamma9Vdelta2+ T cells: modulation by chloroquine. Infect Immun 70, 4019-4027.

Selin, L. K., Stewart, S., Shen, C., Mao, H. Q., and Wilkins, J. A. (1992). Reactivity of gamma delta T cells induced by the tumour cell line RPMI 8226: functional heterogeneity of clonal populations and role of GroEL heat shock proteins. Scand J Immunol 36, 107-117.

Shen, Y., Zhou, D., Qiu, L., Lai, X., Simon, M., Shen, L., Kou, Z., Wang, Q., Jiang, L., Estep, J., et al. (2002). Adaptive immune response of Vgamma2Vdelta2+ T cells during mycobacterial infections. Science 295, 2255-2258.

Sicard, H., Al Saati, T., Delsol, G., and Fournie, J. J. (2001). Synthetic phosphoantigens enhance human Vgamma9Vdelta2 T lymphocytes killing of non-Hodgkin's B lymphoma. Mol Med 7, 711-722.

Sicard, H., and Fournie, J. J. (2000). Metabolic routes as targets for immunological discrimination of host and parasite. Infect Immun 68, 4375-4377.

Smyth, M. J., Cretney, E., Takeda, K., Wiltrout, R H., Sedger, L. M., Kayagaki, N., Yagita, H., and Okumura, K. (2001). Tumor necrosis factor-related apoptosis-inducing ligand (TRAIL) contributes to interferon gamma-dependent natural killer cell protection from tumor metastasis. J Exp Med 193, 661-670.

Smyth, M. J., Thia, K. Y., Street, S. E., Cretney, E., Trapani, J. A., Taniguchi, M., Kawano, T., Pelikan, S. B., Crowe, N. Y., and Godfrey, D. 1. (2000). Differential tumor surveillance by natural killer (NK) and NKT cells. J Exp Med 191, 661-668.

Sturm, E., Braakman, E., Fisch, P., Vreugdenhil, R. J., Sondel, P., and Bolhuis, R. L. (1990). Human V gamma 9-V delta 2 T cell receptor-gamma delta lymphocytes show specificity to Daudi Burkitt's lymphoma cells. J Immunol 145, 3202-3208.

Tanaka, Y., Morita, C. T., Nieves, E., Brenner, M. B., and Bloom, B. R. (1995). Natural and synthetic non-peptide antigens recognized by human gamma delta T cells. Nature 375, 155-158.

Umbreit and Sharpless, JACS, 99-16, pp 5526-5527 (1977).

Valentijn et al., Syn. Lett., p 663-664 (1991).

Wilhelm, M., Kunzmann, V., Eckstein, S., Reimer, P., Weissinger, F., Ruediger, T., and Tony, H. P. (2003). {gamma} {delta} T cells for immune therapy of patients with lymphoid malignancies. Blood.

Wolff et al, Tetrahedron Letters (2002) 43:2555.

Yamaguchi, T., Fujimiya, Y., Suzuki, Y., Katakura, R., and Ebina, T. (1997). A simple method for the propagation and purification of gamma delta T cells from the peripheral blood of glioblastoma patients using solid-phase anti-CD3 antibody and soluble IL-2. J Immunol Methods 205, 19-28.

Zheng, B., Lam, C., Im, S., Huang, J., Luk, W., Lau, S. Y., Yau, K. K., Wong, C., Yao, K., and Ng, M. H. (2001a). Distinct tumour specificity and I-7 requirements of CD56(-)and CD56(+) subsets of human gamma delta T cells. Scand J Immunol 53, 40-48.

Zheng, B. J., Chan, K. W., Im, S., Chua, D., Sham, J. S., Tin, P. C., He, Z. M., and Ng, M. H. (2001b). Anti-tumor effects of human peripheral gammadelta T cells in a mouse tumor model. Int J Cancer 92, 421-425.

Zoretic and Zhang, Tetrahedron Letters, vol 37-11, p1751-1754 (1996).

## Claims

**1.** The use of a γδT cell activator, together with a pharmaceutically acceptable carrier, for the manufacture of a pharmaceutical composition for the treatment of a solid tumor in a warm-blooded animal in need of such treatment, wherein said γδ T cell activator is provided in an amount sufficient to induce an at least 5-fold increase in the γδ T cell population in a subject and wherein the γδ T cell activator is a compound of formula (II):

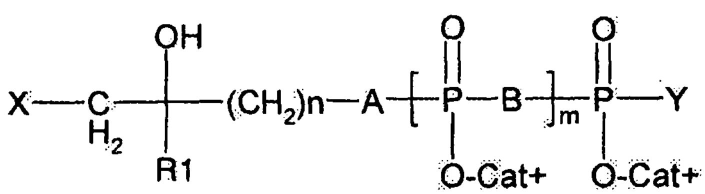

(II)

in which X is an halogen, B is O or NH, m is an integer from 1 to 3, R1 is a methyl or ethyl group, Cat+ represents one (or several, identical or different) organic or mineral cation(s) including the proton, and n is an integer from 2 to 20, A is O, NH, CHF, $CF_2$ or $CH_2$, and Y is O-Cat+, a nucleoside, or a radical -A-R, wherein R is selected from the group consisting of:

1)

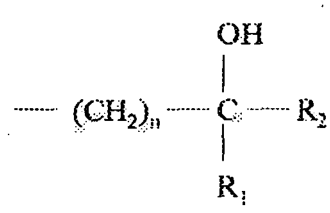

wherein n is an integer from 2 to 20, $R_1$ is a $(C_1\text{-}C_3)$alkyl group, and $R_2$ is an halogenated $(C_1\text{-}C_3)$alkyl, a $(C_1\text{-}C_3)$alkoxy-$(C_1\text{-}C_3)$alkyl, an halogenated $(C_2\text{-}C_3)$acyl or a $(C_1\text{-}C_3)$alkoxy-$(C_2\text{-}C_3)$acyl ;

2)

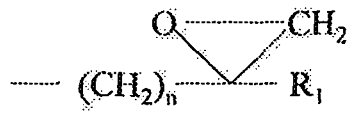

wherein n is an integer from 2 to 20, and $R_1$ is a methyl or ethyl group; and
3)

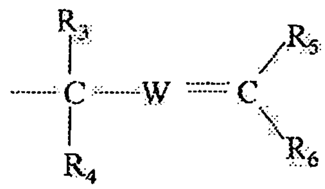

wherein $R_3$, $R_4$, and $R_5$, identical or different, are a hydrogen or ($C_1$-$C_3$)alkyl group, W is -CH- or -N-, and $R_6$ is an ($C_2$-$C_3$)acyl, an aldehyde, an ($C_1$-$C_3$)alcohol, or an ($C_2$-$C_3$)ester.

**2.** The use of claim 1, wherein said γδ T cell activator is provided in an amount sufficient to induce an at least 10-fold increase in the γδ T cell population in a subject.

**3.** The use of claim 1 or 2, wherein at least two treatments are administered to said animal.

**4.** The use of any one of claims 1-3, wherein at least four treatments are administered to said animal.

**5.** The use of any one of the above claims, wherein the γδ T cell activator is administered in more than one treatment with an interval of about two to about eight weeks between treatments.

**6.** The use of any one of the above claims wherein the γδ T cell activator is administered in more than one treatment with an interval of about three to about four weeks between treatments.

**7.** The use of any one of the above claims, wherein said γδ T cell activator is provided in an amounts sufficient to expand the γδ T cell population in a subject to reach between 30-90% of total circulating lymphocytes in a subject.

**8.** The use of any one of the above claims, wherein the biological activity of γδ T cells are increased in said warm-blooded animal or said subject.

**9.** The use of any one of claims 1 to 8 wherein the solid tumor to be treated is a renal cancer.

**10.** The use of any one of claims 1 to 8 wherein the solid tumor to be treated is selected from the group consisting of a melanoma, ovarian cancer, colon cancer, lung cancer, pancreatic cancer, neuroblastoma, head or neck cancer, bladder cancer, breast cancer, brain cancer and gastric cancer.

**11.** The use of any one of the above claims therein the γδ T cell activator is a composition comprising a compound capable of inducing the proliferation of a γδ T cell in a pure population of γδ T cell clones when said compound is present in culture at a concentration of less than 1 mM.

**12.** The use of any one of claims 1-11, wherein the compound of formula (II) is: (R, S)-3-(bromomethyl)-3-butanol-1-yl-diphosphate.

**13.** The use of any one of claims 1-12, wherein the γδ T cell activator is administered in a dose to humans "between 10 mg/kg to 100 mg/kg.

**14.** The use of any one of claims 1-12, wherein the γδ T cell activator is administered in a dose to humans between 5 mg/kg to 60 mg/kg.

**15.** The use of any one of claims 1-12, wherein said γδ activator is administered by intravenous infusion in a dose to humans that is calculated according to the formula (I): single dose (mg/kg)=(10 to 100) * N (I), where N is the number of weeks between treatments such that N is between about 3 and about 4.

**16.** The use of any one of the above claims, wherein the γδ T cell activator is administered by intravenous infusion and wherein each infusion takes place during about 5 to about 120 min.

**17.** The use of any one of the above claims, further comprising separately administering to a subject in need thereof an effective amount of a γδ activator and an interleukin-2 polypeptide.

**18.** The use of claim 17, wherein the interleukin-2 polypeptide is administered over a period of time comprised between 1 and 10 days.

## Patentansprüche

**1.** Verwendung eines γδT Zellaktivators, zusammen mit einem pharmazeutisch verträglichen Träger, für die Herstellung einer pharmazeutischen Zusammensetzung für die Behandlung eines festen Tumors in einem warmblütigen Tier, das eine solche Behandlung benötigt, wobei der γδT Zellaktivator in einer Menge bereitgestellt wird, die ausreicht, um eine mindestens 5-fache Erhöhung der γδT Zellpopulation in einem Subjekt zu induzieren und wobei der γδT Zellaktivator eine Verbindung der Formel (II) ist:

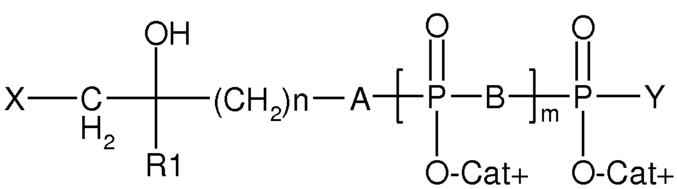

(II)

in der X ein Halogen ist, B ein O oder NH ist, m eine ganze Zahl von 1 bis 3 ist, R1 eine Methyl- oder Ethylgruppe ist, Cat+ ein (oder mehrere identische oder verschiedene) organische(s) oder anorganische(s) Kation(en) einschließlich des Protons repräsentiert, und n eine ganze Zahl von 2 bis 20 ist, A ein O, NH, CHF, $CF_2$ oder $CH_2$ ist und Y ein $O^-Cat+$, ein Nukleosid, oder ein Rest -A-R ist, wobei R ausgewählt ist aus:

1)

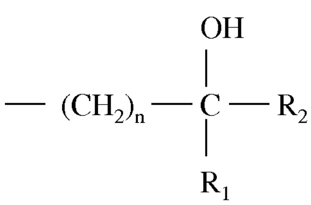

wobei n eine ganze Zahl von 2 bis 20 ist, $R_1$ eine ($C_1$-$C_3$)Alkylgruppe ist und $R_2$ ein halogeniertes ($C_1$-$C_3$)Alkyl, ein ($C_1$-$C_3$)Alkoxy-($C_1$-$C_3$)Alkyl, ein halogeniertes ($C_2$-$C_3$)Acyl oder ein ($C_1$-$C_3$)Alkoxy-($C_2$-$C_3$)Acyl ist;

2)

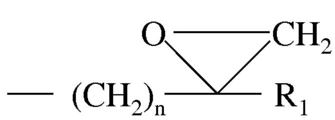

wobei n eine ganze Zahl von 2 bis 20 ist, und $R_1$ eine Methyl- oder Ethylgruppe ist; und
3)

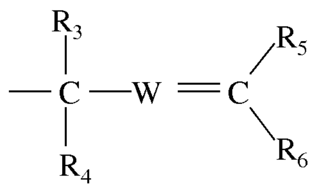

wobei $R_3$, $R_4$, und $R_5$, die identisch oder unterschiedlich sind, eine Wasserstoff- oder $(C_1-C_3)$Alkylgruppe sind, W ein -CH- oder -N- ist, und $R_6$ ein $(C_2-C_3)$Acyl, ein Aldehyd, ein $(C_1-C_3)$Alkohol, oder ein $(C_2-C_3)$Ester ist.

**2.** Die Verwendung nach Anspruch 1, wobei der γδT Zellaktivator in einer Menge bereitgestellt wird, die ausreicht, um eine mindestens 10-fache Erhöhung der γδT Zellpopulation in einem Subjekt zu induzieren.

**3.** Die Verwendung nach Anspruch 1 oder 2, wobei dem Tier mindestens zwei Behandlungen verabreicht werden.

**4.** Die Verwendung nach einem der Ansprüche 1-3, wobei dem Tier mindestens vier Behandlungen verabreicht werden.

**5.** Die Verwendung nach einem der obigen Ansprüche, wobei der γδT Zellaktivator bei mehr als einer Behandlung verabreicht wird, mit einem Intervall von ungefähr zwei bis ungefähr acht Wochen zwischen den Behandlungen.

**6.** Die Verwendung nach einem der obigen Ansprüche, wobei der γδT Zellaktivator bei mehr als einer Behandlung verabreicht wird, mit einem Intervall von ungefähr drei bis ungefähr vier Wochen zwischen den Behandlungen.

**7.** Die Verwendung nach einem der obigen Ansprüche, wobei der γδT Zellaktivator in einer Menge bereitgestellt wird, die ausreicht, um die γδT Zellpopulation in einem Subjekt so zu vergrößern, dass sie zwischen 30-90% der gesamten zirkulierenden Lymphozyten in einem Subjekt erreicht.

**8.** Die Verwendung nach einem der obigen Ansprüche, wobei die biologische Aktivität der γδT Zellen in dem warmblütigen Tier oder dem Subjekt erhöht ist.

**9.** Die Verwendung nach einem der Ansprüche 1 bis 8, wobei der zu behandelnde feste Tumor ein renaler Krebs ist.

**10.** Die Verwendung nach einem der Ansprüche 1 bis 8, wobei der zu behandelnde feste Tumor ausgewählt ist aus einem Melanom, Eierstockkrebs, Dickdarmkrebs, Lungenkrebs, Bauchspeicheldrüsenkrebs, Neuroblastom, Hals- oder Kopfkrebs, Blasenkrebs, Brustkrebs, Hirnkrebs und Magenkrebs.

**11.** Die Verwendung nach einem der obigen Ansprüche, wobei der γδT Zellaktivator eine Zusammensetzung ist, wobei die Zusammensetzung eine Verbindung umfasst, die fähig ist, die Proliferation einer γδT Zelle in einer reinen Population von γδT Zellklonen zu induzieren, wenn die Verbindung in Kultur bei einer Konzentration von weniger als 1mM vorliegt.

**12.** Die Verwendung nach einem der Ansprüche 1-11, wobei die Verbindung der Formel (II) ein (R, S)-3-(Brommethyl)-3-Butanol-1-yl-Diphosphat ist.

**13.** Die Verwendung nach einem der Ansprüche 1-12, wobei der γδT Zellaktivator in einer Dosis zwischen 10 mg/kg bis 100 mg/kg an Menschen verabreicht wird.

**14.** Die Verwendung nach einem der Ansprüche 1-12, wobei der γδT Zellaktivator in einer Dosis zwischen 5 mg/kg bis 60 mg/kg an Menschen verabreicht wird.

**15.** Die Verwendung nach einem der Ansprüche 1-12, wobei der γδT Aktivator durch intravenöse Infusion in einer Dosis an Menschen verabreicht wird, die berechnet wird nach der Formel (I): Einzeldosis (mg/kg)=(10 bis 100) * N (I), wobei N die Anzahl der Wochen zwischen Behandlungen ist, so dass N zwischen ungefähr 3 und ungefähr 4 ist.

**16.** Die Verwendung nach einem der obigen Ansprüche, wobei der γδT Zellaktivator durch intravenöse Infusion verabreicht wird und wobei jede Infusion während ungefähr 5 bis ungefähr 120 Minuten stattfindet.

**17.** Die Verwendung nach einem der obigen Ansprüche, wobei die Verwendung weiterhin die getrennte Verabreichung einer effektiven Menge eines γδT Aktivators und eines Interleukin-2 Polypeptids an ein Subjekt umfasst, das diese Verabreichung benötigt.

**18.** Die Verwendung nach Anspruch 17, wobei das Interleukin-2 Polypeptid über einen Zeitraum verabreicht wird, der zwischen 1 und 10 Tagen beträgt.

**Revendications**

**1.** Utilisation d'un activateur de cellule γδT avec un support pharmaceutiquement acceptable pour la préparation d'une composition pharmaceutique destinée au traitement d'une tumeur solide chez un animal à sang-chaud nécessitant un tel traitement, dans laquelle ledit activateur de cellule γδT est fourni en une quantité suffisante pour induire une augmentation d'au moins 5 fois dans la population de cellules γδT chez un sujet et dans laquelle l'activateur de cellule γδT est un composé de formule (II) :

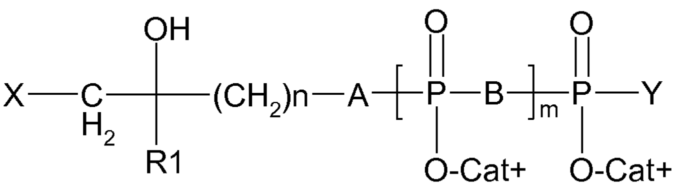
(II)

dans laquelle X est un halogène, B est O ou NH, m est un entier de 1 à 3, R1 est un groupe méthyle ou éthyle, Ca+ représente un (ou plusieurs, identiques ou différents) cation(s) organique ou minéral incluant le proton, et n est un entier de 2 à 20, A est O, NH, CHF, $CF_2$ ou $CH_2$, et Y est $O^-Cat+$, un nucléoside ou un radical -A-R, dans lequel R est sélectionné parmi le groupe consistant en :

1)

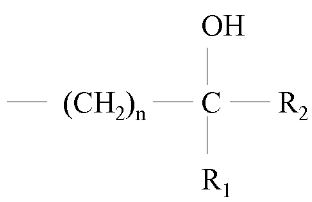

dans lequel n est un entier de 2 à 20, $R_1$ est un groupe $(C_1-C_3)$alkyle, et $R_2$ est un $(C_1-C_3)$alkyle halogéné, un $(C_1-C_3)$alkoxy-$(C_1-C_3)$alkyle, un $(C_2-C_3)$acyle halogéné ou un $(C_1-C_3)$alkoxy-$(C_2-C_3)$acyle ;
2)

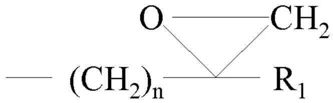

dans lequel n est un entier de 2 à 20, $R_1$ est un groupe méthyle ou éthyle ; et
3)

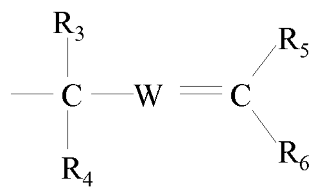

dans lequel $R_3$, $R_4$ et $R_5$, identiques ou différents, sont un hydrogène ou un groupe $(C_1-C_3)$alkyle, W est -CH- ou -N-, et $R_6$ est un $(C_2-C_3)$acyle, un aldéhyde, un $(C_1-C_3)$alcool ou un $(C_2-C_3)$ester.

**2.** Utilisation selon la revendication 1, dans laquelle ledit activateur de cellule γδT est fourni en une quantité suffisante pour induire une augmentation d'au moins 10 fois dans la population de cellules γδT chez un sujet.

**3.** Utilisation selon la revendication 1 ou 2, dans laquelle au moins deux traitements sont administrés audit animal.

**4.** Utilisation selon l'une quelconque des revendications 1 à 3, dans laquelle au moins quatre traitements sont administrés audit animal.

**5.** Utilisation selon l'une quelconque des revendications précédentes, dans laquelle l'activateur de cellule γδT est administré en plus d'un traitement avec un intervalle d'environ deux à environ huit semaines entre les traitements.

**6.** Utilisation selon l'une quelconque des revendications précédentes, dans laquelle l'activateur de cellule γδT est administré en plus d'un traitement avec un intervalle d'environ trois à environ quatre semaines entre les traitements.

**7.** Utilisation selon l'une quelconque des revendications précédentes, dans laquelle l'activateur de cellule γδT est fourni en une quantité suffisante pour étendre la population de cellules γδT chez un sujet jusqu'à entre 30-90% des lymphocytes totaux circulants chez un sujet.

**8.** Utilisation selon l'une quelconque des revendications précédentes, dans laquelle l'activité biologique des cellules γδT est augmentée dans ledit animal à sang-chaud ou ledit sujet.

**9.** Utilisation selon l'une quelconque des revendications 1-8, dans laquelle la tumeur solide à traiter est un cancer du rein.

**10.** Utilisation selon l'une quelconque des revendications 1-8, dans laquelle la tumeur solide à traiter est sélectionnée parmi le groupe constitué d'un mélanome, un cancer des ovaires, un cancer du colon, un cancer du poumon, un cancer du pancréas, un neuroblastome, un cancer de la tête ou du cou, un cancer de la vessie, une cancer du sein, un cancer du cerveau et un cancer gastrique.

**11.** Utilisation selon l'une quelconque des revendications précédentes, dans laquelle l'activateur de cellule γδT est une composition comprenant un composé capable d'induire la prolifération d'une cellule γδT en une population pure de clones de cellule γδT lorsque ledit composé est présent dans la culture à une concentration de moins de 1 mM.

**12.** Utilisation selon l'une quelconque des revendications 1-11, dans laquelle le composé de formule (II) est (R,S)-3-(bromométhyl)-3-butanol-1-yl-diphosphate.

**13.** Utilisation selon l'une quelconque des revendications 1-12, dans laquelle l'activateur de cellule γδT est administré aux humains à une dose comprise entre 10 mg/kg à 100 mg/kg.

**14.** Utilisation selon l'une quelconque des revendications 1-12, dans laquelle l'activateur de cellule γδT est administré aux humains à une dose comprise entre 5 mg/kg à 60 mg/kg.

**15.** Utilisation selon l'une quelconque des revendications 1-12, dans laquelle l'activateur de cellule γδT est administré par infusion intraveineuse aux humains à une dose qui est calculée selon la formule (I) : dose unique (mg/kg) = (10 à 100) * N (I), où N est le nombre de semaines entre les traitements de sorte que N est compris entre environ 3 et environ 4.

**16.** Utilisation selon l'une quelconque des revendications précédentes, dans laquelle l'activateur de cellule γδT est

administré par infusion intraveineuse et dans laquelle chaque infusion dure entre environ 5 à environ 120 min.

**17.** Utilisation selon l'une quelconque des revendications précédentes, comprenant en outre l'administration séparément à un sujet le nécessitant d'une quantité efficace d'un activateur de cellule γδT et d'un polypeptide d'interleukine-2.

**18.** Utilisation selon la revendication 17, dans laquelle le polypeptide d'interleukine-2 est administré sur une période de temps comprise entre 1 et 10 jours.

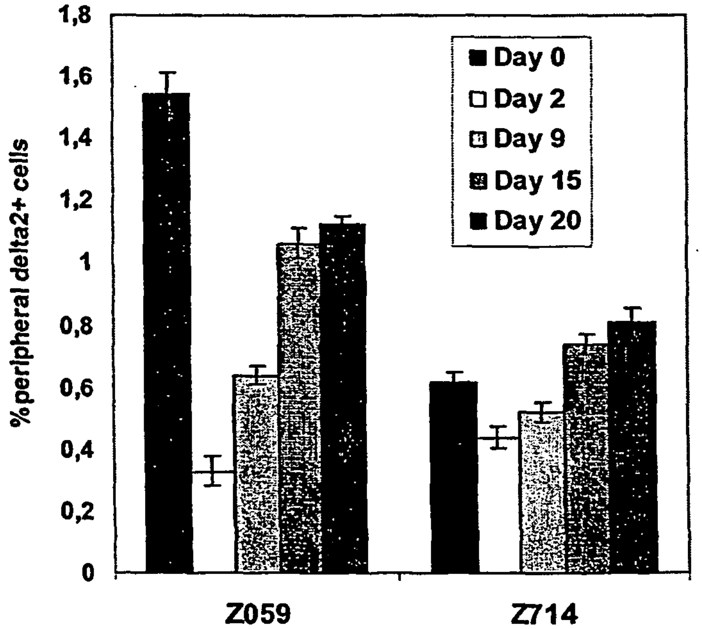
1,8
1,6
1,4
1,2
1
0,8
0,6
0,4
0,2
0
% peripheral delta2+ cells
Day 0
Day 2
Day 9
Day 15
Day 20
Z059
Z714

Figure 1

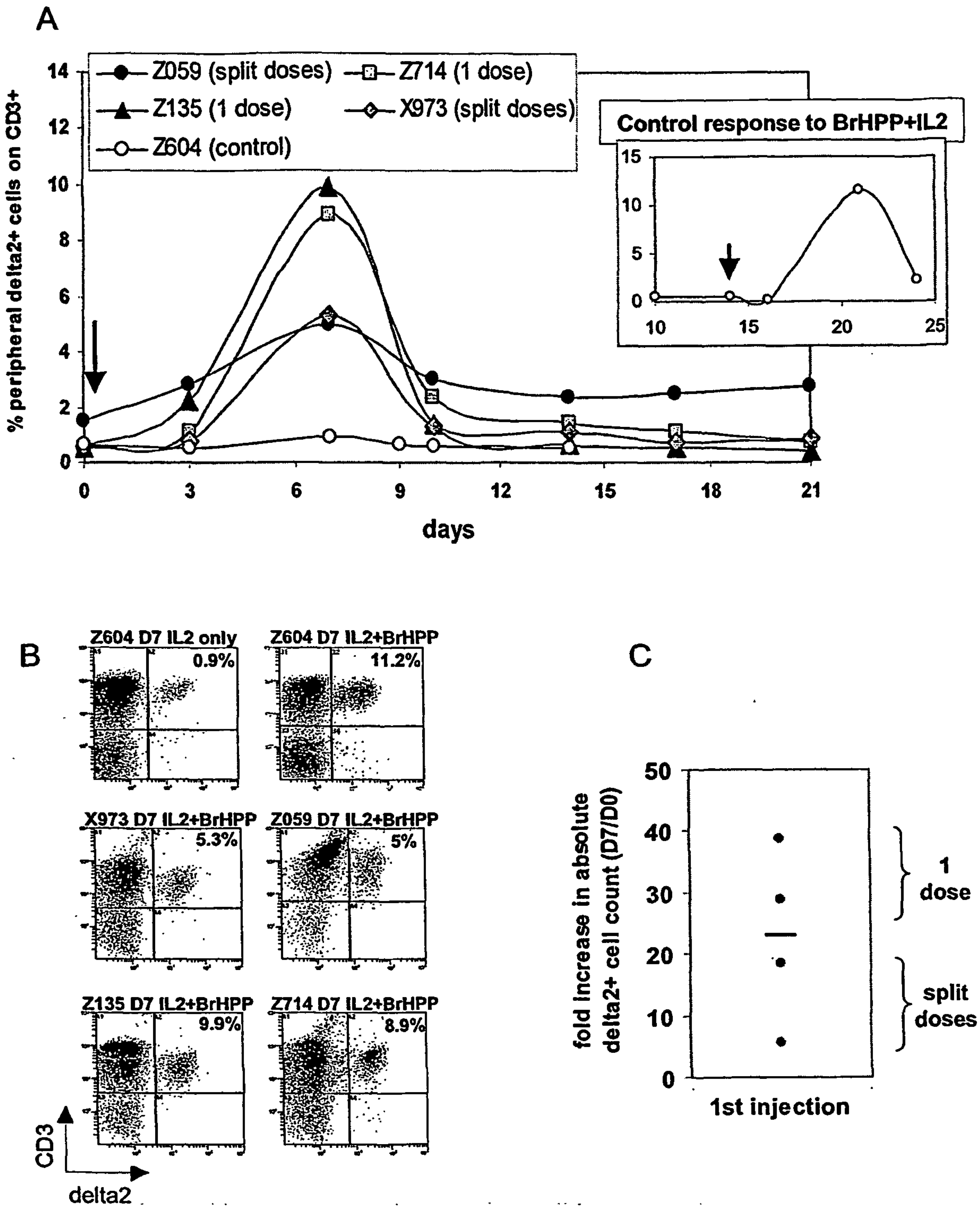
A
Z059 (split doses)
Z714 (1 dose)
Z135 (1 dose)
X973 (split doses)
Z604 (control)
% peripheral delta2+ cells on CD3+
days
Control response to BrHPP+IL2
B
Z604 D7 IL2 only
0.9%
Z604 D7 IL2+BrHPP
11.2%
X973 D7 IL2+BrHPP
5.3%
Z059 D7 IL2+BrHPP
5%
Z135 D7 IL2+BrHPP
9.9%
Z714 D7 IL2+BrHPP
8.9%
CD3
delta2
C
fold increase in absolute delta2+ cell count (D7/D0)
1 dose
split doses
1st injection

Figure 2

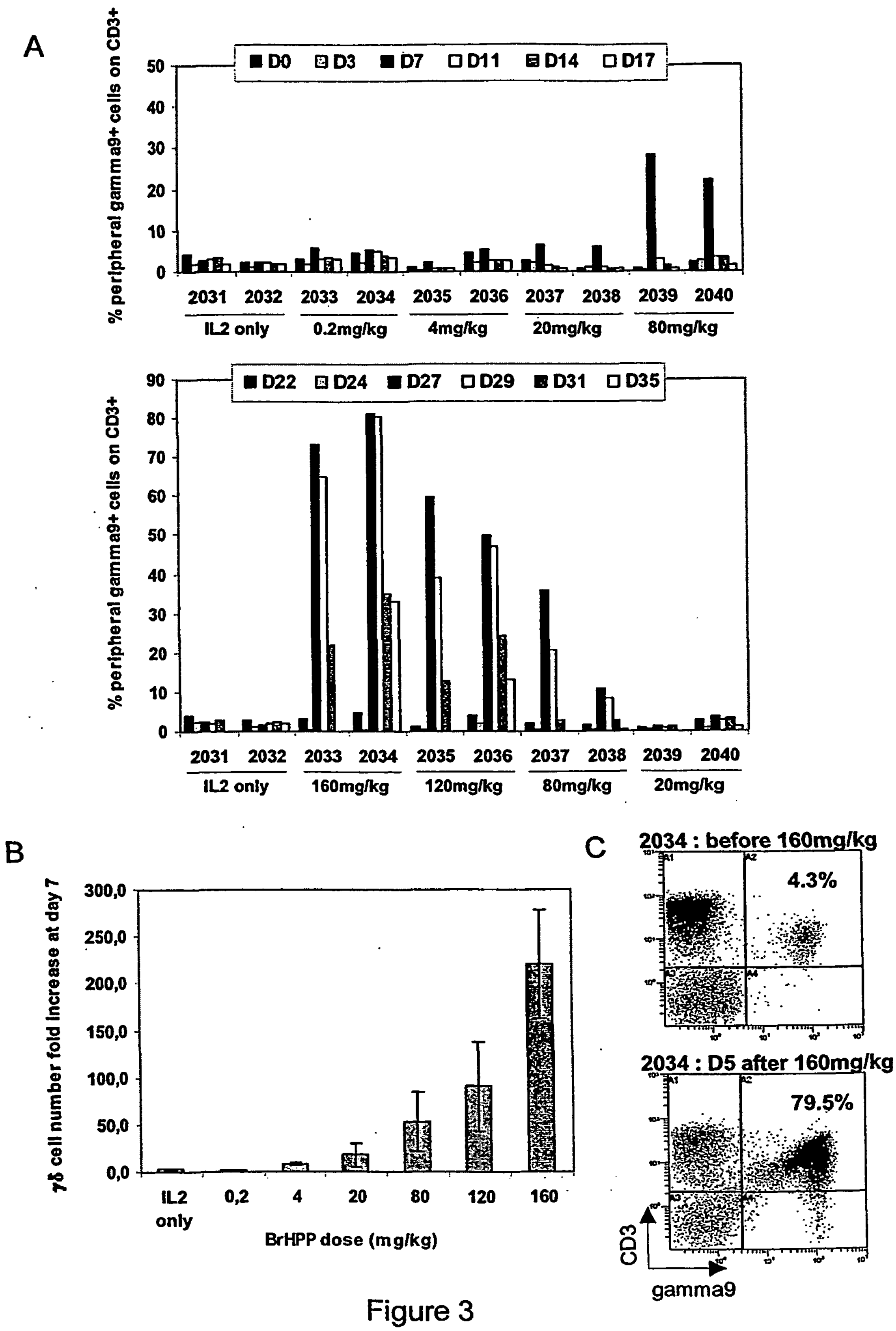
A
% peripheral gamma9+ cells on CD3+
D0 D3 D7 D11 D14 D17
50
40
30
20
10
0
2031 2032 2033 2034 2035 2036 2037 2038 2039 2040
IL2 only
0.2mg/kg
4mg/kg
20mg/kg
80mg/kg
% peripheral gamma9+ cells on CD3+
D22 D24 D27 D29 D31 D35
90
80
70
60
50
40
30
20
10
0
2031 2032 2033 2034 2035 2036 2037 2038 2039 2040
IL2 only
160mg/kg
120mg/kg
80mg/kg
20mg/kg
B
γδ cell number fold increase at day 7
300,0
250,0
200,0
150,0
100,0
50,0
0,0
IL2 only
0,2
4
20
80
120
160
BrHPP dose (mg/kg)
C
2034 : before 160mg/kg
4.3%
2034 : D5 after 160mg/kg
79.5%
CD3
gamma9

Figure 3

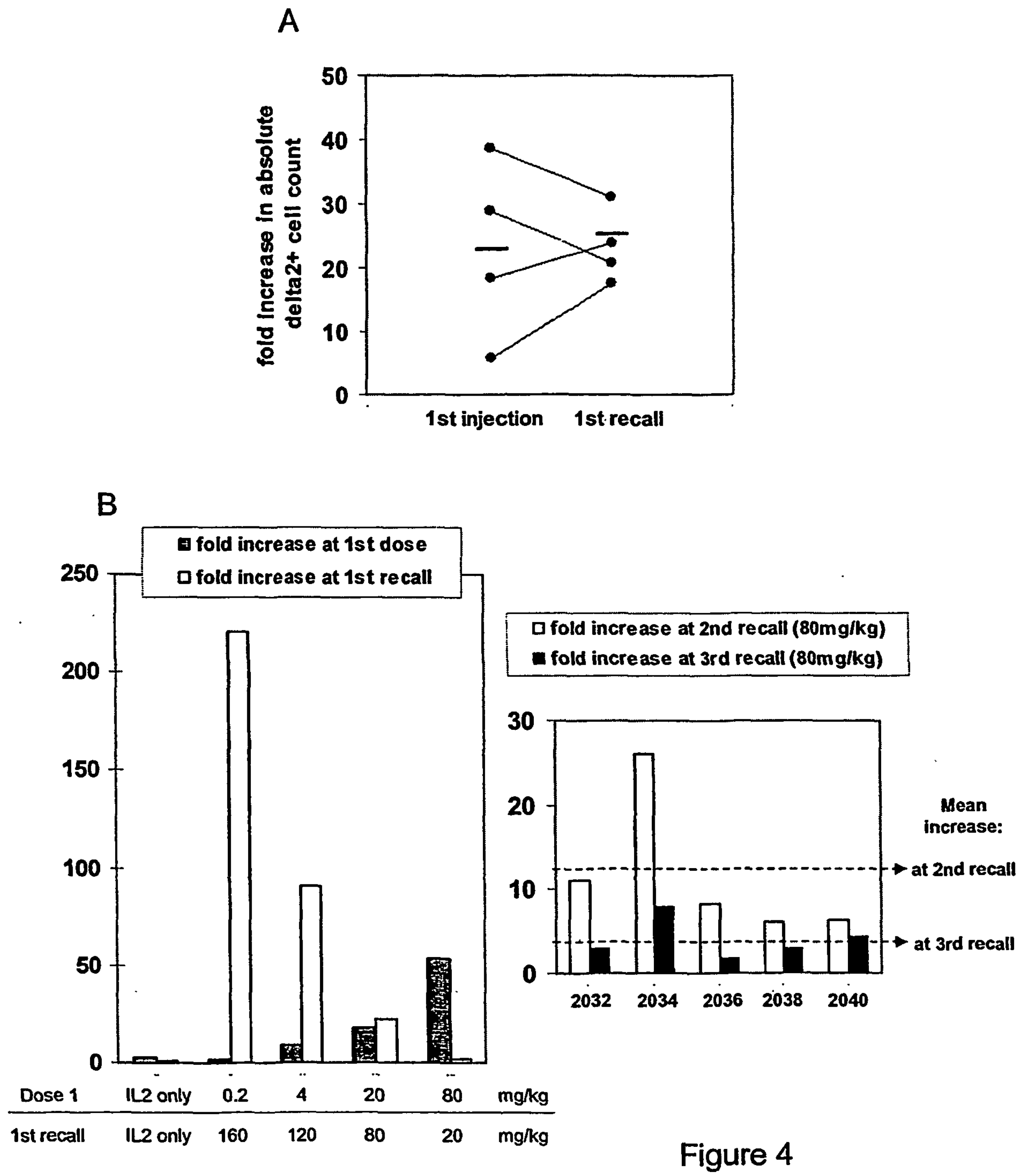
A
fold increase in absolute delta2+ cell count
50
40
30
20
10
0
1st injection
1st recall
B
fold increase at 1st dose
fold increase at 1st recall
250
200
150
100
50
0
Dose 1
IL2 only
0.2
4
20
80
mg/kg
1st recall
IL2 only
160
120
80
20
mg/kg
fold increase at 2nd recall (80mg/kg)
fold increase at 3rd recall (80mg/kg)
30
20
10
0
2032
2034
2036
2038
2040
Mean increase:
at 2nd recall
at 3rd recall

Figure 4

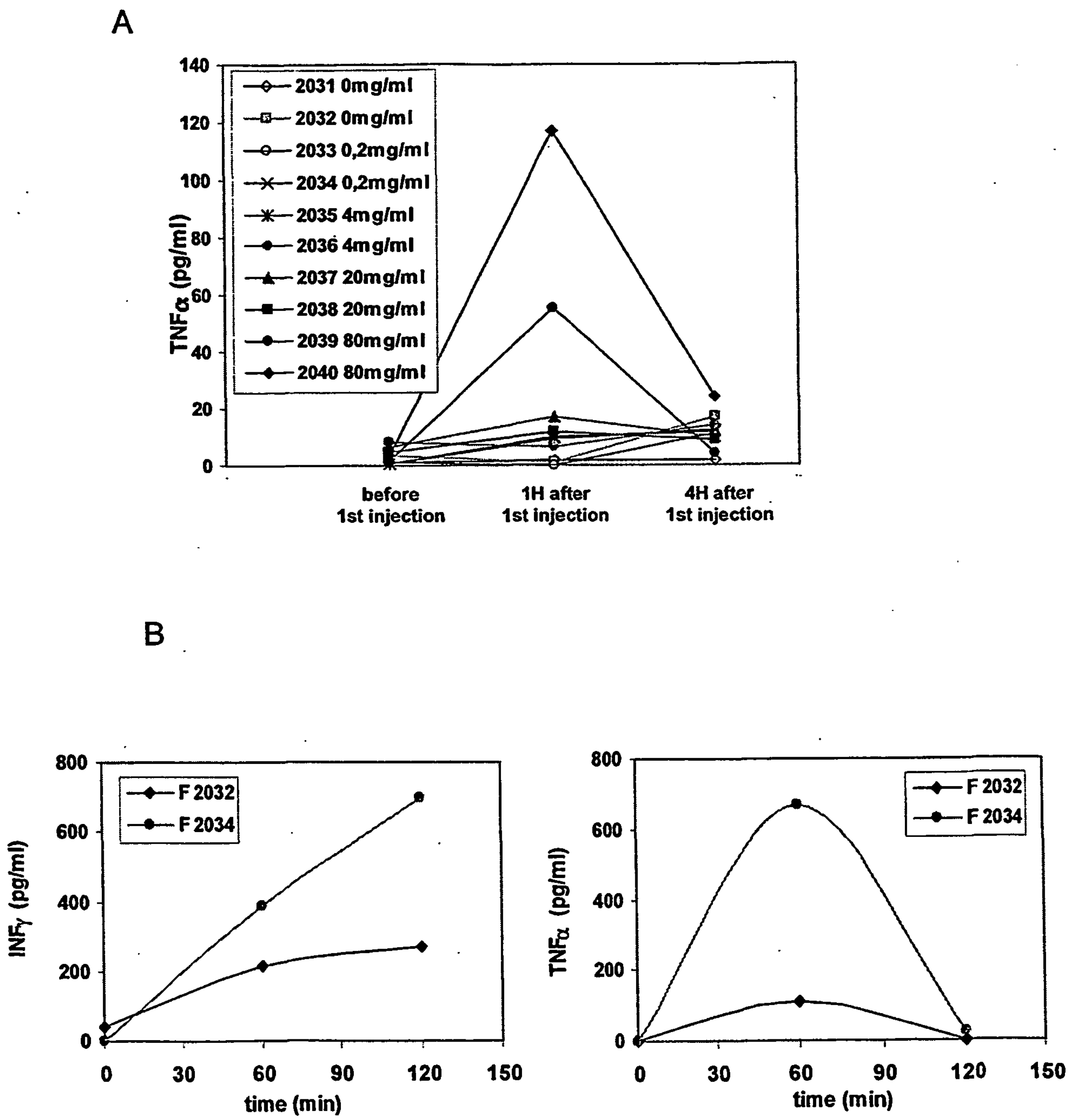
A
140
120
100
80
60
40
20
0
TNFα (pg/ml)
2031 0mg/ml
2032 0mg/ml
2033 0,2mg/ml
2034 0,2mg/ml
2035 4mg/ml
2036 4mg/ml
2037 20mg/ml
2038 20mg/ml
2039 80mg/ml
2040 80mg/ml
before 1st injection
1H after 1st injection
4H after 1st injection
B
800
600
400
200
0
INFγ (pg/ml)
F 2032
F 2034
0
30
60
90
120
150
time (min)
TNFα (pg/ml)

Figure 5

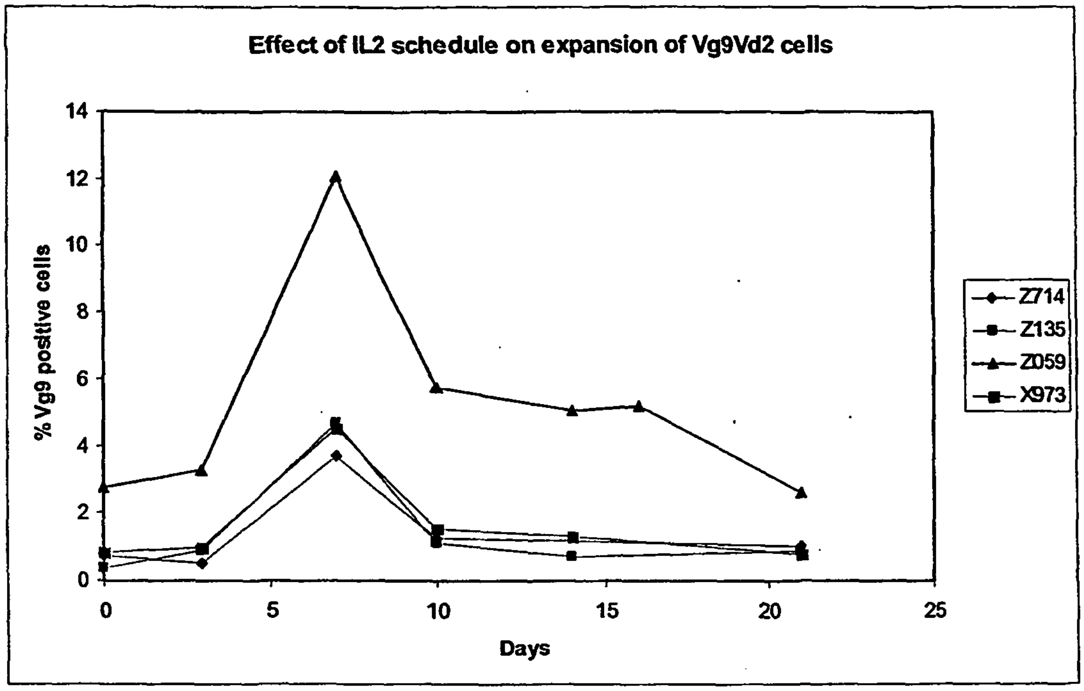
Effect of IL2 schedule on expansion of Vg9Vd2 cells
% Vg9 positive cells
14
12
10
8
6
4
2
0
0
5
10
15
20
25
Days
Z714
Z135
Z059
X973

Figure 6

FIG 7

**Primate γδ cell number fold increase in response to increasing doses of Phosphostim**

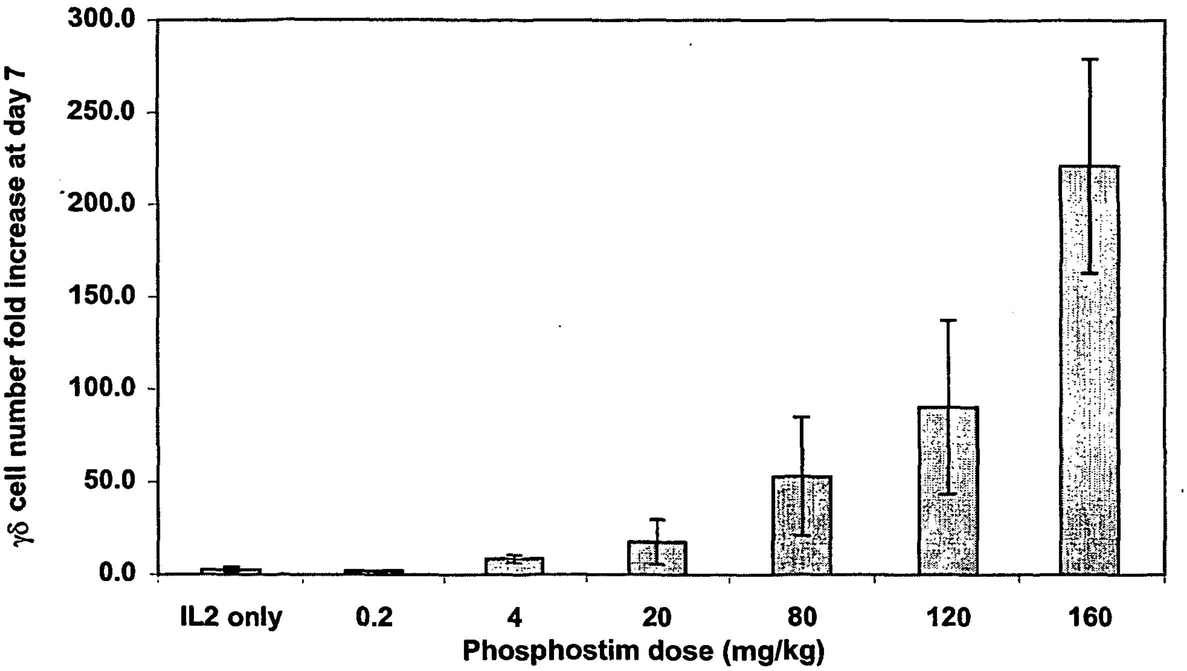

**FIG 8**

**Serum cytokine (INFγ and TNFα) production in Phosphostim treated primates**

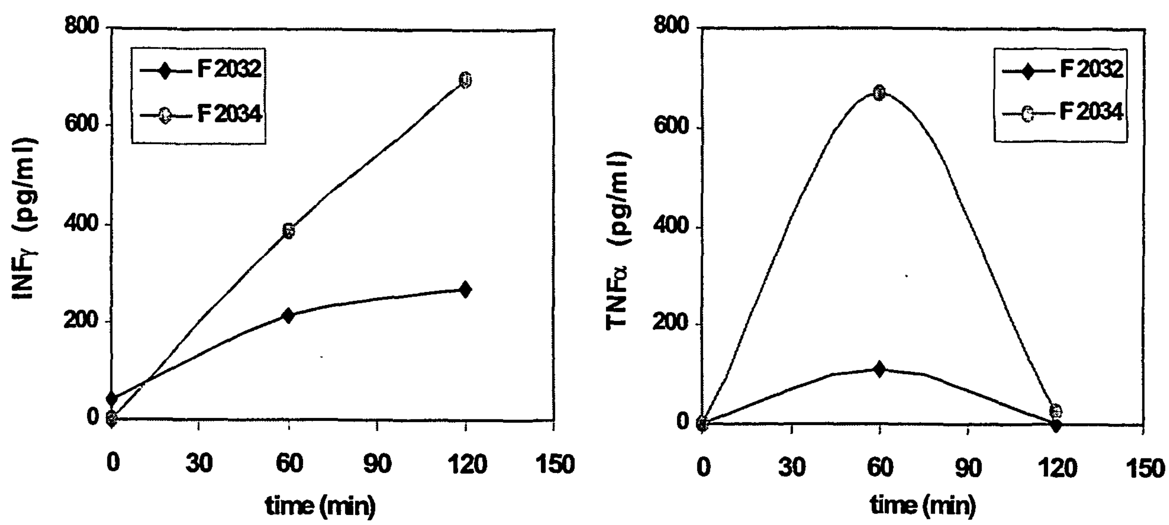

FIG 9A

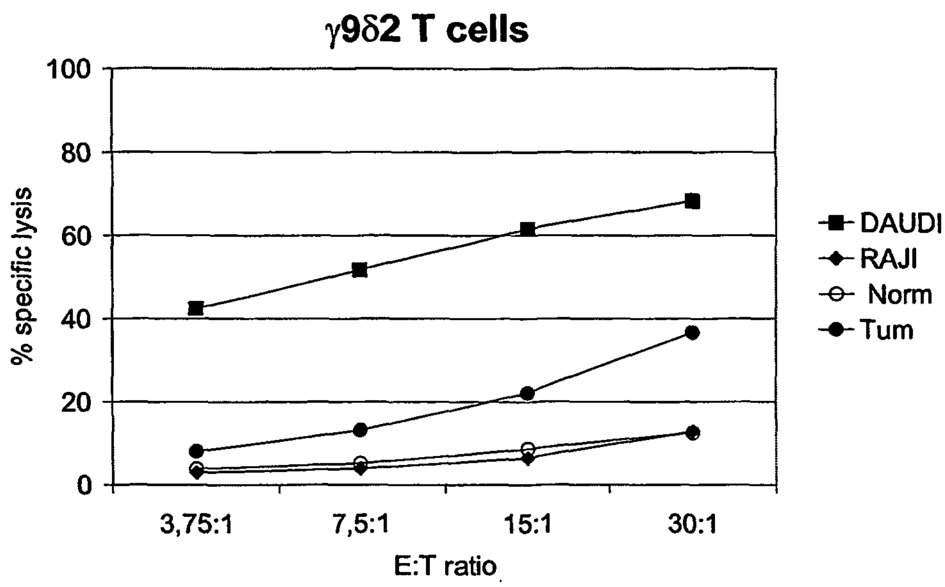
γ9δ2 T cells
100
80
60
40
20
0
% specific lysis
3,75:1
7,5:1
15:1
30:1
E:T ratio
DAUDI
RAJI
Norm
Tum

FIG9B

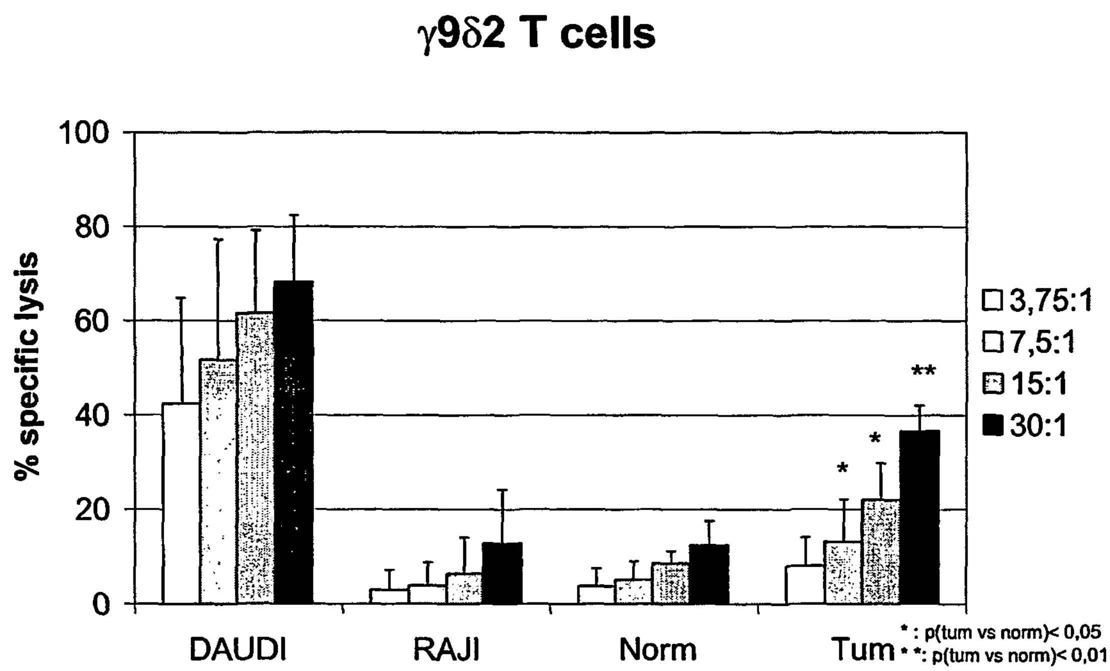
γ9δ2 T cells
% specific lysis
100
80
60
40
20
0
DAUDI
RAJI
Norm
Tum
3,75:1
7,5:1
15:1
30:1
*
*
**
* : p(tum vs norm)< 0,05
* *: p(tum vs norm)< 0,01

FIG 10

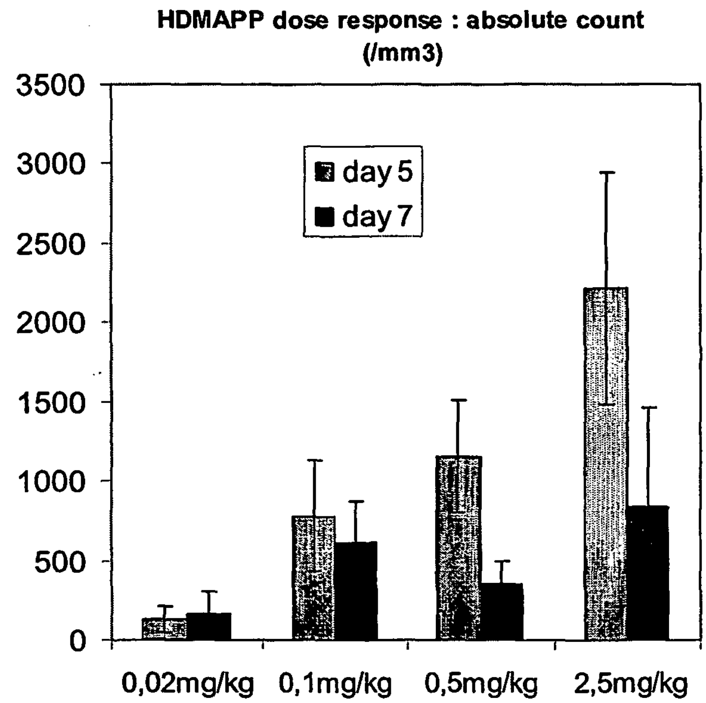
HDMAPP dose response : absolute count (/mm3)
3500
3000
2500
2000
1500
1000
500
0
day 5
day 7
0,02mg/kg
0,1mg/kg
0,5mg/kg
2,5mg/kg

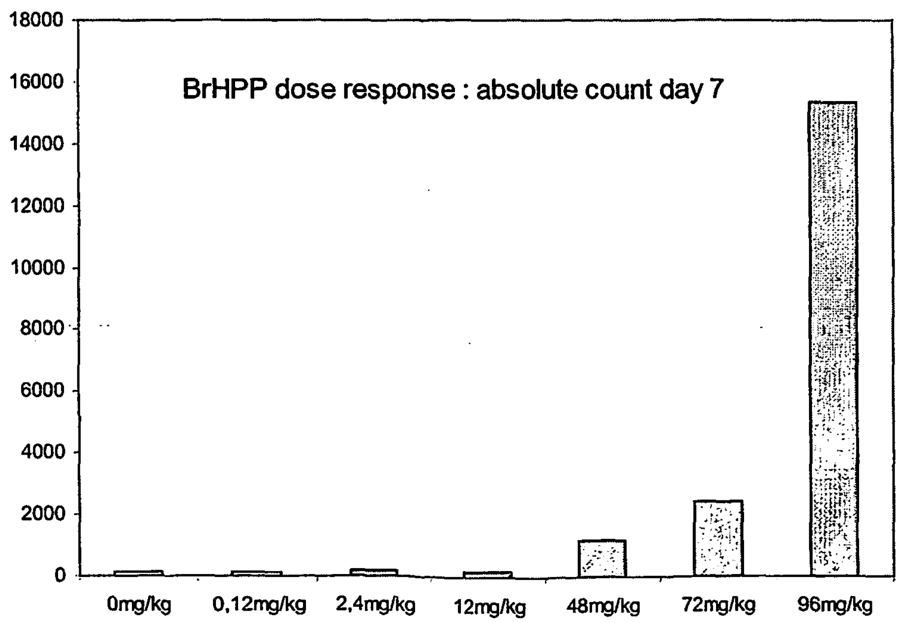
BrHPP dose response : absolute count day 7
18000
16000
14000
12000
10000
8000
6000
4000
2000
0
0mg/kg
0,12mg/kg
2,4mg/kg
12mg/kg
48mg/kg
72mg/kg
96mg/kg

FIG 11

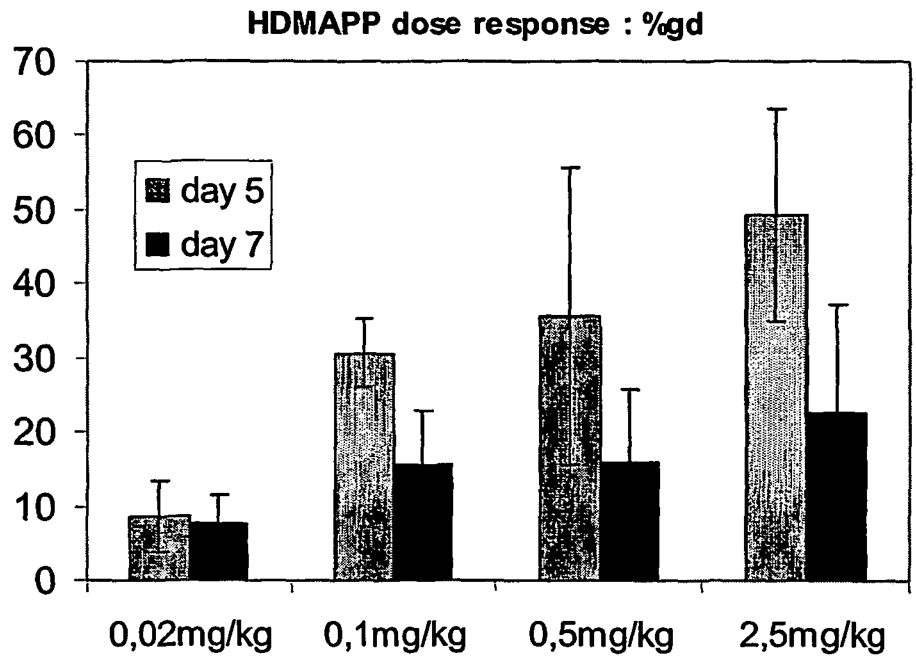
HDMAPP dose response : %gd
70
60
50
40
30
20
10
0
day 5
day 7
0,02mg/kg
0,1mg/kg
0,5mg/kg
2,5mg/kg

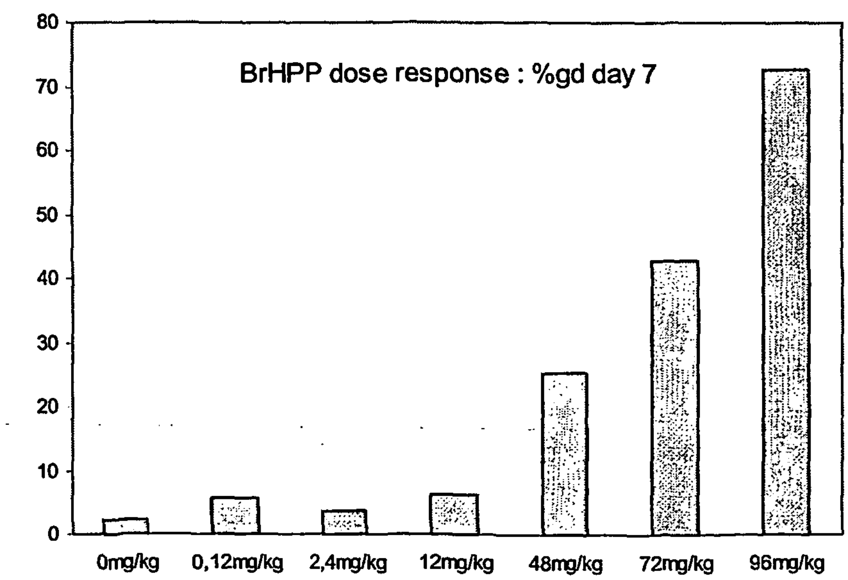
BrHPP dose response : %gd day 7
80
70
60
50
40
30
20
10
0
0mg/kg
0,12mg/kg
2,4mg/kg
12mg/kg
48mg/kg
72mg/kg
96mg/kg

FIG 12

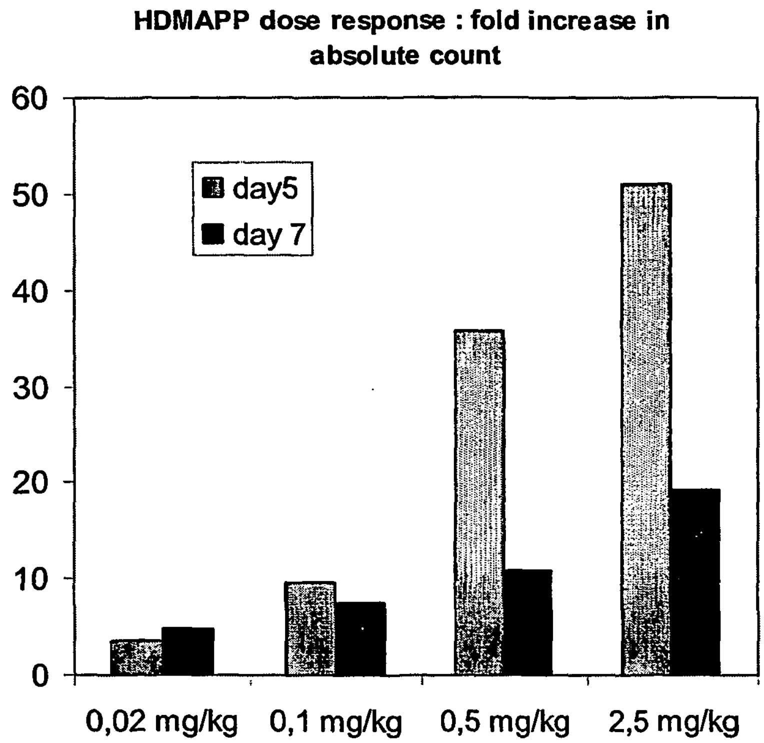
HDMAPP dose response : fold increase in absolute count
60
50
40
30
20
10
0
day5
day 7
0,02 mg/kg
0,1 mg/kg
0,5 mg/kg
2,5 mg/kg

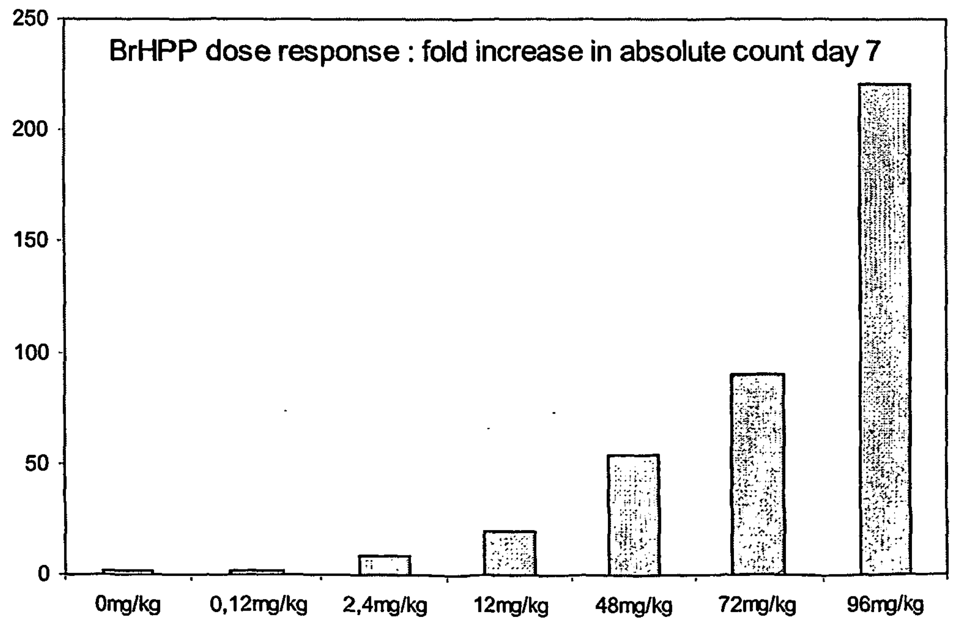
BrHPP dose response : fold increase in absolute count day 7
250
200
150
100
50
0
0mg/kg
0,12mg/kg
2,4mg/kg
12mg/kg
48mg/kg
72mg/kg
96mg/kg

**FIG 13**

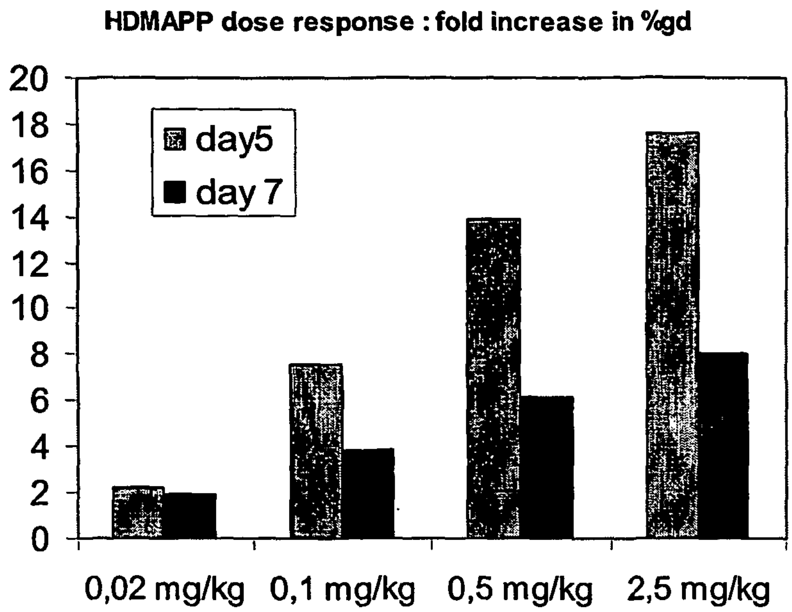
HDMAPP dose response : fold increase in %gd
20
18
16
14
12
10
8
6
4
2
0
day5
day 7
0,02 mg/kg
0,1 mg/kg
0,5 mg/kg
2,5 mg/kg

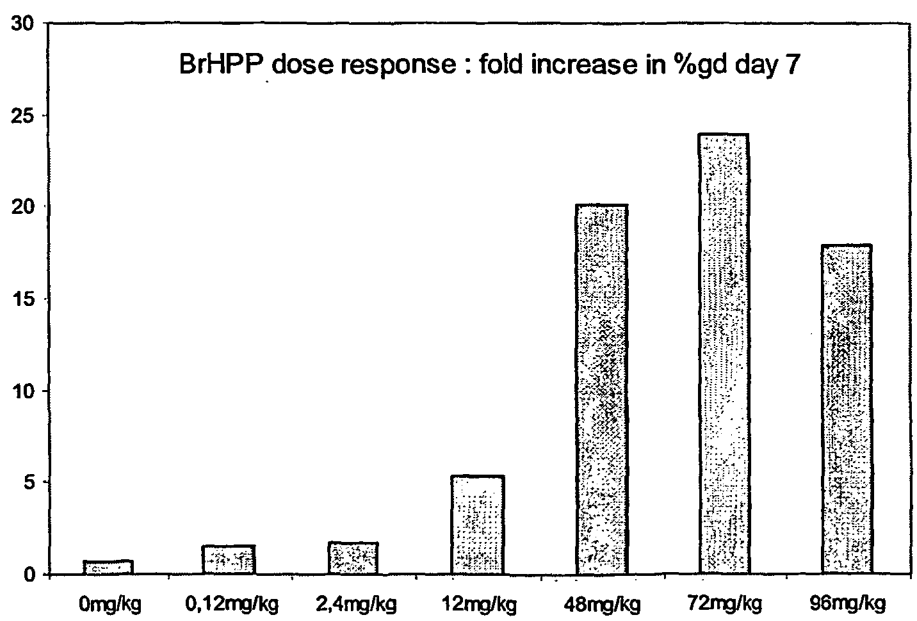
BrHPP dose response : fold increase in %gd day 7
30
25
20
15
10
5
0
0mg/kg
0,12mg/kg
2,4mg/kg
12mg/kg
48mg/kg
72mg/kg
96mg/kg

FIG 14A

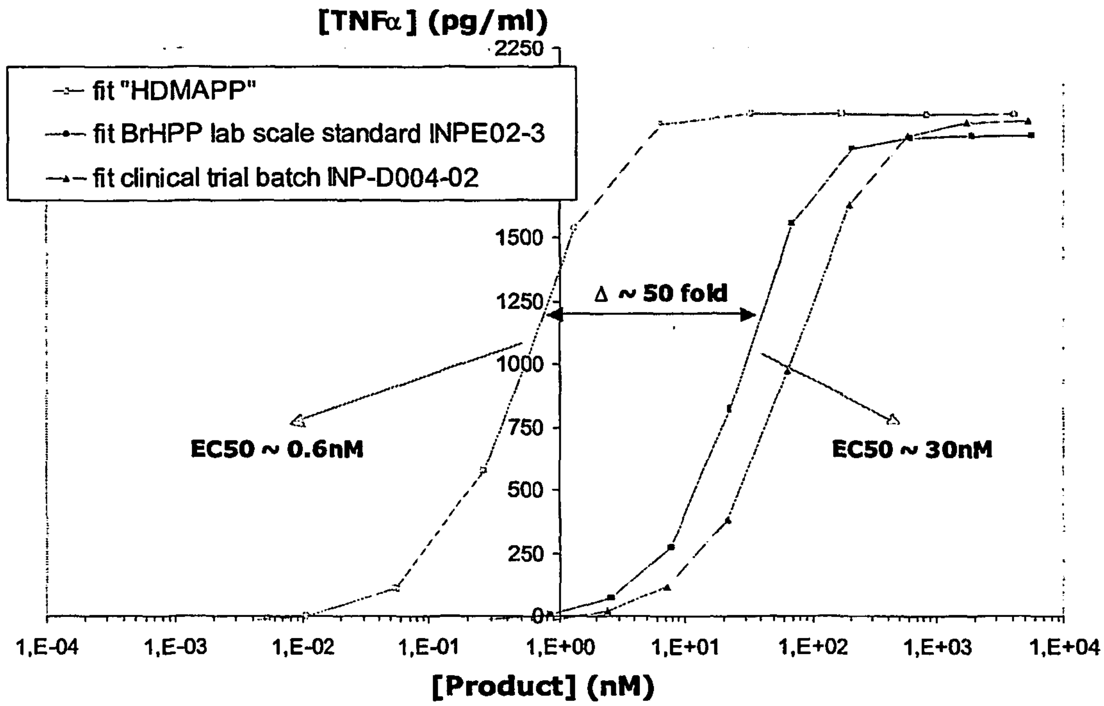


FIG 14B

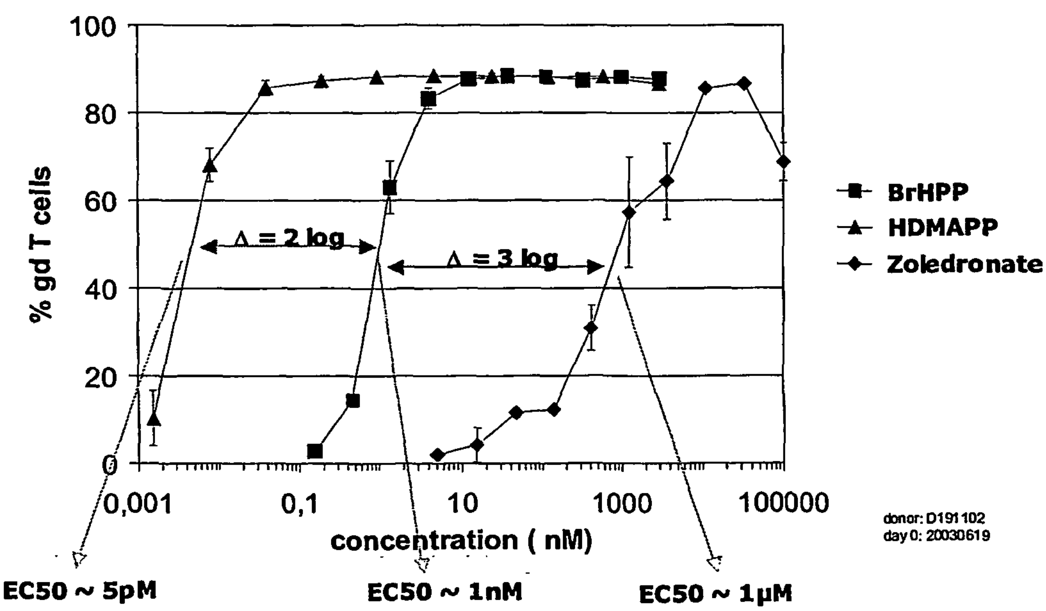

**FIG 15**
In vivo tumor development

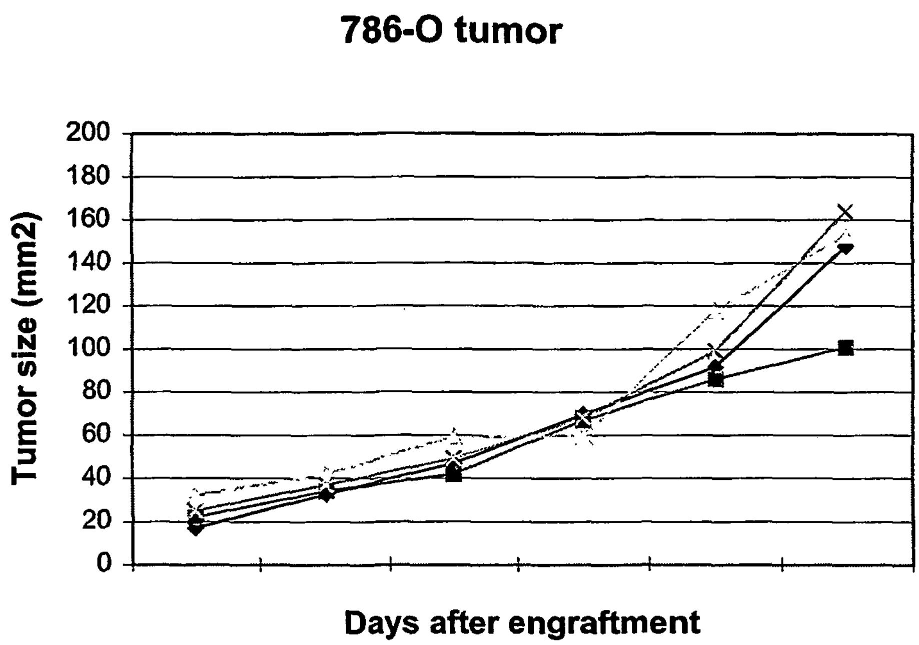

**FIG 16**

In vivo BrHPP stimulation and gdT cell proliferation

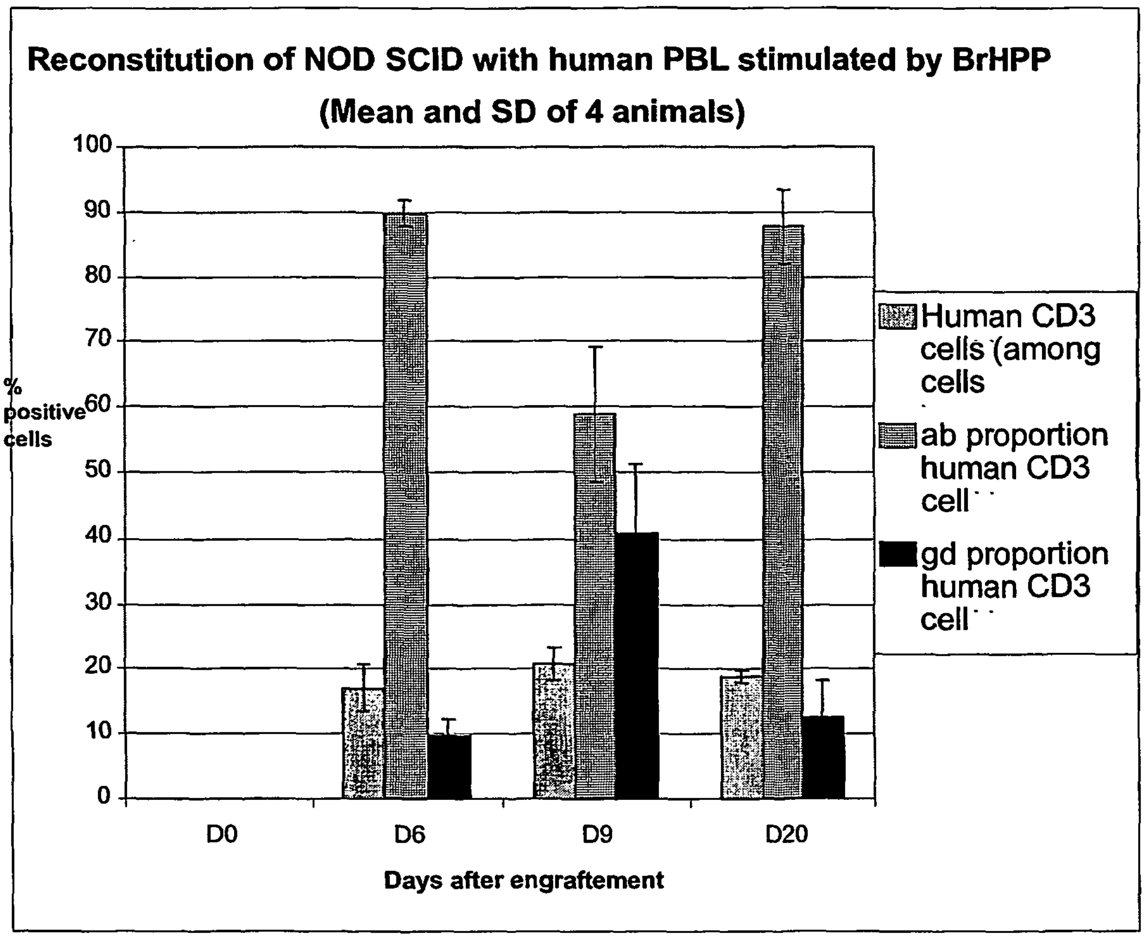

**FIG 17**

In vivo BrHPP stimulation and gdT cell proliferation

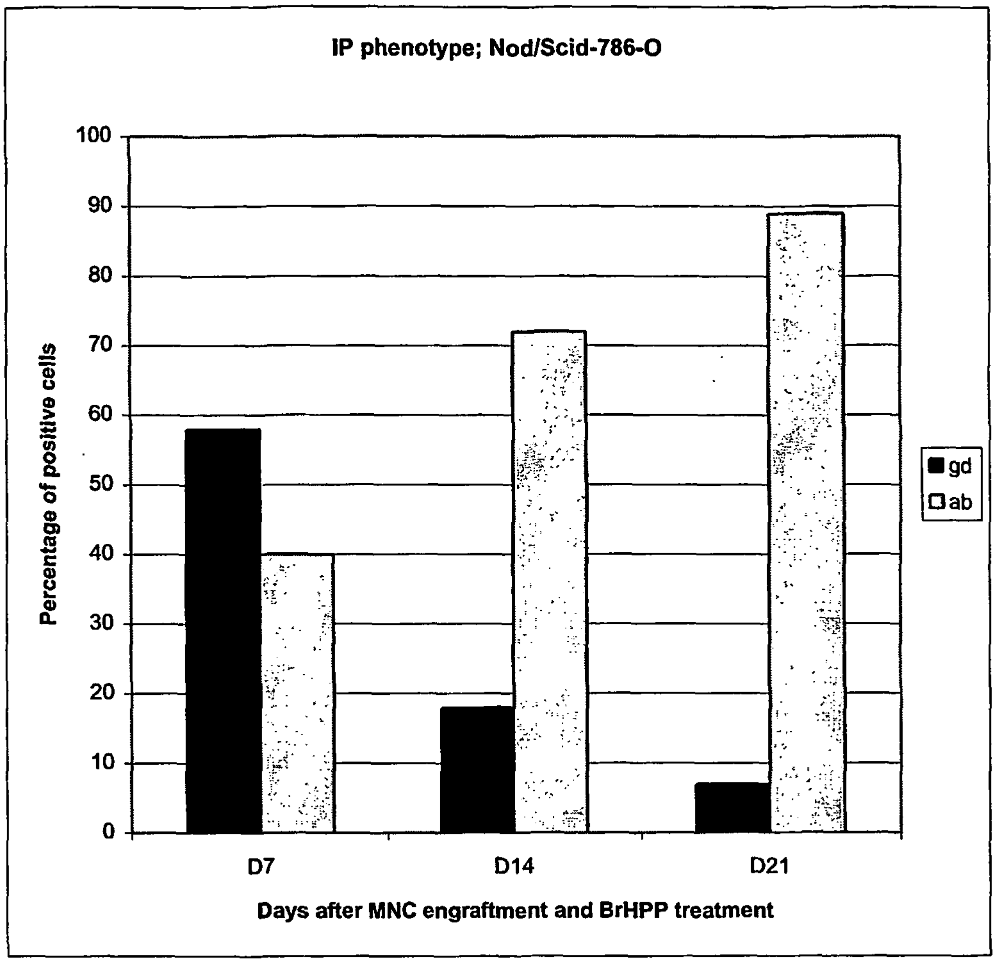

**FIG 18**

Long-term anti-tumoral activity

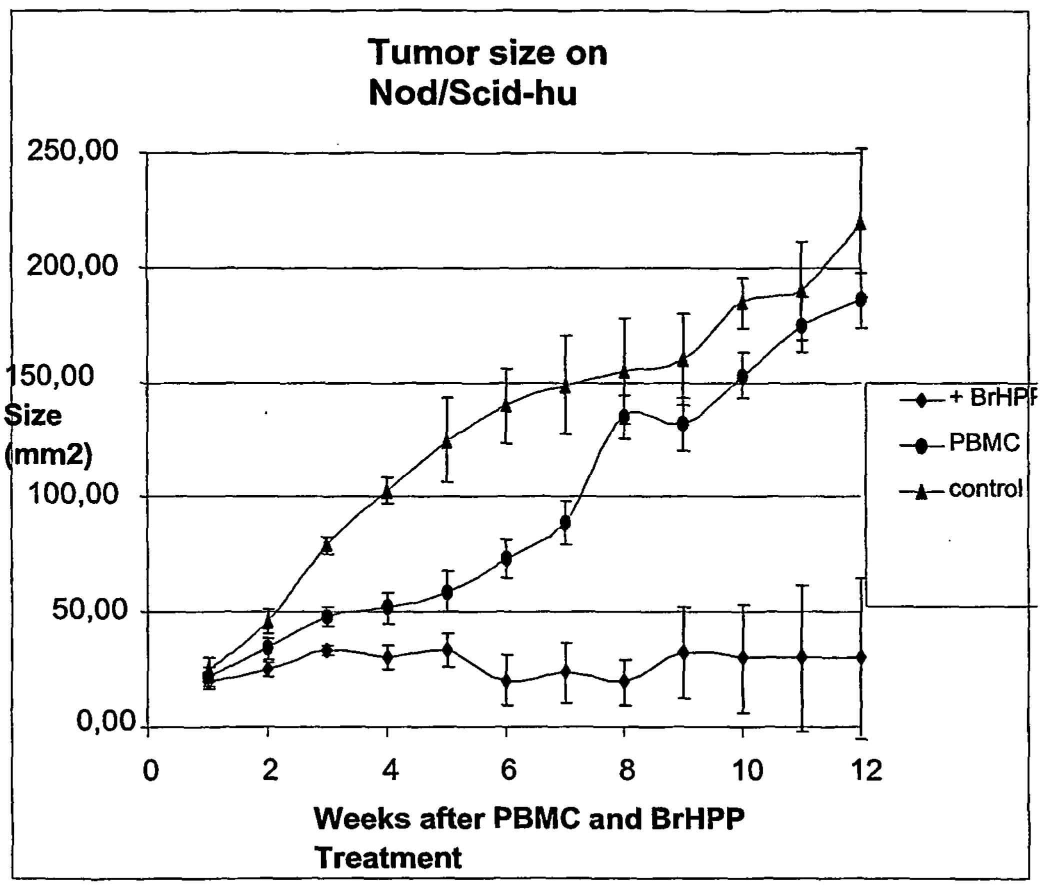

FIG 19

Gamma delta anti-tumoral activity

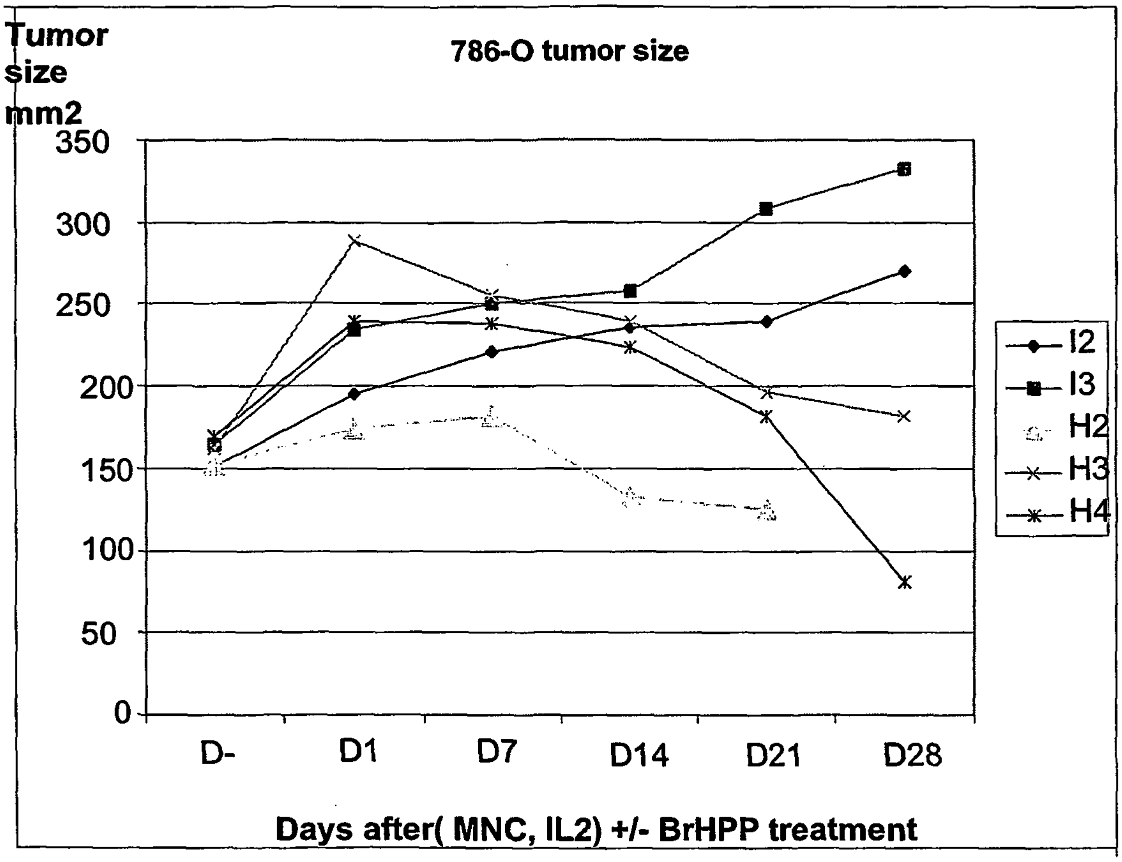

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0012516 A
- WO 0012519 A
- WO 03050128 A
- WO 03009855 A
- WO 9520673 A
- US 5639653 A
- EP 1109818 B1
- WO 03050128 PCT, Brondino


### Non-patent literature cited in the description


- **Espinosa et al.** *J. Biol. Chem.,* 2001, vol. 276 (21), 18337-18344
- **Simon, R. et al.** *J. Nat. Cancer Inst.,* 1997, vol. 89 (15), 1138-1147
- *Organic syntheses,* vol. 77, 225-228
- **Wolff et al.** *Tetrahedron Letters,* 2002, vol. 43, 2555
- **Hecht et al.** *Tetrahedron Letters,* 2002, vol. 43, 8929
- **Alison, T.J. ; Winter, C. C. ; Fournie, J. J. ; Bonneville, M. ; Garboczi, D. N.** Structure of a human gammadelta T-cell antigen receptor. *Nature,* 2001, vol. 411, 820-824
- **Bank, I. ; Book, M. ; Huszar, M. ; Baram, Y. ; Schnirer, I. ; Brenner, H.** V delta 2+ gamma delta T lymphocytes are cytotoxic to the MCF 7 breast carcinoma cell line and can be detected among the T cells that infiltrate breast tumors. *Clin Immunol Immunopathol,* 1993, vol. 67, 17-24
- **Behr, C. ; Poupot, R. ; Peyrat, M. A. ; Poquet, Y. ; Constant, P. ; Dubois, P. ; Bonneville, M. ; Fournie, J. J.** Plasmodium falciparum stimuli for human gammadelta T cells are related to phosphorylated antigens of mycobacteria. *Infect Immun,* 1996, vol. 64, 2892-2896
- **Belmant, C. ; Espinosa, E. ; Halary, F. ; Apostolou, I. ; Sicard, H. ; Peyrat, M. A. ; Vercellone, A. ; Kourilsky, P. ; Gachelin, G. ; Poupot, R. et al.** Conventional and non-conventional recognition of non-peptide antigens by T lymphocytes. *C R Acad Sci III,* 1999, vol. 322, 919-924
- **Belmant, C. ; Espinosa, E. ; Halary, F. ; Tang, Y. ; Peyrat, M. A. ; Sicard, H. ; Kozikowski, A ; Buelow, R. ; Poupot, R. ; Bonneville, M.** A chemical basis for selective recognition of nonpeptide antigens by human delta T cells. *Faseb J,* 2000, vol. 14, 1669-1670
- **Brondino et al.** *Journal of Fluorine Chemistry,* 1996, vol. 76, 193-200
- **Bukowski, J. F. ; Morita, C. T. ; Tanaka, Y. ; Bloom, B. R. ; Brenner, M. B. ; Band, H.** V gamma 2V delta 2 TCR-dependent recognition of non-peptide antigens and Daudi cells analyzed by TCR gene transfer. *J Immunol,* 1995, vol. 154, 998-1006
- **Buzio, C. ; Andrulli, S. ; Santi, R. ; Pavone, L. ; Passalacqua, R. ; Potenzoni, D. ; Ferrozzi, F. ; Giacosa, R. ; Vaglio, A.** Long-term immunotherapy with low-dose interleukin-2 and interferon-alpha in the treatment of patients with advanced renal cell carcinoma. *Cancer,* 2001, vol. 92, 2286-2296
- **Choudhary, A. ; Davodeau, F. ; Moreau, A. ; Peyrat, M. A. ; Bonneville, M. ; Jotereau, F.** Selective lysis of autologous tumor cells by recurrent gamma delta tumor-infiltrating lymphocytes from renal carcinoma. *J Immunol,* 1995, vol. 154, 3932-3940
- **Constant, P. ; Poquet, Y. ; Peyrat, M. A. ; Davodeau, F. ; Bonneville, M. ; Fournie, J. J.** The antituberculous Mycobacterium bovis BCG vaccine is an attenuated mycobacterial producer of phosphorylated nonpeptidic antigens for human gamma delta T cells. *Infect Immun,* 1995, vol. 63, 4628-4633
- **Davisson et al.** *J. Org. Chem.,* 1987, vol. 52, 1794-1801
- **Davisson et al.** *J. Org. Chem.,* 1986, vol. 51, 4768-4779
- **Davodeau et al.** *J. Immunology,* 1993, vol. 151 (3), 1214-1223
- **Espinosa, E. ; Belmant, C. ; Pont, F. ; Luciani, B. ; Poupot, R. ; Romagne, F. ; Brailly, H. ; Bonneville, M. ; Fournie, J. J.** Chemical synthesis and biological activity of bromohydrin pyrophosphate, a potent stimulator of human gamma delta T cells. *J Biol Chem,* 2001, vol. 276, 18337-18344
- **Espinosa, E. ; Belmant, C. ; Sicard, H. ; Poupot, R. ; Bonneville, M. ; Fournie, J. J.** Y2K+1 state-of-the-art on non-peptide phosphoantigens, a novel category of immunostimulatory molecules. *Microbes Infect,* 2001, vol. 3, 645-654

- **Ferrarini, M. ; Heltai, S. ; Pupa, S. M. ; Mernard, S. ; Zocchi, R.** Killing of laminin receptor-positive human lung cancers by tumor infiltrating lymphocytes bearing gammadelta(+) t-cell receptors. *J Natl Cancer Inst,* 1996, vol. 88, 436-441
- **Ferrarini, M. ; Pupa, S. M. ; Zocchi, M. R. ; Rugarli, C. ; Menard, S.** Distinct pattern of HSP72 and monomeric laminin receptor expression in human lung cancers infiltrated by gamma/delta T lymphocytes. *Int J Cancer,* 1994, vol. 57, 486-490
- **Feurle, J. ; Espinosa, E. ; Eckstein, S. ; Pont, F. ; Kunzmann, V. ; Fournie, J. J. ; Herderich, M. ; Wilhelm, M.** Escherichia coli produces phosphoantigens activating human gamma delta T cells. *J Biol Chem,* 2002, vol. 277, 148-154
- **Fisch, P. ; Moris, A. ; Rammensee, H. G. ; Handgretinger, R.** Inhibitory MHC class I receptors on gammadelta T cells in tumour immunity and autoimmunity. *Immunol Today,* 2000, vol. 21, 187-191
- **Fournie, J. J. ; Bonneville, M.** Stimulation of gamma delta T cells by phosphoantigens. *Res Immunol, 66th Forum in Immunology,* 1996, vol. 147, 338-347
- **Fujimiya, Y. ; Suzuki, Y. ; Katakura, R. ; Miyagi, T. ; Yamaguchi, T. ; Yoshimoto, T. ; Ebina, T.** In vitro interleukin 12 activation of peripheral blood CD3(+)CD56(+) and CD3(+)CD56(-) gammadelta T cells from glioblastoma patients. *Clin Cancer Res,* 1997, vol. 3, 633-643
- **Girardi, M. ; Oppenheim, D. E. ; Steele, C. R. ; Lewis, J. M. ; Glusac, E. ; Filler, R. ; Hobby, P. ; Sutton, B. ; Tigelaar, R. E. ; Hayday, A. C.** Regulation of cutaneous malignancy by gammadelta T cells. *Science,* 2001, vol. 294, 605-609
- **Gober, H. J. ; Kistowska, M. ; Angman, L. ; Jeno, P. ; Mori, L. ; De Libero, G.** Human T cell receptor gammadelta cells recognize endogenous mevalonate metabolites in tumor cells. *J Exp Med,* 2003, vol. 197, 163-168
- **Hayday, A. C.** [gamma] [delta] cells: a right time and a right place for a conserved third way of protection. *Annu Rev Immunol,* 2000, vol. 18, 975-1026
- **Hintz et al.** *FEBS Lett.,* 07 December 2001, vol. 509 (2), 317-22
- **Hintz et al.** *FEBS Letters,* 2001, vol. 509, 317-322
- **Jomaa, H. ; Feurle, J. ; Luhs, K. ; Kunzmann, V. ; Tony, H. P. ; Herderich, M. ; Wilhelm, M.** Vgamma9/Vdelta2 T cell activation induced by bacterial low molecular mass compounds depends on the 1-deoxy-D-xylulose 5-phosphate pathway of isoprenoid biosynthesis. *FEMS Immunol Med Microbiol,* 1999, vol. 25, 371-378
- **Jomaa, H. ; Wiesner, J. ; Sanderbrand, S. ; Altincicek, B. ; Weidemeyer, C. ; Hintz, M. ; Turbachova, I. ; Eberl, M. ; Zeidler, J. ; Lichtenthaler, H. K. et al.** Inhibitors of the nonmevalonate pathway of isoprenoid biosynthesis as antimalarial drugs. *Science,* 1999, vol. 285, 1573-1576
- **Kato, Y. ; Tanaka, Y. ; Miyagawa, F. ; Yamashita, S. ; Minato, N.** Targeting of tumor cells for human gammadelta T cells by nonpeptide antigens. *J Immunol,* 2001, vol. 167, 5092-5098
- **Kim, S. ; Iizuka, K. ; Aguila, H. L. ; Weissman, I. L. ; Yokoyama, W. M.** In vivo natural killer cell activities revealed by natural killer cell-deficient mice. *Proc Natl Acad Sci U S A,* 2000, vol. 97, 2731-2736
- **Knorre et al.** *Febs letters,* 1976, vol. 70, 105-108
- **Kobayashi, H. ; Tanaka, Y. ; Yagi, J. ; Toma, H. ; Uchiyama, T.** Gamma/delta T cells provide innate immunity against renal cell carcinoma. *Cancer Immunol Immunother,* 2001, vol. 50, 115-124
- **Kunzmann, V. ; Bauer, E. ; Feurle, J. ; Weissinger, F. ; Tony, H. P. ; Wilhelm, M.** Stimulation of gammadelta T cells by aminobisphosphonates and induction of antiplasma cell activity in multiple myeloma. *Blood,* 2000, vol. 96, 384-392
- **Kunzmann, V. ; Bauer, E. ; Wilhelm, M.** Gamma/delta T-cell stimulation by pamidronate. *N Engl J Med,* 1999, vol. 340, 737-738
- **Lang, F. ; Peyrat, M. A. ; Constant, P. ; Davodeau, F. ; David-Ameline, J. ; Poquet, Y. ; Vie, H. ; Fournie, J. J. ; Bonneville, M.** Early activation of human V gamma 9V delta 2 T cell broad cytotoxicity and TNF production by nonpeptidic mycobacterial ligands. *J Immunol,* 1995, vol. 154, 5986-5994
- **Liu et al.** *Angew. Chem. Int. Ed.,* 1999, vol. 38 (9), 1245-1247
- **Mitropoulos, D. ; Kooi, S. ; Rodriguez-Villanueva, J. ; Platsoucas, C. D.** Characterization of fresh (uncultured) tumour-infiltrating lymphocytes (TIL) and TIL-derived T cell lines from patients with renal cell carcinoma. *Clin Exp Immunol,* 1994, vol. 97, 321-327
- **Miyagawa, F. ; Tanaka, Y. ; Yamashita, S. ; Minato, N.** Essential requirement of antigen presentation by monocyte lineage cells for the activation of primary human gamma delta T cells by aminobisphosphonate antigen. *J Immunol,* 2001, vol. 166, 5508-5514
- **Moreau et al.** *J. Clin. Invest.,* 1986, vol. 78, 874
- **Morita, C. T. ; Beckman, E. M. ; Bukowski, J. F. ; Tanaka, Y. ; Band, H. ; Bloom, B. R. ; Golan, D. E. ; Brenner, M. B.** Direct presentation of nonpeptide prenyl pyrophosphate antigens to human gamma delta T cells. *Immunity,* 1995, vol. 3, 495-507
- **Nakamura et al.** *Tetrahedron Letters,* 1973, 111-112

- **Nilsson et al.** *Acta Chemica Scandinavia B,* 1981, vol. 35, 19-27
- **Nilsson ; Mosbach.** *Eur. J. Biochem.,* 1980, vol. 112, 397-402
- **Poccia, F. ; Cipriani, B. ; Vendetti, S. ; Colizzi, V. ; Poquet, Y. ; Battistini, L. ; Lopez-Botet, M. ; Fournie, J. J. ; Gougeon, M. L.** CD94/NKG2 inhibitory receptor complex modulates both anti-viral and anti-tumoral responses of polyclonal phosphoantigen-reactive V gamma 9V delta 2 T lymphocytes. *J Immunol,* 1997, vol. 159, 6009-6017
- **Poccia, F. ; Malkovsky, M. ; Gougeon, M. L. ; Bonneville, M. ; Lopez-Botet, M. ; Fournie, J. J. ; Colizzi, V.** Gammadelta T cell activation or anergy during infections: the role of nonpeptidic TCR ligands and HLA class I molecules. *J Leukoc Biol,* 1997, vol. 62, 287-291
- **Poquet, Y. ; Kroca, M. ; Halary, F. ; Stenmark, S. ; Peyrat, M. A. ; Bonneville, M. ; Fournie, J. J. ; Sjostedt, A.** Expansion of Vgamma9 Vdelta2 T cells is triggered by Francisella tularensis-derived phosphoantigens in tularemia but not after tularemia vaccination. *Infect Immun,* 1998, vol. 66, 2107-2114
- **Rohmer, M. ; Knani, M. ; Simonin, P. ; Sutter, B. ; Sahm, H.** Isoprenoid biosynthesis in bacteria: a novel pathway for the early steps leading to isopentenyl diphosphate. *Biochem J,* 1993, vol. 295, 517-524
- **Rojas, R. E. ; Torres, M. ; Fournie, J. J. ; Harding, C. V. ; Boom, W. H.** Phosphoantigen presentation by macrophages to mycobacterium tuberculosis-reactive Vgamma9Vdelta2+ T cells: modulation by chloroquine. *Infect Immun,* 2002, vol. 70, 4019-4027
- **Selin, L. K. ; Stewart, S. ; Shen, C. ; Mao, H. Q. ; Wilkins, J. A.** Reactivity of gamma delta T cells induced by the tumour cell line RPMI 8226: functional heterogeneity of clonal populations and role of GroEL heat shock proteins. *Scand J Immunol,* 1992, vol. 36, 107-117
- **Shen, Y. ; Zhou, D. ; Qiu, L. ; Lai, X. ; Simon, M. ; Shen, L. ; Kou, Z. ; Wang, Q. ; Jiang, L. ; Estep, J. et al.** Adaptive immune response of Vgamma2Vdelta2+ T cells during mycobacterial infections. *Science,* 2002, vol. 295, 2255-2258
- **Sicard, H. ; Al Saati, T. ; Delsol, G. ; Fournie, J. J.** Synthetic phosphoantigens enhance human Vgamma9Vdelta2 T lymphocytes killing of non-Hodgkin's B lymphoma. *Mol Med,* 2001, vol. 7, 711-722
- **Sicard, H. ; Fournie, J. J.** Metabolic routes as targets for immunological discrimination of host and parasite. *Infect Immun,* 2000, vol. 68, 4375-4377
- **Smyth, M. J. ; Cretney, E. ; Takeda, K. ; Wiltrout, R H. ; Sedger, L. M. ; Kayagaki, N. ; Yagita, H. ; Okumura, K.** Tumor necrosis factor-related apoptosis-inducing ligand (TRAIL) contributes to interferon gamma-dependent natural killer cell protection from tumor metastasis. *J Exp Med,* 2001, vol. 193, 661-670
- **Smyth, M. J. ; Thia, K. Y. ; Street, S. E. ; Cretney, E. ; Trapani, J. A. ; Taniguchi, M. ; Kawano, T. ; Pelikan, S. B. ; Crowe, N. Y. ; Godfrey, D. 1.** Differential tumor surveillance by natural killer (NK) and NKT cells. *J Exp Med,* 2000, vol. 191, 661-668
- **Sturm, E. ; Braakman, E. ; Fisch, P. ; Vreugdenhil, R. J. ; Sondel, P. ; Bolhuis, R. L.** Human V gamma 9-V delta 2 T cell receptor-gamma delta lymphocytes show specificity to Daudi Burkitt's lymphoma cells. *J Immunol,* 1990, vol. 145, 3202-3208
- **Tanaka, Y. ; Morita, C. T. ; Nieves, E. ; Brenner, M. B. ; Bloom, B. R.** Natural and synthetic non-peptide antigens recognized by human gamma delta T cells. *Nature,* 1995, vol. 375, 155-158
- **Umbreit ; Sharpless.** *JACS,* 1977, vol. 99-16, 5526-5527
- **Valentijn et al.** *Syn. Lett.,* 1991, 663-664
- **Wilhelm, M. ; Kunzmann, V. ; Eckstein, S. ; Reimer, P. ; Weissinger, F. ; Ruediger, T. ; Tony, H. P.** {gamma} {delta} T cells for immune therapy of patients with lymphoid malignancies. *Blood,* 2003
- **Yamaguchi, T. ; Fujimiya, Y. ; Suzuki, Y. ; Katakura, R. ; Ebina, T.** A simple method for the propagation and purification of gamma delta T cells from the peripheral blood of glioblastoma patients using solid-phase anti-CD3 antibody and soluble IL-2. *J Immunol Methods,* 1997, vol. 205, 19-28
- **Zheng, B. ; Lam, C. ; Im, S. ; Huang, J. ; Luk, W. ; Lau, S. Y. ; Yau, K. K. ; Wong, C. ; Yao, K. ; Ng, M. H.** Distinct tumour specificity and I-7 requirements of CD56(-)and CD56(+) subsets of human gamma delta T cells. *Scand J Immunol,* 2001, vol. 53, 40-48
- **Zheng, B. J. ; Chan, K. W. ; Im, S. ; Chua, D. ; Sham, J. S. ; Tin, P. C. ; He, Z. M. ; Ng, M. H.** Anti-tumor effects of human peripheral gammadelta T cells in a mouse tumor model. *Int J Cancer,* 2001, vol. 92, 421-425
- **Zoretic ; Zhang.** *Tetrahedron Letters,* 1996, vol. 37 (11), 1751-1754